# EUROPEAN PATENT SPECIFICATION

(11) **EP 3 930 461 B1**
(45) Date of publication and mention of the grant of the patent: **07.10.2026**
(21) Application number: 20728769.9
(22) Date of filing: 29.05.2020
(51) Int. Cl.: A01N 31/02, A01N 37/02, A01N 43/30, C07C 35/32, C07C 69/04, C07D 305/14, C07D 319/08, A01P 17/00

(54) **ARTHROPOD CONTROLLING COMPOSITION**
GLIEDERFÜSSLERBEKÄMPFUNGSZUSAMMENSETZUNG
COMPOSITION DE CONTRÔLE DES ARTHROPODES

(30) Priority: 31.05.2019 EP 19177668
(43) Date of publication of application: 05.01.2022
(73) Proprietor: Firmenich SA, 1242 Satigny (CH)
(72) Inventor: CHAPPUIS, Charles, 1242 Satigny (CH); HARRACA, Vincent, 1242 Satigny (CH); BANDERA, Monica, 1242 Satigny (CH)
(74) Representative: dsm-firmenich IP
(86) International application number: PCT/EP2020/064976
(87) International publication number: WO 2020/239980

(56) References cited:
- EP-A1- 1 022 265
- WO-A1-2016/113151
- WO-A1-2017/005517
- WO-A2-2019/101821
- US-A1- 2018 042 225
- SONNENBICHLER J ET AL: "SECONDARY FUNGAL METABOLITES AND THEIR BIOLOGICAL ACTIVITIES, IV. SYNTHESIS OF COMPOUNDS WITH STRUCTURAL SIMILARITIES TO THE TOXIC METABOLITES OF THE PATHOGENIC FUNGUS HETEROBASIDION ANNOSUM AND INVESTIGATION OF THEIR ANTIBIOTIC ACTIVITIES", BIOLOGICAL CHEMISTRY HOPPE-SEYLER, WALTER DE GRUYTER, BERLIN, DE, vol. 374, no. 11, 1 November 1993 (1993-11-01), pages 1047 - 1055, XP000874559, ISSN: 0177-3593

## Description

### Technical Field

The present invention relates to the non therapeutic use of an arthropod controlling composition comprising a compound according to formula (I), as well as to the corresponding method for arthropod control and to some specific instances of compounds (I) *per se.* In the present specification, apart from the very compounds according to claim 12, compounds and compositions according to the present inventions must be read as compounds and compositions used according to the present invention.

### Background

Many mammals, including humans, are suffering from the action of arthropods. Some arthropods, such as for example mosquitoes and ticks, are not desirable for vertebrates such as mammals and in particular human subjects as they bite and, consequently, cause itching, transmission of diseases and/or germs or may be the cause for other diseases and/or conditions.

Arthropod control compositions include active substances and when applied to skin, clothing, or other surfaces, they may discourage arthropods from landing or climbing on that surface. Arthropod control agents help preventing and controlling the outbreak of arthropod-borne diseases, such as malaria, etc.

The prior art describes structurally diverse arthropod control agents, such as for example N,N-diethyl-3-methylbenzamide (also known as DEET) or ethyl butylacetylaminopropionate (also known as insect repellent 3535, IR3535).

Some of the known arthropod control agents, however, have certain drawbacks, such as being irritant to the skin, to a subject, in particular of children, and/or have negative olfactive properties, such as bad smell.

There is a need to provide arthropod controlling compounds and compositions which use limited amount of or are totally free from irritant arthropod control agents.

EP 1 022 265 describes the olfactory properties of some compounds used in compositions according to the present invention and their use as perfuming ingredients providing odoriferous characteristics of the lily of the valley type, but does not disclose or suggest said compounds as having an arthropod controlling effect. US 2018/042225 discloses some insect repellents amongst which some indene derivatives.

No prior art discloses or suggests the compounds according to the present invention as having an arthropod controlling effect.

### Description of the Figures

- Figure 1:: *In-vitro* repulsion of mosquitoes *Aedes aegypti* measured against different dilutions of (2,5-dimethyl-2,3-dihydro-1H-inden-2yl)methanol in the K&D test.
- Figure 2:: *In-vivo* repulsion of mosquitoes *Aedes aegypti* measured against different dilutions of (2,5-dimethyl-2,3-dihydro-1H-inden-2yl)methanol in the K&D test.
- Figure 3:: Numbers of mosquitoes *Aedes aegypti* landing on the Warm Body with different dilutions of DEET (circle plain line), IR3535 (triangle dashed line) or (2,5-dimethyl-2,3-dihydro-1H-inden-2yl)methanol (cross, dotted line) applied on it.
- Figure 5:: *In-vitro* repulsion of mosquitoes *Aedes aegypti* measured in the Warm Body Assay against two different perfumes containing 33% and 10% of (2,5-dimethyl-2,3-dihydro-1H-inden-2yl)methanol (square; black & *triangle; black respectively),* two reference compounds: DEET (diamond; *gray),* IR3535 (circle; *gray)* and the solvent ethanol (no index; *black).*

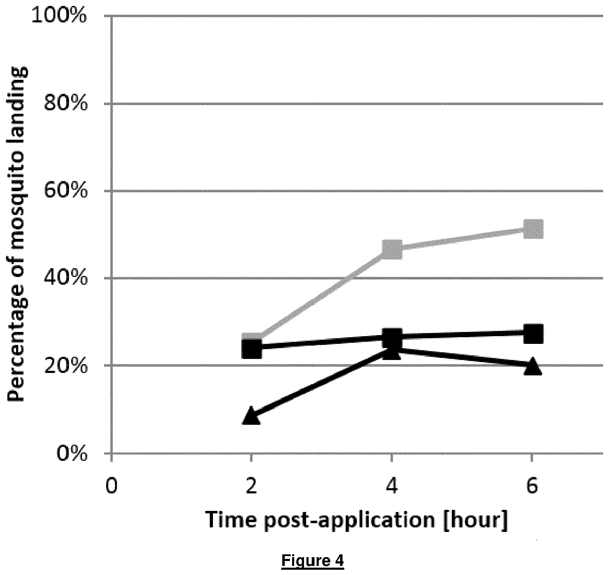

- Figure 4:: *In-vitro* repulsion overtime of mosquitoes *Aedes aegypti* measured in the Warm Body Assay against two different perfumes containing 33% and 6.5% of (2,5-dimethyl-2,3-dihydro-1H-inden-2yl)methanol (square & triangle respectively) plus 7.5% or 12.5% of IR3535 (gray and black respectively) in an aqueous phase.

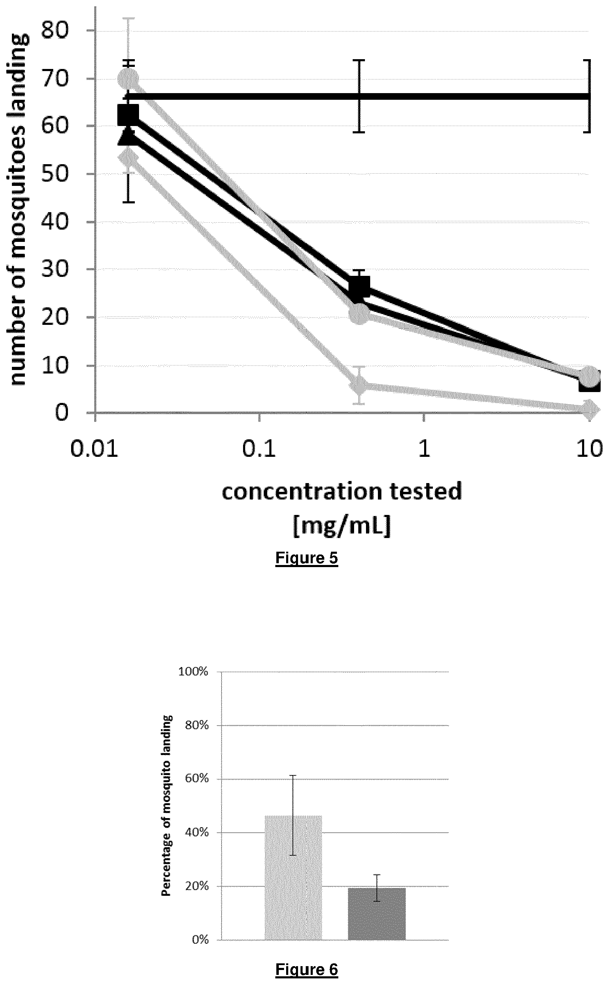

- Figure 6:: *In-vitro* repulsion of mosquitoes *Aedes aegypti* measured in the Warm Body Assay 4h post application against two different blends made of 0.25% or 1% of perfume 1a containing 33% of (2,5-dimethyl-2,3-dihydro-1H-inden-2yl)methanol (light and dark gray respectively) plus 12.5% of IR3535 in an aqueous phase.

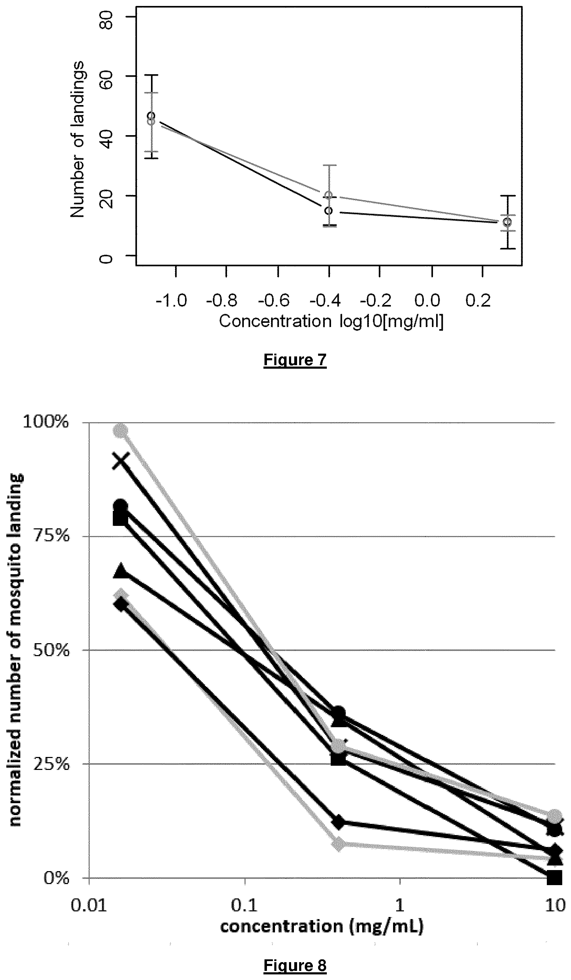

- Figure 7:: *In-vitro* repulsion of mosquitoes *Aedes aegypti* measured in the Warm Body Assay against a blend (gray) containing two perfumery ingredients with arthropods' controlling properties ((2,5-dimethyl-2,3-dihydro-1H-inden-2yl)methanol + methyl (1R)-cis-3-oxo-2-pentyl-1-cyclopentaneacetate [1:1]) or methyl (1R)-cis-3-oxo-2-pentyl-1-cyclopentaneacetate (black) used as reference. All stimuli were diluted in ethanol at 0.0016%, 0.04% and 1% (w/w).
- Figure 8:: *In-vitro* repulsion of mosquitoes *Aedes aegypti* measured in the Warm Body Assay against seven different compounds at 3 different concentrations: DEET (gray diamond) & IR3535 (gray circle) used as references, 4a,8-dimethyl-indano[1,2-d]-1,3-dioxan-2-one (black square), (2,4,6-trimethyl-2,3-dihydro-1*H*-inden-2-yl)methanol (black diamond), 5-ethyl-2-methyl-2-indanmethanol (black triangle), 1-(2,5-dimethyl-2-indanyl)-1-ethanol (black circle) and (2,5-dimethyl-2,3-dihydro-1H-inden-2yl)methanol (black cross).

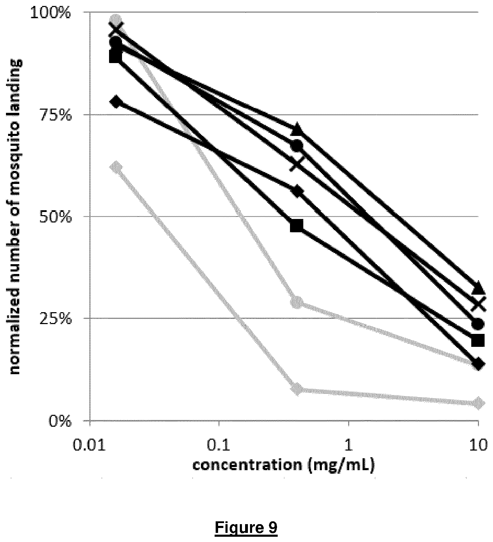

- Figure 9:: *In-vitro* repulsion of mosquitoes *Aedes aegypti* measured in the Warm Body Assay against seven different compounds at 3 different concentrations: DEET (gray diamond) & IR3535 (gray circle) used as references, (2,5-dimethyl-2-indanyl)methyl formate (black square), (2,5-dimethyl-2,3-dihydro-1H-inden-2-yl)methyl methyl ether (black diamond), 2,5-dimethyl-2,3-dihydro-1H-indene-2-carboxylic acid (black triangle), (2,5-dimethyl-2,3-dihydro-1H-inden-2yl)methanol (black circle) and 5-isopropyl-2-methyl-2-indanmethanol (black cross)

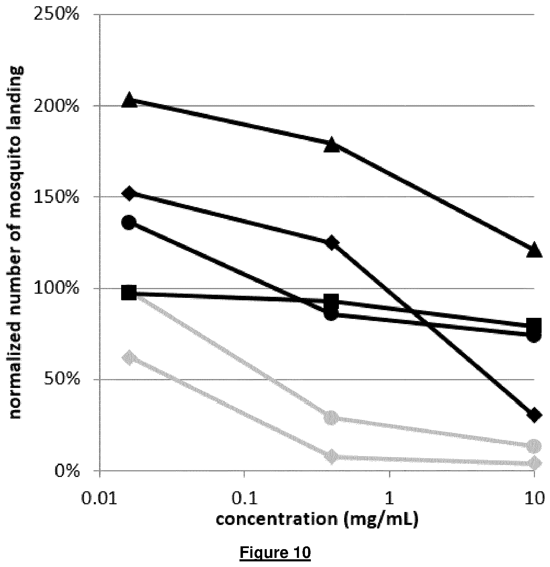

- Figure 10:: *In-vitro* repulsion of mosquitoes *Aedes aegypti* measured in the Warm Body Assay against eleven different compounds at 3 different concentrations: DEET (gray diamond) & IR3535 (gray circle) used as references, (1RS,2SR)-2-(hydroxymethyl)-2,6-dimethyl-2,3-dihydro-1H-inden-1-ol (black circle), 5-tert-butyl-2-methyl-2-indanmethanol (black square), a blend of (1RS,2RS)-2-((2-methoxypropan-2-yloxy)methyl)-2,6-dimethyl-2,3-dihydro-1H-inden-1-ol + (1RS,2SR)-2-((2-methoxypropan-2-yloxy)methyl)-2,6-dimethyl-2,3-dihydro-1H-inden-1-ol (black diamond) and a blend of (1RS,2RS)-2-(hydroxymethyl)-2,6-dimethyl-2,3-dihydro-1H-inden-1-ol + (1RS,2SR)-2-(hydroxymethyl)-2,6-dimethyl-2,3-dihydro-1 H-inden-1-ol (black triangle).

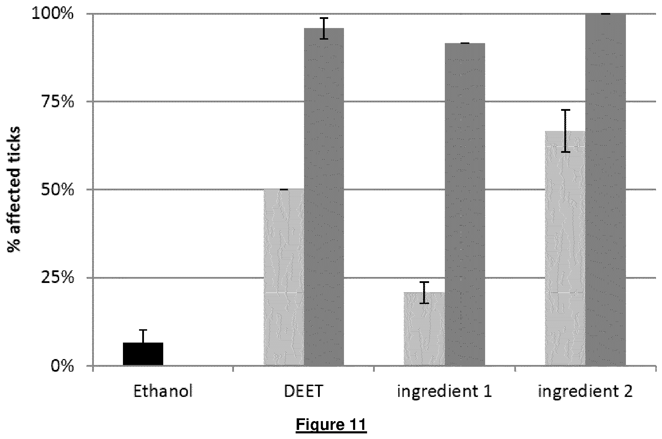

- Figure 11:: *In-vitro* repulsion of ticks *Ixodes ricinus* measured in the Warm Body Assay against ethanol (control; black), (2,4,6-trimethyl-2,3-dihydro-1H-inden-2-yl)methanol (Ingredient1) (2,5-dimethyl-2,3-dihydro-1H-inden-2yl)methanol (Ingredient2) and N,N-diethyl-3-methylbenzamide (DEET) diluted in ethanol at 0.4 mg/mL (light gray) and 10 mg/mL (dark gray ; mean values of 2-5 replicates of n=11-12 ticks).

### Detailed description

The present invention relates to the use against arthropods of an arthropod, preferably insect, control compositior comprising
- a compound of formula (I) 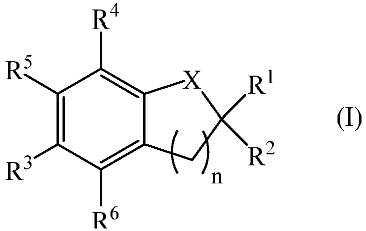
   in the form of any one of its stereoisomers or a mixture thereof, and wherein
   n represents 1 or 2;
   X represents a CH₂, CH(OH) or C=N-OH group;
   R¹ represents a CN, CHR⁷OR⁸ or a COR⁹ group,
   R⁷ being a hydrogen atom or a methyl group,
   R⁸ being a hydrogen atom, a C₁₋₃ hydrocarbon group, optionally substituted by a hydroxyl or a C₁₋₃ alkoxy group, a C₃₋₁₀ trialkylsilyl group, or a COR⁹ group, and R⁹ being a hydrogen atom, a hydroxyl group, a C₁₋₃ alkoxy group, a C₁₋₈ hydrocarbon group, or a phenyl, each optionally substituted by one to three groups selected amongst a C₁₋₃ alkyl, alkoxy or amine group;
   R² represents a hydrogen atom or a methyl, ethyl or CH₂OH group;
   R³ represents a hydrogen atom or a C₁₋₅ hydrocarbon group or a C₁₋₃ alkoxyl group; and
   R⁴, R⁵ and R⁶ represents, independently from each other, a hydrogen atom or a C₁₋₅ alkyl or C₂₋₅ alkenyl group; or
   R¹ and R² when taken together represent a CH₂-O-C(=O)-O, CH₂-O-CH₂-O or CH₂-O-CH₂ group; or
   X and R¹ when taken together represent a CH-O-C(=O)-O-C(R¹⁰)₂ or CH-O-C(R¹⁰)₂-O-C(R¹⁰)₂ group wherein R¹⁰, independently from each other, represents a hydrogen atom or a C₁₋₃ alkyl group;
- optionally, at least one arthropod control co-ingredient.

In a particular embodiment, the compound of formula (I) is defined in that n is 1.

In a particular embodiment, the compound of formula (I) is defined in that X represents a CH₂ group.

In a particular embodiment, the compound of formula (I) is defined in that R¹ represents a CN, a CH₂OR⁸, CHMeOR⁸ or a COR⁹ group, R⁸ being a hydrogen atom, or a C₁₋₃ hydrocarbon group or a COR⁹ group, and R⁹ being a hydrogen atom or a C₁₋₃ hydrocarbon group, preferably a C₁₋₃ linear or branched alkyl or linear or branched C₂₋₃ alkenyl group.

In a particular embodiment, the compound of formula (I) is defined in that R¹ represents a CN, a CH₂OR⁸ CHMeOR⁸ or a COR⁹ group, R⁸ being a hydrogen atom or a methyl or ethyl group or a COR⁹ group, and R⁹ being a methyl or ethyl group.

In a particular embodiment, the compound of formula (I) is defined in that R¹ represents a CN, a CH₂OR⁸ group, R⁸ being a hydrogen atom or a methyl or ethyl group or a MeCO group.

In a particular embodiment, the compound of formula (I) is defined in that R² represents a hydrogen atom or a methyl group.

In a particular embodiment, the compound of formula (I) is defined in that R² represents a methyl group.

In a particular embodiment, the compound of formula (I) is defined in that R³ represents a hydrogen atom or a C₁₋₄ alkyl group.

In a particular embodiment, the compound of formula (I) is defined in that R³ represents a hydrogen atom or a methylor a tert-butyl group.

In a particular embodiment, the compound of formula (I) is defined in that R⁴, R⁵ and R⁶ represents, independently from each other, a hydrogen atom or a C₁₋₃ alkyl or C₂₋₃ alkenyl.

In a particular embodiment, the compound of formula (I) is defined in that R⁴, R⁵ and R⁶ represents, independently from each other, a hydrogen atom or a C₁₋₃ alkyl group.

In a particular embodiment, the compound of formula (I) is defined in that R⁴, R⁵ and R⁶ represents, independently from each other, a hydrogen atom or a methyl group.

In a particular embodiment, R¹ and R² when taken together represents a CH₂-O-C(=O)-O or CH₂-O-CH₂ group, preferably a CH₂-O-CH₂ group.

In a particular embodiment, X and R¹ when taken together represent a CH-O-C(=O)-O-C(R¹⁰)₂ or CH-O-C(R¹⁰)₂-O-C(R¹⁰)₂ group wherein R¹⁰ represents a hydrogen atom or a C₁₋₂ alkyl group. Preferably, at most two R¹⁰ represent a C₁₋₂ alkyl group, the rest being a hydrogen atom. Preferably, at most two R¹⁰ represent a methyl group the rest being a hydrogen atom.

In a particular embodiment, the compound of formula (I) is defined in that R³, R⁴, R⁵ and R⁶ are not hydrogen atoms at the same time.

In a particular embodiment, the compound of formula (I) is 2,3-dihydro-1H-indene-2-carbonitrile, 2,4-dimethyl-4,4a,5,9b-tetrahydroindeno[1,2-d][1,3]dioxine, 2,4-dimethyl-4,4a,5,9b-tetrahydroindeno[1,2-d][1,3]dioxine, (2,5-dimethyl-2,3-dihydro-1H-inden-2yl)methanol, (4aRS,9bRS)-4a,7-dimethyl-4,4a,5,9b-tetrahydroindeno[1,2-d][1,3]dioxine, (2RS,4aRS,9bRS)-2,4a,7-trimethyl-4,4a,5,9b-tetrahydroindeno[1,2-d][1,3]dioxine (2RS,4aSR,9bSR)-2,4a,7-trimethyl-4,4a,5,9b-tetrahydroindeno[1,2-d][1,3]dioxine, (2-methyl-1,3-dihydroinden-2-yl)methanol, 2,3-dihydro-1H-inden-2-ylmethanol, (2RS,4RS,4ASR,9BRS)-2,4-dimethyl-4,4a,5,9b-tetrahydroindeno[1,2-d][1,3]dioxine, (2RS,4aRS,9bSR)-2-methyl-4,4a,5,9b-tetrahydroindeno[1,2-d][1,3]dioxin (2RS,4aSR,9bRS)-2-methyl-4,4a,5,9b-tetrahydroindeno[1,2-d][1,3]dioxin, (4aRS,9bSR)-2,2-dimethyl-4,4a,5,9b-tetrahydroindeno[1,2-d][1,3]dioxin, (2,4,6-trimethyl-2,3-dihydro-1H-inden-2-yl)methanol, 5-(tert-butyl)-1,3-dihydrospiro[indene-2,3'-oxetane], (2RS,4RS,4ARS,9BSR)-2,4-dimethyl-4,4a,5,9b-tetrahydroindeno[1,2-d][1,3]dioxine, 4A,8-dimethyl-indano[1,2-d]-1,3-dioxan-2-one, 1-(2,5-dimethyl-2,3-dihydro-1H-inden-2-yl)ethanone, (5-methyl-2,3-dihydro-1H-inden-2-yl)methanol, (4aRS,9bSR)-8-methyl-4,4a,5,9b-tetrahydroindeno[1,2-d][1,3]dioxin, (2RS,4aRS,9bSR)-2-ethyl-4,4a,5,9b-tetrahydroindeno[1,2-d][1,3]dioxine (2RS,4aSR,9bRS)-2-ethyl-4,4a,5,9b-tetrahydroindeno[1,2-d][1,3]dioxine, (2R,4aS,9bS)-2,4a,8-trimethyl-4,4a,5,9b-tetrahydroindeno[1,2-d][1,3]dioxin, 1-(2,5-dimethyl-2-indanyl)-1-ethanol, (2,5-dimethyl-2,3-dihydro-1H-inden-2yl)methanol, (2RS,4aRS,9bSR)-2,8-dimethyl-4,4a,5,9b-tetrahydroindeno[1,2-d][1,3]dioxine (2RS,4aSR,9bRS)-2,8-dimethyl-4,4a,5,9b-tetrahydroindeno[1,2-d][1,3]dioxine, (2RS,4aSR,9bSR)-2,4a-dimethyl-4,4a,5,9b-tetrahydroindeno[1,2-d][1,3]dioxin, (4aRS,9bRS)-4a,8-dimethyl-4,4a,5,9b-tetrahydroindeno[1,2-d][1,3]dioxin, 1-(2,4,5-trimethyl-2,3-dihydro-1H-inden-2-yl)ethenone, (2RS,4aRS,9bSR)-2-ethyl-8-methyl-4,4a,5,9b-tetrahydroindeno[1,2-d][1,3]dioxin, (2RS,4aSR,9bRS)-2-ethyl-8-methyl-4,4a,5,9b-tetrahydroindeno[1,2-d][1,3]dioxin, (4aRS,9bRS)-2,2,4a,8-tetramethyl-4,4a,5,9b-tetrahydroindeno[1,2-d][1,3]dioxine, 5-tert-butyl-indan-2-spiro-3'-oxetane, (2,6-dimethyl-1,2,3,4-tetrahydro-2-naphthalenyl)methanol, (4aRS,9bRS)-2,2,4a-trimethyl-4,4a,5,9b-tetrahydroindeno[1,2-d][1,3]dioxine, (4aRS,9bRS)-2,2,4a,7-tetramethyl-4,4a,5,9b-tetrahydroindeno[1,2-d][1,3]dioxine.

In a preferred embodiment, the compound of formula (I) is 2,3-dihydro-1H-indene-2-carbonitrile, 2,4-dimethyl-4,4a,5,9b-tetrahydroindeno[1,2-d][1,3]dioxine, 2,4-dimethyl-4,4a,5,9b-tetrahydroindeno[1,2-d][1,3]dioxine, (2,5-dimethyl-2,3-dihydro-1H-inden-2yl)methanol, (4aRS,9bRS)-4a,7-dimethyl-4,4a,5,9b-tetrahydroindeno[1,2-d][1,3]dioxine, (2RS,4aRS,9bRS)-2,4a,7-trimethyl-4,4a,5,9b-tetrahydroindeno[1,2-d][1,3]dioxine, (2RS,4aSR,9bSR)-2,4a,7-trimethyl-4,4a,5,9b-tetrahydroindeno[1,2-d][1,3]dioxine, (2-methyl-1,3-dihydroinden-2-yl)methanol, 2,3-dihydro-1H-inden-2-ylmethanol, (2RS,4RS,4ASR,9BRS)-2,4-dimethyl-4,4a,5,9b-tetrahydroindeno|[1,2-d][1,3]dioxine, (2RS,4aRS,9bSR)-2-methyl-4,4a,5,9b-tetrahydroindeno[1,2-d][1,3]dioxin, (2RS,4aSR,9bRS)-2-methyl-4,4a,5,9b-tetrahydroindeno[1,2-d][1,3]dioxin, (4aRS,9bSR)-2,2-dimethyl-4,4a,5,9b-tetrahydroindeno[1,2-d][1,3]dioxin, (2,4,6-trimethyl-2,3-dihydro-1H-inden-2-yl)methanol, 5-(tert-butyl)-1,3-dihydrospiro[indene-2,3'-oxetane], (2RS,4RS,4ARS,9BSR)-2,4-dimethyl-4,4a,5,9b-tetrahydroindeno[1,2-d][1,3]dioxine, 4A,8-dimethyl-indano[1,2-d]-1,3-dioxan-2-one, 1-(2,5-dimethyl-2,3-dihydro-1H-inden-2-yl)ethanone, (5-methyl-2,3-dihydro-1H-inden-2-yl)methanol, (4aRS,9bSR)-8-methyl-4,4a,5,9b-tetrahydroindeno[1,2-d][1,3]dioxin, (2RS,4aRS,9bSR)-2-ethyl-4,4a,5,9b-tetrahydroindeno[1,2-d][1,3]dioxine, (2RS,4aSR,9bRS)-2-ethyl-4,4a,5,9b-tetrahydroindeno[1,2-d][1,3]dioxine, (2R,4aS,9bS)-2,4a,8-trimethyl-4,4a,5,9b-tetrahydroindeno[1,2-d][1,3]dioxin.

In a particular embodiment, the compound of formula (I) is (2,5-dimethyl-2,3-dihydro-1H-inden-2yl)methanol, 5-(tert-butyl)-1,3-dihydrospiro[indene-2,3'-oxetane], 5-tert-butyl-indan-2-spiro-3'-oxetane, 5-ethyl-2-methyl-2-indanmethanol, (2,4,6-trimethyl-2,3-dihydro-1*H-*inden-2-yl)methanol, 1-(2,5-dimethyl-2,3-dihydro-1H-inden-2-yl)ethanone, 2,3-dihydro-1H-inden-2-ylmethanol, 2,3-dihydro-1H-indene-2-carbonitrile, 5, 1-(2,5-dimethyl-2-indanyl)-1-ethanol, (2,5-dimethyl-2-indanyl)methyl formate, (2,5-dimethyl-2,3-dihydro-1H-inden-2-yl)methyl methyl ether, (2,6-dimethyl-1,2,3,4-tetrahydro-2-naphthalenyl)methanol, 4a,8-dimethyl-indano[1,2-d]-1,3-dioxan-2-one, 8-methyl-4,4a-5,9b-tetrahydroindeno[1,2-d][1,3]dioxin, in particular (4aRS,9bSR)-8-methyl-4,4a-5,9b-tetrahydroindeno[1,2-d][1,3]dioxin, (5-methyl-2,3-dihydro-1H-inden-2-yl)methanol, (2-methyl-2,3-dihydro-1H-inden-2-yl)methyl acetate, 2,3-dihydro-1H-inden-2-ylmethanol or 2,3-dihydro-1H-indene-2-carbonitrile.

In a particular embodiment, the arthropod control composition comprises:(2,5-dimethyl-2,3-dihydro-1H-inden-2yl)methanol, 5-tert-butyl-indan-2-spiro-3'-oxetane, 2-methyl-1,3-dihydroinden-2-yl)methanol, 5-(tert-butyl)-1,3-dihydrospiro[indene-2,3'-oxetane], (+-)-5-isopropyl-2-methyl-2-indanmethanol, 1-(2,5-dimethyl-2-indanyl)-1-ethanol, (2,5-dimethyl-2-indanyl)methyl formate, 2,5-dimethyl-2,3-dihydro-1H-inden-2-yl)methyl methyl ether, (2,5,6-trimethyl-2,3-dihydro-1H-inden-2-yl)methanol, (2,5-dimethyl-2-indanyl)methyl formate)methanol, (2,5-dimethyl-2,3-dihydro-1H-inden-2yl)methanol, (+-)-5-tert-butyl-2-methyl-2-indanmethanol, (+-)-(2,4,6-trimethyl-2,3-dihydro-1H-inden-2-yl)methanol, blend of (1RS,2RS)-2-((2-methoxypropan-2-ylo xy)methyl)-2,6-dimethyl-2,3-dihydro-1H-inden-1-ol + (1RS,2SR)-2-((2-methoxypropan-2-yloxy)methyl)-2,6-dimethyl-2,3-dihydro-1H-inden-1-ol, 4A,8-dimethyl-indano[1,2-D]-1,3-dioxan-2-one, (+-)-(2,5-dimethyl-2,3-dihydro-1H-inden-2-yl)methyl methyl carbonate, (+-)-methyl 2,5-dimethyl-2,3-dihydro-1H-indene-2-carboxylate, (+-)-(2,6-dimethyl-1,2,3,4-tetrahydro-2-naphthalenyl)methanol, (+-)-1-(2,5-dimethyl-2,3-dihydro-1H-inden-2-yl)ethanone, (+-)-(5-methyl-2,3-dihydro-1H-inden-2-yl)methanol, (+-)-1-(2,4,5-trimethyl-2,3-dihydro-1H-inden-2-yl)ethanone, 2,3-dihydro-1H-inden-2-ylmethanol, (2-methyl-2,3-dihydro-1H-inden-2-yl)methyl acetate, (4aRS,9bSR)-8-methyl-4,4a,5,9b-tetrahydroindeno[1,2-d][1,3]dioxin, (+-)-2-((methoxymethoxy)methyl)-2,5-dimethyl-2,3-dihydro-1H-indene, (+-)-2-[(1-methoxyethoxy)methyl]-2,5-dimethylindane, 2,3-dihydro-1H-indene-2-carbonitrile, blend of (+-)-2,4-dimethyl-4,4a,5,9b-tetrahydroindeno[1,2-d][1,3]dioxine + (+-)-2,4-dimethyl-4,4a,5,9b-tetrahydroindeno[1,2-d][1,3]dioxine, 4,4A,5,9B-tetrahydro-indeno[1,2-D]-1,3-dioxin, (4aRS,9bRS)-4a,8-dimethyl-4,4a,5,9b-tetrahydroindeno[1,2-d][1,3]dioxin, (2S,4AS,9BS)-2,4A,8-trimethyl-4,4a,5,9b-tetrahydroindeno[1,2-d][1,3]dioxine, (2R,4aS,9bS)-2,4a,8-trimethyl-4,4a,5,9b-tetrahydroindeno[1,2-d][1,3]dioxin, (2RS,4RS,4ARS,9BSR)-2,4-dimethyl-4,4a,5,9b-tetrahydroindeno[1,2-d][1,3]dioxine, (2RS,4RS,4ASR,9BRS)-2,4-dimethyl-4,4a,5,9b-tetrahydroindeno[1,2-d][1,3]dioxine, (4aRS,9bRS)-2,2,4a,8-tetramethyl-4,4a,5,9b-tetrahydroindeno[1,2-d][1,3]dioxine, (4aRS,9bRS)-2,2,4a,7-tetramethyl-4,4a,5,9b-tetrahydroindeno[1,2-d][1,3]dioxine, (2RS,4aSR,9bSR)-2,4a,7-trimethyl-4,4a,5,9b-tetrahydroindeno[1,2-d][1,3]dioxine, (4aRS,9bRS)-4a,7-dimethyl-4,4a,5,9b-tetrahydroindeno[1,2-d][1,3]dioxine, blend of (2RS,4aRS,9bRS)-2,4a,7-trimethyl-4,4a,5,9b-tetrahydroindeno[1,2-d][1,3]dioxine + (2RS,4aSR,9bSR)-2,4a,7-trimethyl-4,4a,5,9b-tetrahydroindeno[1,2-d][1,3]dioxine, (2RS,4aSR,9bSR)-2,4a-dimethyl-4,4a,5,9b-tetrahydroindeno[1,2-d][1,3]dioxin, (4aRS,9bRS)-2,2,4a-trimethyl-4,4a,5,9b-tetrahydroindeno[1,2-d][1,3]dioxine, blend of (2RS,4aSR,9bRS)-2-methyl-4,4a,5,9b-tetrahydroindeno[1,2-d][1,3]dioxin + (2RS,4aRS,9bSR)-2-methyl-4,4a,5,9b-tetrahydroindeno[1,2-d][1,3]dioxin, blend of (2RS,4aRS,9bSR)-2-ethyl-4,4a,5,9b-tetrahydroindeno[1,2-d][1,3]dioxine + (2RS,4aSR,9bRS)-2-ethyl-4,4a,5,9b-tetrahydroindeno[1,2-d][1,3]dioxine, (4aRS,9bSR)-2,2-dimethyl-4,4a,5,9b-tetrahydroindeno[1,2-d][1,3]dioxin, blend of (2RS,4aRS,9bSR)-2,8-dimethyl-4,4a,5,9b-tetrahydroindeno[1,2-d][1,3]dioxine + (2RS,4aSR,9bRS)-2,8-dimethyl-4,4a,5,9b-tetrahydroindeno[1,2-d][1,3]dioxine, blend of (2RS,4aRS,9bSR)-2-ethyl-8-methyl-4,4a,5,9b-tetrahydroindeno[1,2-d][1,3]dioxin + (2RS,4aSR,9bRS)-2-ethyl-8-methyl-4,4a,5,9b-tetrahydroindeno[1,2-d][1,3]dioxin, (4aRS,9bSR)-2,2,8-trimethyl-4,4a,5,9b-tetrahydroindeno[1,2-d][1,3]dioxin, (2R,4aS,9bS)-2,4a,8-trimethyl-4,4a,5,9b-tetrahydroindeno[1,2-d][1,3]dioxin, (2RS,4RS,4ARS,9BSR)-2,4-dimethyl-4,4a,5,9b-tetrahydroindeno[1,2-d][1,3]dioxine, (2RS,4RS,4ASR,9BRS)-2,4-dimethyl-4,4a,5,9b-tetrahydroindeno[1,2-d][1,3]dioxine, blend of (2RS,4aRS,9bSR)-2-methyl-4,4a,5,9b-tetrahydroindeno[1,2-d][1,3]dioxin + (2RS,4aSR,9bRS)-2-methyl-4,4a,5,9b-tetrahydroindeno[1,2-d][1,3]dioxin, (4aRS,9bSR)-2,2-dimethyl-4,4a,5,9b-tetrahydroindeno[1,2-d][1,3]dioxin, (4aRS,9bRS)-4a,8-dimethyl-4,4a,5,9b-tetrahydroindeno[1,2-d][1,3]dioxin, (4aRS,9bRS)-2,2,4a,8-tetramethyl-4,4a,5,9b-tetrahydroindeno[1,2-d][1,3]dioxine, (4aRS,9bRS)-2,2,4a,7-tetramethyl-4,4a,5,9b-tetrahydroindeno[1,2-d][1,3]dioxine, (2RS,4aSR,9bSR)-2,4a-dimethyl-4,4a,5,9b-tetrahydroindeno[1,2-d][1,3]dioxin or a blend of (2RS,4aRS,9bSR)-2-ethyl-8-methyl-4,4a,5,9b-tetrahydroindeno[1,2-d][1,3]dioxin + (2RS,4aSR,9bRS)-2-ethyl-8-methyl-4,4a,5,9b-tetrahydroindeno[1,2-d][1,3]dioxin.

In a particular embodiment, the arthropod, preferably insect, control composition comprisies (2,5-dimethyl-2,3-dihydro-1H-inden-2yl)methanol, 2-methyl-1,3-dihydroinden-2-yl)methanol, 5-(tert-butyl)-1,3-dihydrospiro[indene-2,3'-oxetane], 1-(2,5-dimethyl-2-indanyl)-1-ethanol, (2,5-dimethyl-2,3-dihydro-1H-inden-2yl)methanol, (+-)-(2,4,6-trimethyl-2,3-dihydro-1H-inden-2-yl)methanol, 4A,8-dimethyl-indano[1,2-D]-1,3-dioxan-2-one, (+-)-(2,6-dimethyl-1,2,3,4-tetrahydro-2-naphthalenyl)methanol, (+-)-1-(2,5-dimethyl-2,3-dihydro-1H-inden-2-yl)ethanone, (+-)-(5-methyl-2,3-dihydro-1H-inden-2-yl)methanol, (+-)-1-(2,4,5-trimethyl-2,3-dihydro-1H-inden-2-yl)ethanone, 2,3-dihydro-1H-inden-2-ylmethanol, (4aRS,9bSR)-8-methyl-4,4a,5,9b-tetrahydroindeno[1,2-d][1,3]dioxin, 2,3-dihydro-1H-indene-2-carbonitrile, blend of (+-)-2,4-dimethyl-4,4a,5,9b-tetrahydroindeno[1,2-d][1,3]dioxine + (+-)-2,4-dimethyl-4,4a,5,9b-tetrahydroindeno[1,2-d][1,3]dioxine, 4,4A,5,9B-tetrahydro-indeno[1,2-D]-1,3-dioxin, (4aRS,9bRS)-4a,7-dimethyl-4,4a,5,9b-tetrahydroindeno[1,2-d][1,3]dioxine, blend of (2RS,4aRS,9bRS)-2,4a,7-trimethyl-4,4a,5,9b-tetrahydroindeno[1,2-d][1,3]dioxine & (2RS,4aSR,9bSR)-2,4a,7-trimethyl-4,4a,5,9b-tetrahydroindeno[1,2-d][1,3]dioxine, (4aRS,9bRS)-2,2,4a-trimethyl-4,4a,5,9b-tetrahydroindeno[1,2-d][1,3]dioxine, blend of (2RS,4aRS,9bSR)-2-ethyl-4,4a,5,9b-tetrahydroindeno[1,2-d][1,3]dioxine + (2RS,4aSR,9bRS)-2-ethyl-4,4a,5,9b-tetrahydroindeno[1,2-d][1,3]dioxine, blend of (2RS,4aRS,9bSR)-2,8-dimethyl-4,4a,5,9b-tetrahydroindeno[1,2-d][1,3]dioxine + (2RS,4aSR,9bRS)-2,8-dimethyl-4,4a,5,9b-tetrahydroindeno[1,2-d][1,3]dioxine, (2R,4aS,9bS)-2,4a,8-trimethyl-4,4a,5,9b-tetrahydroindeno[1,2-d][1,3]dioxin, {2RS,4RS,4ARS,9BSR)-2,4-dimethyl-4,4a,5,9b-tetrahydroindeno[1,2-d][1,3]dioxine, (2RS,4RS,4ASR,9BRS)-2,4-dimethyl-4,4a,5,9b-tetrahydroindeno[1,2-d][1,3]dioxine, blend of (2RS,4aRS,9bSR)-2-methyl-4,4a,5,9b-tetrahydroindeno[1,2-d][1,3]dioxin + (2RS,4aSR,9bRS)-2-methyl-4,4a,5,9b-tetrahydroindeno[1,2-d][1,3]dioxin, (4aRS,9bSR)-2,2-dimethyl-4,4a,5,9b-tetrahydroindeno[1,2-d][1,3]dioxin, (4aRS,9bRS)-4a,8-dimethyl-4,4a,5,9b-tetrahydroindeno[1,2-d][1,3]dioxin, (4aRS,9bRS)-2,2,4a,8-tetramethyl-4,4a,5,9b-tetrahydroindeno[1,2-d][1,3]dioxine, (4aRS,9bRS)-2,2,4a,7-tetramethyl-4,4a,5,9b-tetrahydroindeno[1,2-d][1,3]dioxine, (2RS,4aSR,9bSR)-2,4a-dimethyl-4,4a,5,9b-tetrahydroindeno[1,2-d][1,3]dioxin or a blend of (2RS,4aRS,9bSR)-2-ethyl-8-methyl-4,4a,5,9b-tetrahydroindeno[1,2-d][1,3]dioxin + (2RS,4aSR,9bRS)-2-ethyl-8-methyl-4,4a,5,9b-tetrahydroindeno[1,2-d][1,3]dioxin.

In a particular embodiment, the arthropod, preferably insect, control composition comprises (2,5-dimethyl-2,3-dihydro-1H-inden-2yl)methanol, 2-methyl-1,3-dihydroinden-2-yl)methanol, 5-(tert-butyl)-1,3-dihydrospiro[indene-2,3'-oxetane], (+-)-(2,4,6-trimethyl-2,3-dihydro-1H-inden-2-yl)methanol, 4A,8-dimethyl-indano[1,2-D]-1,3-dioxan-2-one, (+-)-1-(2,5-dimethyl-2,3-dihydro-1H-inden-2-yl)ethanone, (+-)-(5-methyl-2,3-dihydro-1 H-inden-2-yl)methanol, 2,3-dihydro-1H-inden-2-ylmethanol, (4aRS,9bSR)-8-methyl-4,4a,5,9b-tetrahydroindeno[1,2-d][1,3]dioxin, 2,3-dihydro-1H-indene-2-carbonitrile, 4,4A,5,9B-tetrahydro-indeno[1,2-D]-1,3-dioxin, (4aRS,9bRS)-4a,7-dimethyl-4,4a,5,9b-tetrahydroindeno[1,2-d][1,3]dioxine, blend of (2RS,4aRS,9bRS)-2,4a,7-trimethyl-4,4a,5,9b-tetrahydroindeno[1,2-d][1,3]dioxine & (2RS,4aSR,9bSR)-2,4a,7-trimethyl-4,4a,5,9b-tetrahydroindeno[1,2-d][1,3]dioxine, blend of (2RS,4aRS,9bSR)-2-ethyl-4,4a,5,9b-tetrahydroindeno[1,2-d][1,3]dioxine + (2RS,4aSR,9bRS)-2-ethyl-4,4a,5,9b-tetrahydroindeno[1,2-d][1,3]dioxine, (2R,4aS,9bS)-2,4a,8-trimethyl-4,4a,5,9b-tetrahydroindeno[1,2-d][1,3]dioxin, (2RS,4RS,4ARS,9BSR)-2,4-dimethyl-4,4a,5,9b-tetrahydroindeno[1,2-d][1,3]dioxine, (2RS,4RS,4ASR,9BRS)-2,4-dimethyl-4,4a,5,9b-tetrahydroindeno[1,2-d][1,3]dioxine, blend of (2RS,4aRS,9bSR)-2-methyl-4,4a,5,9b-tetrahydroindeno[1,2-d][1,3]dioxin + (2RS,4aSR,9bRS)-2-methyl-4,4a,5,9b-tetrahydroindeno[1,2-d][1,3]dioxin & (4aRS,9bSR)-2,2-dimethyl-4,4a,5,9b-tetrahydroindeno[1,2-d][1,3]dioxin.

In a particular embodiment, the arthropod, preferably tick, control composition comprises (2,5-dimethyl-2,3-dihydro-1H-inden-2yl)methanol, (+-)-5-isopropyl-2-methyl-2-indanmethanol, (+-)-(2,4,6-trimethyl-2,3-dihydro-1H-inden-2-yl)methanol, (+-)-(2,6-dimethyl-1,2,3,4-tetrahydro-2-naphthalenyl)methanol, (+-)-(5-methyl-2,3-dihydro-1H-inden-2-yl)methanol, (2-methyl-2,3-dihydro-1H-inden-2-yl)methyl acetate, (4aRS,9bSR)-8-methyl-4,4a,5,9b-tetrahydroindeno[1,2-d][1,3]dioxin, (+-)-2-((methoxymethoxy)methyl)-2,5-dimethyl-2,3-dihydro-1H-indene, (+-)-2-[(1-methoxyethoxy)methyl]-2,5-dimethylindane, 2,3-dihydro-1H-indene-2-carbonitrile, 4,4A,5,9B-tetrahydro-indeno[1,2-D]-1,3-dioxin.

By the expression "hydrocarbon group" it is understood that said group consists of hydrogen and carbon atoms and can be in the form of an aliphatic hydrocarbon, i.e. linear or branched saturated hydrocarbon (e.g. alkyl group), a linear or branched unsaturated hydrocarbon (e.g. alkenyl or alkynyl group), a saturated cyclic hydrocarbon (e.g. cycloalkyl) or an unsaturated cyclic hydrocarbon (e.g. cycloalkenyl), or can be in the form of an aromatic hydrocarbon, i.e. aryl group, or can also be in the form of a mixture of said type of groups, e.g. a specific group may comprise a linear alkyl, a branched alkenyl (e.g. having one or more carbon-carbon double bonds), a (poly)cycloalkyl and an aryl moiety, unless a specific limitation to only one type is mentioned. Similarly, in all the embodiments of the invention, when a group is mentioned as being in the form of one type of saturation or unsaturation, (e.g. alkyl), it is meant that said group can be in any type of topology (e.g. linear, cyclic or branched) or having several moieties with various topologies.

By the expression "optionally substituted" it is understood that the optionally substituted group can be substituted or cannot be substituted with the corresponding substituents.

The term "arthropod" has the normal meaning for a skilled person in the technical field. Arthropods include invertebrate animals, such as insects, arachnids, and crustaceans, that have a segmented body and jointed appendages. Arthropods usually have a chitinous exoskeleton molted at intervals, and a dorsal anterior brain connected to a ventral chain of ganglia.

Arthropods in the present invention's understanding relate to undesired arthropods, meaning that their presence in the air, on the surface of an article, the surface of a plant or the surface of an vertebrate, such as a human subject or other mammal, preferably human subject, is not desired. Preferably undesired arthropods are pest arthropods that impact plants and animals, e.g. thrips, aphids, beetles, moth, mealybug, scale etc., more preferably pest arthropods that impact animals, e.g. ants, termites, cockroaches, flies, etc., even more preferably blood feeding arthropods that impact vertebrates, e.g. biting fly, bed bug, kissing bug, flea, lice, mosquitos and ticks, even more preferably mosquitos and ticks.

The reason why the presence of an arthropod is not desired might be that the arthropod's presence in the air is unpleasant to a subject, the contact of an arthropod on an article transfers diseases and/or germs or the arthropod bites an organism and causes itching, the transmission of diseases and/or germs or the arthropod feeding may be the cause for other diseases and/or conditions.

In a particular embodiment, the arthropod is an insect or an arachnid, preferably an insect.

The term "insect" has the normal meaning for a skilled person in the technical field. An insect is described by a well-defined head, thorax, and abdomen, only three pairs of legs, and typically one or two pairs of wings.

In a particular embodiment, the insect is a mosquito, biting fly, bedbug, kissing bug, flea, lice, ant, termite, cockroach, fly, aphid, beetle, thrips, moths, mealybug or scale bug, more preferably a mosquito.

The term "arachnid" has the normal meaning for a skilled person in the technical field. An arachnid is described having a segmented body divided into two regions of which the anterior bears four pairs of legs but no antennae.

In a particular embodiment, the arachnid is a tick, mite, chigger or spider, more preferably a tick.

The expression "control", "arthropod control", "insect control" or "arachnid control" or the like has the normal meaning for a skilled person in the technical field.

"Controlling" in the context of the present invention defines the ability of a compound according to formula (I) or an arthropod controlling composition according to the present invention to attract, deter, kill or repel an arthropod, preferably deter or repel an arthropod and even more preferably repel an arthropod.

"Attracting" according to the present invention defines the ability of a compound of formula (I) or an arthropod attractant composition according to the invention to increase or encourage contact or the presence of an arthropod at the arthropod attractant source, such as in the air, on the surface of an article or on the surface of an vertebrate, such as a human subject or other mammal, preferably an article such as a trapping device, the arthropod attractant compound or composition has been applied to.

"Deterring" according to the present invention defines the ability of a compound of formula (I) or an arthropod deterrent composition according to the invention to minimize, reduce, discourage or prevent contact or the presence of an arthropod at the arthropod deterrent source, such as in the air, on the surface of an article or on the surface of an vertebrate, such as a human subject or other mammal, preferably human subject, to which the arthropod deterrent compound or composition has been applied to. Typically, the deterrent effect is shown when used as feeding deterrent hindering a pest from subsequent food intake or oviposition after an initial tasting of the arthropod deterrent compound or composition.

"Killing" according to the present invention defines the ability of a compound of formula (I) or an art report killing composition according to the present invention to kill an arthropod at the arthropod killing source, such as in the air, on the surface of an article or on the surface of an vertebrate, such as a human subject or other mammal, preferably human subject, to which the arthropod killing compound or composition has been applied to. When an arthropod killing composition is applied to a plant, an animal or human subject, it is applied in an amount which is killing to the arthropod but not to the subject.

"Repellency" according to the present invention defines the ability of a compound of formula (I) or an arthropod repellent composition according to the present invention to minimize, reduce, discourage or prevent approach or the presence of an arthropod at the arthropod repellent source, such as in the air, on the surface of an article or on the surface of an vertebrate, such as a human subject or other mammal, preferably human subject, to which the arthropod repellent compound or composition has been applied to.

In a particular embodiment, the arthropod controlling source is the surface and/or the air in the vicinity of an article, preferably a candle, coil, electric diffuser, wristband, patch, collar, ear tag, clothes, fabrics, papers, biochar, cardboard, cellulosic pads, bed nets, screen, curtains, furniture, walls, ground or paint, or the surface of a subject, preferably the surface of a an vertebrate, such as a human subject or other mammal, preferably human subject, i.e. the skin of a human subject treated with a product, such as spray, aerosol, cream, roll on, wristband, lotion, soap, shampoo, sunscreen or patch or a cloth treated with a product such as laundry powder, liquid detergent, spray, lotion, powder.

The arthropod controlling effect according to the present invention is determined on mosquitoes using an adapted Warm Body assay as defined in Kröber T, Kessler S, Frei J, Bourquin M, Guerin PM. An in vitro assay for testing mosquito controlling compounds employing a warm body and carbon dioxide as a behavioral activator. J Am Mosq Control Assoc. 2010; 26:381-386.

The controlling effect according to the present invention is determined by testing the Warm Body assay against the yellow fever mosquito, *Aedes aegypti* Rockefeller strain. *A. aegypti* is a model organism for controlling tests and one of the recommended model organisms by the World Health Organization (WHO) as it is a very aggressive, anthropophilic mosquito species that shows generally low sensitivity to arthropod controlling compounds. Observations of controlling efficacy were made on host-seeking females of uniform age, 5 to 10 days old selected as mentioned in the publication mentioned hereinabove. Tested hungry females had access to 10% sugar solution but were not blood-fed.

The published protocol has been adapted in not manually counting the landing mosquitoes but automatically using an automatic counting software, the switch from *Anopheles gambiae* to *A. aegypti* led to a decrease of mosquitoes' number placed in the tested cage due to the size difference *(i.e.* 30 mosquitoes instead of 50) and to an increase of lighting as *A. aegypti* is a diurnal mosquitoes *(i.e.* 150 lux instead of 4 lux).

In addition, by using the *in-vitro* systems mentioned above, it is possible to assess at which concentration all tested arthropods are controlled by the compound according to formula (I) either alone or in a composition.

Moreover, by testing the controlling effect of the compound according to formula (I) either alone or in a composition at different concentrations, it is possible to compare the efficacy at isodose with reference compounds such as DEET or IR3535.

In a particular embodiment, the arthropod control composition according to the invention comprises a compound of formula (I) in an arthropod control effective amount. Said compound can be used alone or in combination with other ingredients of formula (I) and/or with arthropod control co-ingredients. Thereby, it is understood that the concentration of the compound according to formula (I) either alone or in a composition provides a higher repellency compared to pure (i.e. 100%) ethanol.

In a particular embodiment, the composition comprises the compound according to formula (I) in an amount of from 0.02 to 80 wt.%, more preferably in an amount of from 0.05 to 70 wt.%, even more preferably in an amount of from 0.1 to 60 wt.%, based on the total weight of the composition. Thereby, it is understood that the composition preferably comprises the compound according to formula (I) in a minimum amount of at least 0.2 wt.%, at least 0.05 wt.% or at least 0.1 wt.% and a maximum amount of not more than 80 wt.%, not more than 70 wt.% or not more than 60 wt.%, based on the total weight of the composition.

In a particular embodiment, the composition comprises the compound according to formula (I) in an amount of from 0.1 to 5 wt.%, preferably from 0.15 to 4 wt.%, more preferably 0.2 to 3 wt.% or from 11 to 35 wt.%, preferably from 15 to 30 wt.%, more preferably from 18 to 25 wt.%, based on the total weight of the composition. Thereby, it is understood that the composition in one embodiment comprises the compound according to formula (I) in a minimum amount of at least 0.1 wt.%, at least 0.15 wt.% or at least 0.2 wt.% and a maximum amount of not more than 5 wt.%, not more than 4 wt.% or not more than 3 wt.%, based on the total weight of the composition and in another embodiment comprises the compound according to formula (I) in a minimum amount of at least 11 wt.%, at least 15 wt.% or at least 18 wt.% and a maximum amount of not more than 35 wt.%, not more than 30 wt.% or not more than 25 wt.%.

According to a particular embodiment, the arthropod controlling composition according to the present invention, comprises one or more arthropod control co-ingredient(s).

By "arthropod control co-ingredient" is understood an ingredient capable of imparting additional arthropod controlling benefits to the arthropod controlling effect of the compounds of formula (I) described hereinabove.

In one embodiment, the compound of formula (I) is capable to modify, enhance or improve the arthropod controlling effect of the arthropod control co-ingredient, e.g. by reducing the amount of the arthropod control co-ingredient within a composition. This can be particularly beneficial in case the arthropod control co-ingredient is harmful to human subjects at a certain dose or in case the arthropod control co-ingredient has negative olfactive properties at a certain dose.

According to a particular embodiment, the combination of a compound of formula (I) and an arthropod control co-ingredient results in a synergistic arthropod controlling effect.

Arthropod control co-ingredients include N,N-diethyl-3-methylbenzamide (DEET), ethyl butylacetylaminopropionate (IR3535), (13Z)-Hexadec-13-en-11-yn-1-yl acetate, (2E)-3,7-Dimethylocta-2,6-dien-1-ol, (Z,E)-tetradeca-9,12-dienyl acetate, 2-Hydroxybutanedioic acid, 2-Hydroxypropane-1,2,3-tricarboxylic acid, 2-Hydroxy-α,α,4-trimethylcyclohexanemethanol, 2-Phenylethyl propionate, 4-Allyl-2-methoxyphenol, Acide lactique, Capsicum annuum, ext, Capsicum oleoresin, Castor oil, Cedarwood oil (China), Cedarwood oil (Texas), Cedarwood oil (Virginia), Chrysanthemum cinerariaefolium extract from open and mature flowers of Tanacetum cinerariifolium obtained with supercritical carbondioxide, Chrysanthemum cinerariaefolium, extract from open and mature flowers of Tanacetum cinerariifolium obtained with hydrocarbon solvents, Cinnamon, Cinnamon oil, Citric acid, Citronella, Citronella oil, Clove oil, Cloves, Corn oil, Cornmint, Cornmint oil, Cottonseed oil, Cymbopogon winterianus oil, fractionated, hydrated, cyclized, Decanoic acid, Ethyl butylacetylaminopropionate (IR3535), Eucalyptus citriodora oil and citronellal, hydrated, cyclized, Eucalyptus citriodora oil, hydrated, cyclized, Eugenol, Fulvic acid, Garlic, Garlic oil, Garlic, ext., Geraniol, Geranium oil, Lauryl sulfate, Lavender, Lavandula hybrida, ext./Lavandin oil, Lemon oil, Lemongrass oil, Linseed oil, Malic acid, Margosa extract from cold-pressed oil of the kernels of Azadirachta Indica extracted with super-critical carbon dioxide, Mentha arvensis, ext., methyl nonyl ketone, Metofluthrin, Mixture of cis- and trans-p-menthane-3,8 diol, para-menthan-3,8-diol (PMD), N,N-diethyl-meta-toluamide, Nonanoic acid, Orange, sweet, ext., Pepper (Piper), P. nigrum, ext., Peppermint, Peppermint oil, Potassium (2E,4E)-hexa-2,4- dienoate, Potassium sorbate, Pyrethrins and Pyrethroids, Rosemary, Rosemary oil, 1-(1-methylpropoxycarbonyl)-2-(2-hydroxaethyl)piperidine (Icaridine), Sesame, Sesame oil, Sodium Acetate, Sodium Benzoate, Sodium chloride, Sodium lauryl sulfate, Soybean oil, Spearmint, Spearmint oil, Sulfuric acid monododecyl ester, sodium salt, Thyme, Thyme oil, White pepper, Zinc, oil of wintergreen, (1H-indol-2-yl)acetic acid, (2,5-dioxo-4-imidazolidinyl) urea, (2E)-3,7-dimethylocta-2,6-dienoic acid, (2-isopropenyl-1-methyl-cyclobutyl)-acetic acid, (4-tert-butyl-phenyl)-acetic acid methyl ester, (E)-2-methylbut-2-enoic acid, (E)-but-2-enoic acid, (E)-pent-2-enal, (E,E)-2,4-Hexadienal, (Z)-2-Hexanol, (Z)-3-Hexen-1-ol, [(2E)-3,7-dimethylocta-2,6-dienyl] formate, [(E)-3-phenylprop-2-enyl]formate, [hydroxy-(4-hydroxy-quinolin-2-yl)-methylene]-methyl-oxonium, 1-(1H-pyrrol-2-yl)ethanone, 1-(2,6,6-trimethyl-cyclohex-2-enyl)-hepta-1,6-dien-3-one, 1-(3,5,5,6,8,8-hexamethyl-5,6,7,8-tetrahydro-naphthalen-2-yl)-ethanone, 1-(3,8-dimethyl-1,2,3,4,5,6,7,8-octahydro-azulen-5-yl)-1-methyl-ethyl ester acetic acid, 1,2-dihydro-[1,2,4]triazol-3-one, 1,2-epoxyhexane, 1,3,7-trimethyl-3,7-dihydro-purine-2,6-dione, 1,3-dimethyl-1H-pyrimidine-2,4-dione, 1,3-dimethyl-1H-quinazoline-2,4-dione, 1,3-dinitro-imidazolidin-2-one, 10-isopropylidene-3,7-dimethyl-cyclodeca-3,7-dienone, 10-undecen-1-al, 14-nitrooxy-tetradec-5-ene, 1-ethoxy-1- (2'-phenylethoxy) ethane, 1-ethyl-2-pyrrolidone, 1-hepten-3-ol, 1-Hexanol, 1-hexen-3-ol, 1-methyl cyclohexene, 1-methyl-1,3-cyclohexadiene, 1-methyl-1,4-cyclohexadiene, 1-methylpyrrolidin-2-one, 1-phenylbutan-1-one, 2-phenoxyethanol, 2-(trimethylazaniumyl)acetate, 2,2-dimethyl-3-(2-methyl-propenyl)-cyclopropanecarboxylic acid ethyl ester, 2,3-butanedione, 2-amino-3-(3,4-dihydroxy-phenyl)-propionic acid, 2-amino-3-phenyl-propionic acid, 2-amino-5-guanidino-pentanoic acid, 2-amino-succinamic acid, 2-butyl-4,6-dimethyl-5,6-dihydro-2H-pyran, 2-carbamoyl-benzoic acid anion, 2-ethyl hexanediol-1,3, 2-ethyl-1,3-hexanediol, 2-ethyl-1,3-hexanediol (Rutgers 612), 2-ethyl-2-butyl-1,3-propanediol, 2-hexanol, 2-hydroxy-2-(1-hydroxy-ethyl)-3-methyl-butyric acid 7-hydroxy-5,6,7,7a-tetrahydro-3H-pyrrolizin-1-ylmethyl ester, 2-hydroxy-2-(1-methoxyethyl)-3-methyl-butyric acid 7-hydroxy-5,6,7,7a-tetrahydro-3H-pyrrolizin-1-ylmethyl ester, 2-hydroxy-4,4,6-trimethyl-cyclohexa-2,5-dienone, 2-hydroxymethyl-but-2-enoic acid 7-(2-methyl-but-2-enoyloxy)-5,6,7,7a-tetrahydro-3H-pyrrolizin-1-ylmethyl ester, 2-hydroxymethyl-but-2-enoic acid 7-(2-methyl-but-2-enoyloxy)-5-,6,7,7a-tetrahydro-3H-pyrrolizin-1-ylmethyl ester, 2-isobutyl-4-methyl-1,3-dioxolane, 2-methyl tetrahydrofuran, 2-methyl-4-methylidene-6-phenyloxane, 2-methyl-butyric acid 6,6-dimethyl-bicyclo[3.1.1]hept-2-en-2-ylmethyl ester, 2-methylpropyl formate, 2-methylpropyl-(Z)-but-2-enoate, 2-methylquinoline, 2-oxobutanoic acid, 2-oxopentanoic acid, 2-oxopropanal, 2-phenylcyclohexanol, 2-phenylethyl (E)-2-methylbut-2-enoate, 2-phenylethyl 3-methylbutanoate, 2-phenylethyl butanoate, 2-phenylethyl propanoate, 3,3,6-trimethyl-hepta-1,5-dien-4-one, 3,4-Dihydro-2H-pyran, 3,4-dimethylcyclopentane-1,2-dione, 3,5,5-trimethyl-4-(3-oxo-but-1-enyl)-cyclohex-3-enone, 3-ethyl-2-hydroxycyclopent-2-en-1-one, 3H-benzothiazol-2-one, 3-hexanol, 3-hydroxybutan-2-one, 3-hydroxymethyl-6-methyl-3a,3b,7a,8-tetrahydro-1H-4-oxa-8a-aza-cyclopenta[a]inden-5-one, 3-methyl cyclohexene, 3-Methyl-1-Butanol, 3-methyl-4-(2,6,6-trimethyl-cyclohex-2-enyl)-but-3-en-2-one, 3-methylbut-2-enoic acid, 3-methylcyclopent-2-en-1-one, 3-methylcyclopentane-1,2-dione, 4-(1-hydroxy-4,7-dimethyl-1,4a,5,6,7,7a-hexahydro-cyclopenta[c]pyran-3-yl)-but-3-en-2-one, 4-(2,5,6,6-tetramethyl-cyclohex-2-enyl)-but-3-en-2-one, 4,5-epoxy-(E)-2-decenal, 4,8-dimethyl-octahydro-chromen-2-one, 4-amino-5-(3-(isopropyl amino)-2,2-dimethyl-3-oxopropoxy)-2-methyl quinoline-3-carboxylic acid, 4-ethylbenzaldehyde, 4-ethylidene-7-hydroxy-6,7,14-trimethyl-2,9-dioxa-14-azabicyclo[9.5.1]heptadec-11-ene-3,8,17-trione, 4-hydroxy-2,3-dimethyl-2H-furan-5-one, 4-hydroxy-2,5-dimethyl-3(2H)-furanone, 4-hydroxy-3,7-dimethyl-octahydro-chromen-2-one, 4-methyl cyclohexene, 5,5-diethyl-pyrimidine-2,4,6-trione, 6,6,9a-tetramethyl-naphthaldehyde [2,1-b] furan, 6] decylidene -8) butanal, 6-methoxy-3H-benzooxazol-2-one, 6-methoxyquinoline, 6-methyl quinolone, 6-methyl-2-(3-methyl-cyclohex-3-enyl)-hept-5-en-2-ol, 7,8-dihydro-1-biopterin, 7,8-dimethyl-8-aza-bicyclo[3.2.1]octane-2-carboxylic acid methyl ester, 7,9-dihydro-3H-purine-2,6,8-trione, Acetic Acid, acetic acid 1-[2-(3,3-dimethyl-oxiranyl)-ethyl]-1-methyl-allyl ester, acetic acid 2,2,6-trimethyl-6-vinyl-tetrahydro-pyran-4-yl ester, acetic acid 2,6,10,10-tetramethyl-1-oxa-spiro[4.5]dec-6-yl ester, acetic acid 2-isopropylidene-4,8-dimethyl-1,2,3,3a,4,5,6,8a-octahydro-azulen-6-yl ester, Acetophenone, acetyl cedrene, Acide (+)-tartrique,isopropyl alcohol, aldehyde C11 (undecylenic aldehyde), aldehyde iso C11 (GIV), allspice oil, allyl butyrate, allyl cyclohexyl propionate, allyl propionate, almond bitter oil, alpha cedrene epoxies, alpha-angelica lactone, amyl cinnamaldehyde, amyl salicylate, anethole, anise alcohol, anise oil, anisic aldehyde, methyl anthranilate, Applinal, ascorbic acid, α-Terpinene, basil oil, bay oil, benzil, benzyl benzoate, benzoin gum, benzyl 3-oxobutanoate, benzyl acetate, benzyl benzoate, benzyl cinnamate, benzyl formate, benzyl propionate, benzyl salicylate, Boolean Geonaru (bourgeonal), Brahmanol (Brahmanol), butanal, butoxypolypropylene glycol, camphor, caraway oil, carbamic acid ethyl ester, carbinol, cardamom oil, cedar barge anions (Cedarwood Virginion), Cedar oil, Cedrenol, celery oil, chamomile oil, cinnamic alcohol,Cinnamomum spp. oil, cinnamon leaf oil, cinnamyl acetate, cis-3- hexenol, citronellyl acetate, citronellyloxyacetaldehyde, coriander oil, cumin oil, cyclamen aldehyde, cyclohexanol, cyclohexene, cypress genus oil, delta-3-Carene, decanal, dibenzyl ether, dibutyl adipate, dihydromyrcenol, dihydropyran, dill oil, dimethyl carbate, dimethyl carbate (endo,endo)-dimethyl bicyclo[2.2.1 ]hept-5-ene-2,3-dicarboxylate), dimethyl carbonate, dimethylbenzyl carbinyl acetate, di-normal-butyl succinate, di-normal-butyl succinate (Tabatrex), di-normal-propyl isocinchomeronate, di-normal-propyl isocinchomeronate (MGK Repellent 326), diphenyl oxide, diphenylmethane, Dipropyldisulfide, Dodecanal, dodecanoic acid, dodecanol, E-2-Hexenal, elemi oil, ethyl (E)-but-2-enoate, ethyl 2-oxopropanoate, ethyl acetate, Ethyl Butyrate, ethyl cinnamate, ethyl cyclopentenolone, ethyl formate, ethyl methyl phenyl glycidate, ethyl pyruvate, ethyl Safra sulphonate (Ethyl safranate), ethyl salicylate, evergreen oils (pine oil, fennel oil, floral methanol, furanone acetate, galbanum, geranyl acetate, geranyl formate, ginger oil, glutamine, grapefruit oil, heliotropin, Herukorin (Hercolyn), hexanal, hexanol, hexyl benzoate, hexyl cinnamaldehyde, Huon Pine (Huon pine) oil, Hydra Tropic ( Hydratropic) dimethyl acetal, hydroxy citronellal dimethyl acetal, Icool, indolo[2,1-b]quinazoline-6,12-dione, iso cyclo citral, isobornyl acetate (iso Bornyl asetate), Isobutyl Acetate, isobutyl formate, isobutyl quinolone, isobutyric acid 1-methyl-1-(4-methyl-cyclohex-3-enyl)ethyl ester, isobutyric acid 6,6-dimethyl-bicyclo[3.1.1]hept-2-en-2ylmethyl ester, isolongifolol Hol Anon, isopropyl myristate, isopropyl quinoline, jasmine oil, Jass machine Clen (Jasmacyclene), Jimikuretoru (Dimycretol), Karudamido (Cardamide), lactic acid, Lauric acid, Lavandin Abrialis, Lee Doll, Limonene (-), Limonene (+), linalyl acetate, mandelonitrile benzoate, Masearu (Maceal) (Q), Mechiruio non alpha iso (methyl ionone alpha iso), menthol La Evo (menthol Laevo), menthyl 2-pyrrolidone-5-carboxylate, methyl 2-(4-tert-butylphenyl)acetate, methyl 2-methylprop-2-enoate, methyl 2-methylpropanoate, methyl 2-oxopropanoate, methyl acetate, methyl butanoate, methyl butyrate, methyl cedryl ketone, methyl dihydro jasmonate, methyl ionone, methyl isobutyrate, methyl methacrylate, methyl myristate, methyl N-acetylisoleucinate, methyl naphthyl ketone, methyl propanoate, methyl propionate, methyl pyruvate, methyl salicylate, methyl salicylate Mosutsurimosu (Moss treemoss), methyl nonyl ketone, musk ketone, N-(2-formyl-phenyl)-formamide, N-(3-methylbutyl)-benzamide, N-(tert-butyl)-4-methylbenzamide, N,N-diethyl-4-methyl-benzamide, N-butyl-3-fluoro-benzamide, N-butylacetanilide, N-diethylbenzamide, Neokasupiren (Neocaspirene), Nero phosphorus bromide Melia (Nerolin Bromelia), nerol, nerol oxide, neryl acetate, Nonanal, normal-butyl-6,6-dimethyl-5,6-dihydro-1,4-pyrone-2-carboxylate, normal-butyl-6,6-dimethyl-5,6-dihydro-1,4-pyrone-2-carboxylate (Indalone), normal-butylmesityl oxide oxalate, normal-propyl N,N-diethylsuccinamate, oak moss absolute (Oakmoss absolute), oct-1-en-3-yl butanoate, Octanal, octanoic acid, octanol Oliva Nam (Octanol Olibanum) resinoids, oxolan-2-ylmethyl acetate, oxyquinoline, para - cresyl phenyl acetate, para - methoxyacetophenone, para - tertiary - butyl - cyclohexyl acetate, parsley oil, patchouli oil, Pechigurein oil (Petitgrain oil), Pelargonic acid, pentanal, pentanedione, pentanoic acid 5,5,6-trimethyl-bicyclo[2.2.1]hept-2-yl ester, pentanol, Pentyl Acetate, pepper oil, Peru balsam, phenoxyethyl isobutyrate, phenyl ethyl acetate, phenyl ethyl alcohol, phenyl ethyl butyrate, phenyl ethyl phenyl acetate, phenylalanine, pimento oil, prop-2-enyl-butanoate, prop-2-enyl-propanoate, propan-2-yl benzoate, phenylethyl propionate, propionic acid, propionic acid 5-isopropenyl-2-methyl-cyclohex-2-enyl ester, propyl acetate, propyl formate, propyl salicylate, propylidene phthalide, purine-2,6-dione, quinmerac, Quinn aldehyde (Cuinic aldehyde), resinoid benzoin Shi am (Resinoid Benzoin Siam), rose oil, safrole, sandalwood oil, Sedorenoru, Sedorirua Seteto, Seresutorido, sweet orange oil, synthetic camphor powder, Terpinene, terpineol, Terpinolene, tetrahydro linalool, thyme red, tolu balsam, tryptophan, turmeric oil, undecanal, vanillin, verbena oil, vetiver, yara or ylang ylang, (1,3,4,5,6,7-hexahydro-1,3-dioxo-2H-isoindol-2-yl)methyl (1R-trans)-2,2-dimethyl-3-(2-methylprop-1-enyl)cyclopropanecarboxylate (d-Tetramethrin), (E)-1-(2-Chloro-1,3-thiazol-5-ylmethyl)-3- methyl-2-nitroguanidine (Clothianidin), (RS)-3-Allyl-2-methyl-4-oxocyclopent-2-enyl (1R,3R)-2,2-dimethyl-3-(2-methyl prop-1-enyl)-cyclopropanecarboxylate (mixture of 2 isomers 1R trans: 1R/S only 1:3) (Esbiothrin), (RS)-3-Allyl-2-methyl-4-oxocyclopent-2- enyl-(1R,3R;1R,3S)-2,2-dimethyl-3-(2- methylprop-1-enyl)-cyclopropanecarboxy late (mixture of 4 isomers 1R trans, 1R:1R trans, 1S: 1R cis, 1R: 1R cis,1S 4:4:1:1) (d-Allethrin), (RS)-α-cyano-3phenoxybenzyl-(1RS)-cis, trans-3-(2,2-dichlorovinyl)-2,2-dimethylcyclopropanecarboxylate (Cypermethrin), .alpha.-cyano-3-phenoxybenzyl2,2-dimethyl-3-(2-methylprop-1-enyl)cyclopropanecarboxylate (Cyphenothrin), .alpha.-cyano-4-fluoro-3-phenoxybenzyl3-(2,2-dichlorovinyl)-2,2-dimethylcyclopropanecarboxylate (Cyfluthrin), [1.alpha.(S*),3.alpha.]-(.alpha.)-cyano-(3-phenoxyphenyl)methyl3-(2,2-dichlor-oethenyl)-2,2-dichlorovinyl)-2,2-dimethyl-cyclopropanecarboxylate (alpha-Cypermethrin), [2,4-Dioxo-(2-propyn-1-yl)imidazolidin-3-yl]methyl(1R)-cis-chrysanthemate;[2,4- Dioxo-(2-propyn-1-yl)imidazolidin-3-yl] methyl(1R)-trans-chrysanthemate (Imiprothrin), 1-(3,5-dichloro-4-(1,1,2,2-tetrafluoroethoxy)phenyl)-3-(2,6-difluorobenzoyl) urea (Hexaflumuron), 1-ethynyl-2-methylpent-2-enyl 2,2-dimethyl-3-(2-methylprop-1-enyl)cyclopropanecarboxylate (Empenthrin), 1R-trans phenothrin, 2-(1-Methylpropyl)phenyl methylcarbamate, 2-(2-butoxyethoxy)ethyl 6-propylpiperonyl ether (Piperonyl butoxide), 2-chloro-N-[[[4-(trif luoromethoxy) phenyl]amino]carbonyl]benzamide (Triflumuron), 2-methyl-4-oxo-3-(prop-2-ynyl)cyclopent-2-en-1-yl 2,2-dimethyl-3-(2-methylprop-1-enyl)cyclopropanecarboxylate (Prallethrin), 3-phenoxybenzyl (1R)-cis,trans-2,2- dimethyl-3-(2-methylprop-1-enyl)cyclopropanecarboxylate (d-Phenothrin), 4-bromo-2-(4-chlorophenyl)-1-ethoxy methyl-5-trifluoromethylpyrrole-3-carbonitrile (Chlorfenapyr), Abamectin, Acephate, Acetamiprid, Aldicarb, Aldrin, Aluminium phosphide releasing phosphine, Anabasine, Anethole, Annonin, Asimina, Azadirachtin, Azinphos-methyl, Bendiocarb, Bensulide, Bifenthrin, Borate, Borax, Boric Acid, Caffeine, Carapa, Carbaryl, Carbofuran, Chlorantraniliprole, Chlordane, Chlordecone, Chlorethoxyfos, Chlorpyrifos, Chlorpyriphos-methyl, Chrysanthemum cinerariaefolium extract from open and mature flowers of Tanacetum cinerariifolium obtained with supercritical carbondioxide, Chrysanthemum cinerariaefolium, ext., Chrysanthemum cinerariaefolium, extract from open and mature flowers of Tanacetum cinerariifolium obtained with hydrocarbon solvents, Cinnamaldehyde, Cinnamon leaf oil, Cinnamyl acetate, Citral, Clothianidin, Cyanamide, Cyantraniliprole, Cyfluthrin, Cyhalothrin, Lambda-, Cyromazine, DDT, Decanoic acid, Deguelin, deltamethrin, Derris, Desmodium caudatum, Diazinon, Dichlorvos, Dicrotophos, Dieldrin, diflubenzuron, Dimethoate, Dinotefuran, Dioxacarb, Disulfoton, Endosulfan, Endrin, epsilon-Momfluorothrin, (S)-.alpha.-Cyano-3-phenox ybenzyl (S)-2-(4-chlorophenyl)-3-methylbutyrate (Esfenvalerate), Ethoprop, etofenprox, Etofenprox, Eugenol, Fenamiphos, Fenitrothion, Fenobucarb, Fenoxycarb, Fenthion, Fenvalerate, fipronil, Flubendiamide, Flufenoxuron, Fosthiazate, hexachlorocyclohexane, gamma-, Heptachlor, Hexachlorobenzene, hydrogen cyanide, Hydroprene, imidacloprid, Isoprocarb, lambda-cyhalothrin, Linalool, Magnesium phosphide releasing phosphine, Malathion, Maltodextrin, Margosa extract from cold-pressed oil of the kernels of Azadirachta Indica extracted with super-critical carbon dioxide, Margosa extract from the kernels of Azadirachta Indica extracted with water and further processed with organic solvents, Methamidophos, Methidathion, Methomyl, Methoprene, Methoxychlor, Metofluthrin, Mevinphos, Mirex, Monocrotophos, Myristicin, Naled, N-cyclopropyl-1,3,5-triazine-2,4,6-triamine (Cyromazine), Nicotine, Nithiazine, Nitrogen, Octanoic acid, Omethoate, Oregano oil, Oxydemeton-methyl, Parathion, Parathion-methyl, Pentachlorophenol, Permethrin, Phorate, Phosalone, Phosmet, Phostebupirim, Phoxim, Pirimiphos-methyl, Polyketide, Profenofos, Pyrethrins and Pyrethroids, Pyrethrum, pyriproxyfen, Pyrogenic, synthetic amorphous, nano, surface treated silicon dioxide, Quassia spp., Resmethrin, rotenone, Ryanodine, S-[(6-chloro-2-oxooxazolo[4,5-b]pyridin-3(2H)-yl)methyl] O,O-dimethylthiophosphate (Azamethiphos), Silicic acid, aluminium magnesium sodium salt, Silicium dioxide (Silicium dioxide/Kieselguhr), S-Methoprene, Sodium dimethylarsinate (Sodium Cacodylate), Spinosad (Spinosyn A), Spinosyn D, Substance name, sulfuryl fluoride, Synthetic amorphous silicon dioxide (nano), Tebufenozide, Terbufos, Tetrachlorvinphos, Tetramethrin, Tetranortriterpenoid, Thiacloprid, thiamethoxam, Thymol, Tralomethrin, Transfluthrin, Tribufos, Trichlorfon, and mixtures thereof.

According to one embodiment arthropod control co-ingredient is selected from the group consisting of N,N-diethyl-3-methylbenzamide (DEET), ethyl butylacetylaminopropionate (IR3535); para-menthan-3,8-diol (PMD); 1-(1-methylpropoxycarbonyl)-2-(2-hydroxaethyl)piperidin (Icaridin); Cedarwood oil (China), Cedarwood oil (Texas), Cedarwood oil (Virginia), Cinnamon oil, Citronella oil, Clove oil, Cornmint oil, Cymbopogon winterianus oil, fractionated, hydrated, cyclized, Decanoic acid, Eucalyptus citriodora oil and citronellal, hydrated, cyclized, Eucalyptus citriodora oil, hydrated, cyclized, Eugenol, Garlic oil, Geraniol, Geranium oil, Lavender, Lavandula hybrida, ext./Lavandin oil, Lemon oil, Lemongrass oil, Margosa extract, Mentha arvensis, ext., Metofluthrin, Mixture of cis- and trans-p-menthane-3,8 diol, N,N-diethyl-meta-toluamide, Nonanoic acid, Peppermint oil, Pyrethrins and Pyrethroids, Rosemary oil, Spearmint oil, Thyme oil, Wintergreen oil, 2,3,4,5-bis(butyl-2-ene)tetrahydrofurfural (MGK Repellent 11), cineole, cinnamaldehyde, citral, Citronellal, citronellol, Citronella oil Java, coumarin, dibutyl phthalate, diethyl phthalate, dimethyl anthranilate, dimethyl phthalate, ethyl vanillin, eucalyptus oil, delta - octalactone, delta - nonalactone, delta - decalactone, delta - undecalactone , delta - dodecalactone, gamma - octalactone, gamma - nonalactone, gamma - decalactone, gamma - undecalactone , gamma - dodecalactone, hydroxy citronellal, lime oil, limonene, linalool, methyl anthranilate, Mint arvensis, Mint oil, Mint piperita, Mint spicata, Myrcene, NEEM OIL, Sabinene, β-Caryophyllene, (1H-indol-2-yl)acetic acid, anethole, anise oil, basil oil, bay oil, camphor, ethyl salicylate, evergreen oils (pine oil) , (1,3,4,5,6,7-hexahydro-1,3-dioxo-2H-isoindol-2-yl)methyl (1R-trans)-2,2-dimethyl-3-(2-methylprop-1-enyl)cyclopropanecarboxylate (d-Tetramethrin), (RS)-3-Allyl-2-methyl-4-oxocyclopent-2-enyl-(1R,3R;1R,3S)-2,2-dimethyl-3-(2- methylprop-1-enyl)-cyclopropanecarboxy late (mixture of 4 isomers 1R trans, 1R:1R trans, 1S: 1R cis, 1R: 1R cis,1S 4:4:1:1) (d-Allethrin), (RS)-α-cyano-3phenoxybenzyl-(1RS)-cis, trans-3-(2,2-dichlorovinyl)-2,2-dimethylcyclopropanecarboxylate (Cypermethrin), 2-methyl-4-oxo-3-(prop-2-ynyl)cyclopent-2-en-1-yl 2,2-dimethyl-3-(2-methylprop-1-enyl)cyclopropanecarboxylate (Prallethrin), Acetamiprid, Azadirachtin, Bendiocarb, Bifenthrin, Boric Acid, Chlorpyrifos, deltamethrin, Diazinon, Dichlorvos, Eugenol, fipronil, imidacloprid, Linalool, Malathion, Maltodextrin, Margosa extract, Metofluthrin, Nicotine, Permethrin, Pyrethrins and Pyrethroids, rotenone, Silicium dioxide (Silicium dioxide/Kieselguhr), S-Methoprene, Spinosad (Spinosyn A), Spinosyn D, Tetramethrin, Transfluthrin and mixtures thereof.

According to one embodiment, the arthropod control composition is selected from the group consisting of N,N-diethyl-3-methylbenzamide (DEET), ethyl butylacetylaminopropionate (IR3535), para-menthan-3,8-diol (PMD) or 1-(1-methylpropoxycarbonyl)-2-(2-hydroxaethyl)piperidin (Icaridin) and mixtures thereof.

In a particular embodiment, when the composition comprises an arthropod control co-ingredient, the composition comprises the compound according to formula (I) in an amount of from 0.02 to 80 wt.%, more preferably in an amount of from 0.05 to 70 wt.%, even more preferably in an amount of from 0.1 to 60 wt.%, based on the total weight of the composition. Thereby, it is understood that the composition preferably comprises the compound according to formula (I) in a minimum amount of at least 0.2 wt.%, at least 0.05 wt.% or at least 0.1 wt.% and a maximum amount of not more than 80 wt.%, not more than 70 wt.% or not more than 60 wt.%, based on the total weight of the composition.

In a particular embodiment, when the composition comprises an arthropod control co-ingredient, the composition comprises the compound according to formula (I) in an amount of from 0.1 to 5 wt.%, more preferably from 0.15 to 4 wt.%, even more preferably 0.2 to 3 wt.% or from 11 to 35 wt.%, preferably from 15 to 30 wt.%, more preferably from 18 to 25 wt.%, based on the total weight of the composition. Thereby, it is understood that the composition in one embodiment comprises the compound according to formula (I) in a minimum amount of at least 0.1 wt.%, at least 0.15 wt.% or at least 0.2 wt.% and a maximum amount of not more than 5 wt.%, not more than 4 wt.% or not more than 3 wt.%, based on the total weight of the composition and in another embodiment comprises the compound according to formula (I) in a minimum amount of at least 11 wt.%, at least 15 wt.% or at least 18 wt.% and a maximum amount of not more than 35 wt.%, not more than 30 wt.% or not more than 25 wt.%.

In a particular embodiment, the arthropod control co-ingredient is comprised in an amount of from 0.02 to 80 wt.%, more preferably in an amount of from 0.05 to 70 wt.%, even more preferably in an amount of from 0.1 to 60 wt.%, based on the total weight of the composition. Thereby, it is understood that the composition comprises the arthropod control co-ingredient in a minimum amount of at least 0.2 wt.%, at least 0.05 wt.% or at least 0.1 wt.% and a maximum amount of not more than 80 wt.%, not more than 70 wt.% or not more than 60 wt.%, based on the total weight of the composition.

In a particular embodiment, the compound of formula (I) and the arthropod control co-ingredient are comprised in the composition in a weight range of 90:10 to 10:90, preferably in a weight range of 80:20 to 20:80, more preferably in a weight range of 65:35 to 35:65 and most preferably in a weight range of 60:40 to 40:60. It is herein also understood that the compound of formula (I) and the arthropod control co-ingredient can be comprised in the composition in any weight range combination as mentioned herein-before, such as 90:10 to 20:80, preferably 35:65 and more preferably 40:60, 80:20 to 10:90, preferably 35:65 and more preferably 40:60, 65:35 to 10:90, preferably 20:80 and more preferably 40:60 or 40:60 to 10:90, preferably 20:80 and more preferably 35:65.

In one embodiment, the arthropod control co-ingredient include one or more ingredients capable to contribute to both, the arthropod control activity and to the olfactory character of the composition.

In a particular embodiment, the arthropod control co-ingredient which is capable to contribute to both, the arthropod control activity and the olfactory character of the composition can be selected from the group consisting of
- an acid perfuming raw material family selected from the group consisting of pentanoic to dodecanoic acids, preferably hexanoic acid, heptanoic acid, octanoic acid, nonanoic acid and mixtures thereof,
- an alkene perfuming raw material family selected from the group consisting of 1-isopropyl-4-methyl-1,3-cyclohexadiene, 4-isopropenyl-1-methylcyclohexene, 4-isopropylidene-1-methylcyclohexene, beta-ocimene and mixtures thereof,
- an alcohol and phenol perfuming raw material family selected from the group consisting of 1-decanol, 2-methyl-4-phenyl-2-butanol, 8-p-menthen-2-ol, 3,4-dimethylphenol, 3,7-dimethyl-2,6-octadien-1-ol, 2-isopropylphenol, 2-methoxy-4-vinylphenol, 1,8-p-menthadien-7-ol, 4-ethylphenol, 4-methylphenol, 4-(trimethyl-1-cyclohexen-1-yl)-2-butanone, 2,5,5-trimethyl-1,2,3,4,4a,5,6,7-octahydro-2-naphthalenol and mixtures thereof,
- an aldehyde perfuming raw material family selected from the group consisting of decanal, 2-nonenal, 5-ethyl-2-methylnonanal, 3-phenylpropanal, 7-hydroxy-3,7-dimethyloctanal, 2-decenal, 4-hexenal, 8-nonenal, 2-pentenal, 2,4-nonadienal, 2,6-dimethyl-5-heptenal, 2-octenal, 2,6-nonadienal and mixtures thereof,
- an amine perfuming raw material family selected from the group consisting of methyl 2-aminobenzoate, ethyl 2-aminobenzoate, 3-methylbutylamine, 1-(2-aminophenyl)-1-ethanone, 3-methylindole and mixtures thereof,
- a ketone perfuming raw material family selected from the group consisting of 2-hexylidene-1-cyclopentanone, 3-methyl-2-(pentyloxy)-2-cyclopenten-1-one, 1-(2,6,6-trimethyl-1,3-cyclohexadien-1-yl)-2-buten-1-one, octalactone, preferably 6-propyloxan-2-one and 5-butyloxolan-2-one, nonalactone, preferably 6-butyloxan-2-oneand, decalactone, preferably and 5-hexyloxolan-2-one, undecalactone, preferably 6-hexyloxan-2-one and 5-heptyloxolan-2-one, dodecalactone, preferably 6-heptyloxan-2-one and E, 1-(2,6,6-trimethyl-1-cyclohexen-1-yl)-2-buten-1-one, 1-(2,6,6-trimethyl-2-cyclohexen-1-yl)-2-buten-1-one, methyl {3-oxo-2-[(2Z)-2-penten-1-yl]cyclopentyl}acetate, methyl 2-hexyl-3-oxocyclopentane-1-carboxylate, 6-methyl-5-hepten-2-one, 3-methyl-2-[2-penten-1-yl]-2-cyclopenten-1-one, 1-(5,5-dimethyl-1-cyclohexen-1-yl)-4-penten-1-one, 2-hydroxy-3-methyl-2-cyclopenten-1-one, 4-(2-methyl-2-propanyl)cyclohexanone, 5-methyltricyclo[6.2.1.0^{2,7}]undecan-4-one, 2,2,6-trimethylcyclohexanone, methyl (1R)-cis-3-oxo-2-pentyl-1-cyclopentaneacetate and mixtures thereof,
- an ester perfuming raw material family selected from the group consisting of allyl 3-phenyl-2-propenoate, 3-phenyl-2-propenyl 3-phenyl-2-propenoate, 1,5-dimethyl-1-vinyl-4-hexenyl 3-phenylpropenoate, ethyl 3-phenyl-2-propenoate, isobutyl 3-phenyl-2-propenoate, 2-phenylethyl 2-butenoate, 2-phenylethyl 3-methyl-2-butenoate, methyl 3-hexenoate, 2-phenylethyl 2-methyl-2-butenoate and mixtures thereof,
- an ether, epoxide and acetal perfuming raw material family selected from the group consisting of trimethyl-13-oxabicyclo[10.1.0]trideca-4,8-diene, 1,3,3-trimethyl-2-oxabicyclo[2.2.2]octane, 3,4'-dimethylspiro[oxirane-2,9'-tricyclo[6.2.1.0^{2,7}]undec[4]ene and mixtures thereof,
- a lactone perfuming raw material family selected from the group consisting of 4-nonanolide, 3-propylidene-2-benzofuran-1(3H)-one, 3-butylidene-1-benzo[C]furanone, 6-pentyltetrahydro-2H-pyran-2-one, 2-chromenone, 8-oxatricyclo[5.3.1.0^{2,6}]undecan-9-one, 6-pentyloxan-2-one, ethylidene-3-oxatricyclo[6.2.1.0^{2,7}]undecan-4-one, 8-decen-5-olide, 5-nonanolide, 5-butyloxolan-2-one, 2-chromanone, 1-oxaspiro[4.5]decan-2-one, 6-hexyltetrahydro-2H-pyran-2-one and mixtures thereof,
- a N-hetero aromatic perfuming raw material family selected from the group consisting of 1-(2-pyridyl)-1-ethanone, 3-(1-butenyl)pyridine, 1-(3-pyridyl)-1-ethanone, 3-ethylpyridine, butylquinoline, 2-isobutylquinoline, 2-ethyl-3-methylpyrazine, 4-(4,8-dimethyl-3,7-nonadien-1-yl)pyridine, 5-methylquinoxaline, 6-methylquinoline and mixtures thereof,
- a complex mixture perfuming raw material family selected from the group consisting of artemisia oil, calamus oil, chamomile oil, lemongrass oil, magnolia oil, tansy oil, Pennyroyal oil, patchouli oil, birch oil and mixtures thereof;
- and mixtures thereof.

In a particular embodiment, the at least one arthropod control co-ingredient which is capable to contribute to both, the arthropod control activity and the olfactory character of the composition is selected from the group consisting of
- an acid perfuming raw material family member selected from the group consisting of octanoic acid,
- an alkene perfuming raw material family member selected from the group consisting of 1-isopropyl-4-methyl-1,3-cyclohexadiene, 4-isopropenyl-1-methylcyclohexene, 4-isopropylidene-1-methylcyclohexene and beta-ocimene,
- an alcohol and phenol perfuming raw material family member selected from the group consisting of 2,5,5-trimethyl-1,2,3,4,4a,5,6,7-octahydro-2-naphthalenol, 3,7-dimethyl-2,6-octadien-1-ol, 2-methoxy-4-vinylphenol and 4-(trimethyl-1-cyclohexen-1-yl)-2-butanone,
- an aldehyde perfuming raw material family member selected from the group consisting of decanal, 7-hydroxy-3,7-dimethyloctanal, 2-decenal, 2,6-dimethyl-5-heptenal and 2,6-nonadienal,
- an amine perfuming raw material family member selected from the group consisting of methyl 2-aminobenzoate, and ethyl 2-aminobenzoate
- a ketone perfuming raw material family member selected from the group consisting of 1-(2,6,6-trimethyl-1,3-cyclohexadien-1-yl)-2-buten-1-one, 1-(2,6,6-trimethyl-2-cyclohexen-1-yl)-2-buten-1-one, (2E)-1-(2,6,6-trimethyl-1-cyclohexen-1-yl)-2-buten-1-one, methyl 2-hexyl-3-oxocyclopentane-1-carboxylate, 1-(5,5-dimethyl-1-cyclohexen-1-yl)-4-penten-1-one, 4-(2-methyl-2-propanyl)cyclohexanone, 5-methyltricyclo[6.2.1.0^{2,7}]undecan-4-one and methyl (1R)-cis-3-oxo-2-pentyl-1-cyclopentaneacetate,
- an ether, expoxide and acetal perfuming raw material family member selected from the group consisting of trimethyl-13-oxabicyclo[10.1.0]trideca-4,8-diene, 1,3,3-trimethyl-2-oxabicyclo[2.2.2]octane, and 3,4'-dimethylspiro[oxirane-2,9'-tricyclo[6.2.1.0^{2,7}]undec[4]ene,
- a lactone perfuming raw material family member selected from the group consisting of 4-nonanolide, 6-pentyltetrahydro-2h-pyran-2-one, 2-chromenone, 6-pentyloxan-2-one, 8-decen-5-olide, ethylidene-3-oxatricyclo[6.2.1.0^{2,7}]undecan-4-one, 5-butyloxolan-2-one, 5-nonanolide and 6-hexyltetrahydro-2H-pyran-2-one,
- a N-hetero aromatic perfuming raw material family member is selected from the group consisting of 1-(2-pyridyl)-1-ethanone and isobutylquinoline,
- a complex mixture of perfuming raw material family member selected from the group consisting of lemongrass oil, pennyroyal oil and patchouli oil and
- mixtures thereof.

In a particular embodiment, the at least one arthropod control co-ingredient which is capable to contribute to both, the arthropod control activity and the olfactory character of the composition is selected from the group consisting of
- an alcohol and phenol perfuming raw material family member being selected from the group consisting of 3,7-dimethyl-2,6-octadien-1-ol and 2-methoxy-4-vinylphenol,
- an aldehyde perfuming raw material family member being selected from the group consisting of 7-hydroxy-3,7-dimethyloctanal,
- an amine perfuming raw material family member being selected from the group consisting of methyl 2-aminobenzoate and ethyl 2-aminobenzoate,
- a ketone perfuming raw material family member being selected from the group consisting of 1-(2,6,6-trimethyl-1,3-cyclohexadien-1-yl)-2-buten-1-one, methyl (1R)-cis-3-oxo-2-pentyl-1-cyclopentaneacetate and 4-(2-methyl-2-propanyl)cyclohexanone,
- an ether, expoxide or acetal perfuming raw material family member being selected from the group consisting of trimethyl-13-oxabicyclo[10.1.0]trideca-4,8-diene and 1,3,3-trimethyl-2-oxabicyclo[2.2.2]octane,
- a lactone perfuming raw material family member being selected from the group consisting of 4-nonanolide, 6-pentyltetrahydro-2H-pyran-2-one, 6-propyltetrahydro-2H-pyran-2-one, 2-chromenone, 6-pentyloxan-2-one, 8-decen-5-olide, 5-nonanolide and 6-hexyltetrahydro-2H-pyran-2-one,
- a N-hetero aromatic perfuming raw material family member being selected from the group consisting of 1-(2-pyridyl)-1-ethanone,
- a complex mixture perfuming raw material family member being selected from the group consisting of lemongrass oil and pennyroyal oil, and
- mixtures thereof.

In a particular embodiment, the at least one arthropod control co-ingredient which is capable to contribute to both, the arthropod control activity and the olfactory character of the composition is methyl (1R)-cis-3-oxo-2-pentyl-1-cyclopentaneacetate.

In a particular embodiment, when the composition comprises an arthropod control co-ingredient which is capable to contribute to both, the arthropod control activity and the olfactory character of the composition, the composition comprises the compound according to formula (I) in an amount of from 0.02 to 80 wt.%, more preferably in an amount of from 0.05 to 70 wt.%, even more preferably in an amount of from 0.1 to 60 wt.%, based on the total weight of the composition. Thereby, it is understood that the composition preferably comprises the compound according to formula (I) in a minimum amount of at least 0.2 wt.%, at least 0.05 wt.% or at least 0.1 wt.% and a maximum amount of not more than 80 wt.%, not more than 70 wt.% or not more than 60 wt.%, based on the total weight of the composition.

In a particular embodiment, when the composition comprises an arthropod control co-ingredient which is capable to contribute to both, the arthropod control activity and the olfactory character of the composition, the composition comprises the compound according to formula (I) in an amount of from 0.1 to 5 wt.%, more preferably from 0.15 to 4 wt.%, even more preferably 0.2 to 3 wt.% or from 11 to 35 wt.%, preferably from 15 to 30 wt.%, more preferably from 18 to 25 wt.%, based on the total weight of the composition. Thereby, it is understood that the composition in one embodiment preferably comprises the compound according to formula (I) in a minimum amount of at least 0.1 wt.%, at least 0.15 wt.% or at least 0.2 wt.% and a maximum amount of not more than 5 wt.%, not more than 4 wt.% or not more than 3 wt.%, based on the total weight of the composition and in another embodiment comprises the compound according to formula (I) in a minimum amount of at least 11 wt.%, at least 15 wt.% or at least 18 wt.% and a maximum amount of not more than 35 wt.%, not more than 30 wt.% or not more than 25 wt.%.

In a particular embodiment, the at least one arthropod control co-ingredient which is capable to contribute to both, the arthropod control activity and the olfactory character of the composition being the acid perfuming raw material family member, when present in the composition, is in an amount of 0.002 to 0.007 wt.%, more preferably 0.0025 to 0.0065 wt.%, based on the total weight of the composition.

In a particular embodiment, the at least one arthropod control co-ingredient which is capable to contribute to both, the arthropod control activity and the olfactory character of the composition being the alkene perfuming raw material family member, when present in the composition, is in an amount of 0.2 to 16 wt.%, preferably 1 to 14 wt.%, more preferably 3 to 13 wt.%, based on the total weight of the composition.

In a particular embodiment, the at least one arthropod control co-ingredient which is capable to contribute to both, the arthropod control activity and the olfactory character of the composition being the alcohol or phenol perfuming raw material family member, when present in the composition, is in an amount of 0.15 to 4 wt.%, preferably 0.2 to 3.5 wt.%, more preferably 0.4 to 3 wt.%, based on the total weight of the composition.

In a particular embodiment, the at least one arthropod control co-ingredient which is capable to contribute to both, the arthropod control activity and the olfactory character of the composition being the aldehyde perfuming raw material family member, when present in the composition, is present in an amount of 0.001 to 30 wt.%, preferably 0.005 to 25 wt.%, more preferably 0.01 to 20 wt.%, based on the total weight of the composition.

In a particular embodiment, the at least one arthropod control co-ingredient which is capable to contribute to both, the arthropod control activity and the olfactory character of the composition being the amine perfuming raw material family member, when present in the composition, is in an amount of 0.01 to 3 wt.%, preferably 0.05 to 2 wt.%, more preferably 0.1 to 0.8 wt.%, based on the total weight of the composition.

In a particular embodiment, the at least one arthropod control co-ingredient which is capable to contribute to both, the arthropod control activity and the olfactory character of the composition being the ketone perfuming raw material family member, when present in the composition, is in an amount of 0.001 to 15 wt.%, preferably 0.01 to 10 wt.%, more preferably 0.1 to 5.5 wt.%, based on the total weight of the composition.

In a particular embodiment, the at least one arthropod control co-ingredient which is capable to contribute to both, the arthropod control activity and the olfactory character of the composition being the ether, epoxide and acetal perfuming raw material family member, when present in the composition, is in an amount of 0.002 to 20 wt.%, preferably 0.02 to 15 wt.%, more preferably 0.2 to 12.5 wt.%, based on the total weight of the composition.

In a particular embodiment, the at least one arthropod control co-ingredient which is capable to contribute to both, the arthropod control activity and the olfactory character of the composition being the lactone perfuming raw material family member, when present in the composition, is in an amount of 0.002 to 65 wt.%, preferably 0.02 to 50 wt.%, more preferably 0.2 to 38.8 wt.%, based on the total weight of the composition.

In a particular embodiment, the at least one arthropod control co-ingredient which is capable to contribute to both, the arthropod control activity and the olfactory character of the composition being the N-hetero aromatic perfuming raw material family member, when present in the composition, is in an amount of 0.002 to 5 wt.%, preferably 0.02 to 2 wt.%, more preferably 0.2 to 0.4 wt.%, based on the total weight of the composition.

In a particular embodiment, the at least one arthropod control co-ingredient which is capable to contribute to both, the arthropod control activity and the olfactory character of the composition being the complex mixture perfuming raw material family member, when present in the composition, is in an amount of 0.002 to 10 wt.%, preferably 0.02 to 7.5 wt.%, more preferably 0.2 to 4.8 wt.%, based on the total weight of the composition.

In a particular embodiment, the at least one arthropod control co-ingredient which is capable to contribute to both, the arthropod control activity and the olfactory character of the composition being
- the acid perfuming raw material family member, when present in the composition, is in an amount of 0.002 to 0.007 wt.%, more preferably 0.0025 to 0.0065 wt.%,
- the alkene perfuming raw material family member, when present in the composition, is in an amount of 0.2 to 16 wt.%, preferably 1 to 14 wt.%, more preferably 3 to 13 wt.%,
- the alcohol or phenol perfuming raw material family member, when present in the composition, is in an amount of 0.15 to 4 wt.%, preferably 0.2 to 3.5 wt.%, more preferably 0.4 to 3 wt.%
- the aldehyde perfuming raw material family member, when present in the composition, is present in an amount of 0.001 to 30 wt.%, preferably 0.005 to 25 wt.%, more preferably 0.01 to 20 wt.%,
- the amine perfuming raw material family member, when present in the composition, is in an amount of 0.01 to 3 wt.%, preferably 0.05 to 2 wt.%, more preferably 0.1 to 0.8 wt.%,
- the ketone perfuming raw material family member, when present in the composition, is in an amount of 0.001 to 15 wt.%, preferably 0.01 to 10 wt.%, more preferably 0.1 to 5.5 wt.%,
- the ether, epoxide and acetal perfuming raw material family member, when present in the composition, is in an amount of 0.002 to 20 wt.%, preferably 0.02 to 15 wt.%, more preferably 0.2 to 12.5 wt.%,
- the lactone perfuming raw material family member, when present in the composition, is in an amount of 0.002 to 65 wt.%, preferably 0.02 to 50 wt.%, more preferably 0.2 to 38.8 wt.%,
- the N-hetero aromatic perfuming raw material family member, when present in the composition, is in an amount of 0.002 to 5 wt.%, preferably 0.02 to 2 wt.%, more preferably 0.2 to 0.4 wt.%, and
- the complex mixture perfuming raw material family member, when present in the composition, is in an amount of 0.002 to 10 wt.%, preferably 0.02 to 7.5 wt.%, more preferably 0.2 to 4.8 wt.%,
based on the total weight of the composition.

In a particular embodiment, the compound of formula (I) and the arthropod control co-ingredient which is capable to contribute to both, the arthropod control activity and the olfactory character of the composition are comprised in the composition in a weight range of 90:10 to 10:90, preferably in a weight range of 80:20 to 20:80, more preferably in a weight range of 65:35 to 35:65 and most preferably in a weight range of 60:40 to 40:60. It is herein also understood that the compound of formula (I) and the arthropod control co-ingredient can be comprised in the composition in any weight range combination as mentioned herein-before, such as 90:10 to 20:80, preferably 35:65 and more preferably 40:60, 80:20 to 10:90, preferably 35:65 and more preferably 40:60, 65:35 to 10:90, preferably 20:80 and more preferably 40:60 or 40:60 to 10:90, preferably 20:80 and more preferably 35:65.

The arthropod controlling composition can further comprise a carrier. By "carrier" is understood a material with which the active compound is mixed or formulated to facilitate its application to a locus or other object to be treated, or its storage, transport and/or handling. Said carrier may be of inorganic or organic or of synthetic natural origin. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly cannot be exhaustive. However, one can cite as non-limiting examples, solvents such as butylene or propylene glycol, glycerol, dipropylene glycol and its monoether, 1,2,3-propanetriyl triacetate, dimethyl glutarate, dimethyl adipate 1,3-diacetyloxypropan-2-yl acetate, diethyl phthalate, isopropyl myristate, benzyl benzoate, benzyl alcohol, 2-(2-ethoxyethoxy)-1-ethanol, tri-ethyl citrate, 2-methylprop-1-ene and 2-(2-ethoxyethoxy)ethanol or mixtures thereof, particular suitable are dipropylene glycol, 2-methylprop-1-ene and 2-(2-ethoxyethoxy)ethanol and mixtures thereof.

For the compositions which comprise a carrier, other suitable carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company), or hydrogenated castors oils such as those known under the trademark Cremophor^{®} RH 40 (origin: BASF).

A solid carrier is meant to designate a material to which the arthropod controlling composition or some element of the arthropod controlling composition can be chemically or physically bound. In general, such solid carriers are employed either to stabilize the composition, or to control the rate of evaporation of the compositions or of some ingredients. The use of solid carrier is of current use in the art and a person skilled in the art knows how to reach the desired effect. However, by way of non-limiting example of solid carriers, one may cite absorbing gums or polymers or inorganic material, such as porous polymers, cyclodextrins, wood based materials, organic or inorganic gels, clays, gypsum talc or zeolites.

As other non-limiting examples of solid carriers, one may cite encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloide: Stabilisatoren, Dickungs- und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation technique.

As non-limiting examples of encapsulations, one may cite in particular the core-shell capsules with resins of aminoplast, polyamide, polyester, polyurea or polyurethane type or a mixture thereof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycoluryl, melamine, methylol melamine, methylated methylol melamine, guanazole and the like, as well as mixtures thereof. Alternatively one may use preformed resins alkylolated polyamines such as those commercially available under the trademark Urac^{®} (origin: Cytec Technology Corp.), Cymel^{®} (origin: Cytec Technology Corp.), Urecoll^{®} or Luracoll^{®} (origin: BASF).

Others resins one are the ones produced by the polycondensation of an a polyol, like glycerol, and a polyisocyanate, like a trimer of hexamethylene diisocyanate, a trimer of isophorone diisocyanate or xylylene diisocyanate or a Biuret of hexamethylene diisocyanate or a trimer of xylylene diisocyanate with trimethylolpropane (known with the tradename of Takenate^{®}, origin: Mitsui Chemicals), among which a trimer of xylylene diisocyanate with trimethylolpropane and a Biuret of hexamethylene diisocyanate.

Some of the seminal literature related to the encapsulation by polycondensation of amino resins, namely melamine-based resins with aldehydes includes represented by articles such as those published by K. Dietrich et al. Acta Polymerica, 1989, vol. 40, pages 243, 325 and 683, as well as 1990, vol. 41, page 91. Such articles already describe the various parameters affecting the preparation of such core-shell microcapsules following prior art methods that are also further detailed and exemplified in the patent literature. US 4'396'670, to the Wiggins Teape Group Limited is a pertinent early example of the latter. Since then, many other authors have enriched the literature in this field and it would be impossible to cover all published developments here, but the general knowledge in encapsulation technology is very significant. More recent publications of pertinency, which disclose suitable uses of such microcapsules, are represented for example by the article of H.Y.Lee et al. Journal of Microencapsulation, 2002, vol. 19, pages 559-569, international patent publication WO 01/41915 or yet the article of S. Bône et al. Chimia, 2011, vol. 65, pages 177-181.

According to one embodiment, the compound of formula (I) is chemically bonded to the carrier. The compound of formula (I) can be chemically bonded to the carrier, thereby preferably forming a chemical bond which is labile to certain triggers such as oxidizing environment, moisture, light or enzymes.

As non-limiting example, the compound of formula (I) can be chemically bonded to the carrier *via* an alcohol moiety of the compound of formula (I) to thereby form
- a cinnamyl ether functional group, resulting in an oxygen-labile compound, such as but not limited to the one reported in US9718752, US20180016521;
- a enol-ether functional group, resulting in an oxygen-labile compound;
- one or two ester functional groups, resulting in an enzymatic-labile compound, such as but not limited to the one reported in WO199504809; siloxanes, resulting in moisture-labile compounds, such as but not limited to those reported in WO2000014091.

In a particular embodiment, the arthropod controlling composition comprises of at least one compound of formula (I) and at least one carrier. In a particular embodiment, the arthropod controlling composition comprises at least one compound of formula (I), at least one carrier and at least one arthropod control co-ingredient.

In a particular embodiment, the arthropod controlling composition consists of at least one compound of formula (I) and at least one carrier. In a particular embodiment, the arthropod controlling composition consists of at least one compound of formula (I), at least one carrier and at least one arthropod control co-ingredient.

In a particular embodiment, the compositions mentioned hereinabove, comprise more than one compound of formula (I).

The present invention also relates to a method for arthropod, preferably insect, control which comprises bringing an arthropod, preferably insect, into direct contact or in contact with vapors of a composition as described hereinabove or a compound according to formula (I) as described hereinabove.

For the sake of clarity, the compounds of formula (I) and the arthropod controlling composition according to the present invention can be applied to the air, to the surface of an article, the air in the vicinity of the surface of an article or the surface of a subject by usual methods known in the art such as spraying, applying, wearing or diffusing.

In a particular embodiment, the compound of formula (I) or the arthropod controlling composition according to the present invention is applied to the surface of an article, the air in the vicinity of the surface of an article or to the surface of an animal or subject.

In a particular embodiment, the article can be an arthropod control article as described hereinbelow and in particular can be a candle, coil, electric diffuser, wristband, patch, collar, ear tag, clothes, fabrics, papers, biochar, cardboard, cellulosic pads, bed nets, screen, curtains, furniture, paint, walls, ground, spray, aerosol, cream, roll on, wristband, lotion, soap, shampoo, sunscreen, laundry powder, liquid detergent, spray, lotion, powder .

In a particular embodiment, the surface of a subject is the surface of a human or animal subject, preferably the surface is a human subject, *i.e.* the skin of a human subject.

The present invention also relates to a use of a composition as defined hereinabove or a compound of formula (I) as defined hereinabove to control arthropods, preferably insects.

The present invention also relates to an arthropod control article comprising a compound of formula (I) or an arthropod controlling composition as described hereinabove.

By "arthropod control article" is understood to designate a consumer product which delivers at least an arthropod controlling effect to the surface or space to which it is applied (e.g. skin, hair, textile, or home surface). In other words, an arthropod controlling article according to the invention is a consumer product which comprises a functional formulation, as well as optionally additional benefit agents, corresponding to the desired consumer product, and an arthropod controlling amount of at least one invention's compound. For the sake of clarity, said consumer product is a non-edible product.

The nature and type of the constituents of the consumer product do not warrant a more detailed description here, which in any case would not be exhaustive, the skilled person being able to select them on the basis of his general knowledge and according to the nature and the desired effect of said product.

Non-limiting examples of suitable consumer products include a perfume, such as a fine perfume, a splash or eau de parfum, a cologne or a shave or after-shave lotion or a cream or gel; a fabric care product, such as a liquid or solid detergent, a fabric softener, a liquid or solid scent booster, a fabric refresher, an ironing water, a paper, a bleach, a carpet cleaner, a curtain-care product; a body-care product, such as a hair care product (e.g. a shampoo, a coloring preparation or a hair spray, a color-care product, a hair shaping product, a dental care product), a disinfectant, an intimate care product; a cosmetic preparation (e.g. a skin cream or lotion, a vanishing cream or a deodorant or antiperspirant (e.g. a spray or roll on), a hair remover, a tanning or sun or after sun product, a nail product, a skin cleansing, a makeup); or a skin-care product (e.g. a soap, a shower or bath mousse, oil or gel, or a hygiene product or a foot/hand care products); an air care product, such as an air freshener or a "ready to use" powdered air freshener which can be used in the home space (rooms, refrigerators, cupboards, shoes or car) and/or in a public space (halls, hotels, malls, etc.); or a home care product, such as a mold remover, a furnisher care product, a wipe, a dish detergent or a hard-surface (e.g. a floor, bath, sanitary or a window-cleaning) detergent; a leather care product; a car care product, such as a polish, a wax or a plastic cleaner; a candle; a spray, a coil, an electric diffuser, a diffusor, a rubber septum, a wristband, a patch, a collar, an ear tag, clothes, fabrics, papers, a biochar, a cardboard, celluloic pads, bed nets, a screen, curtains, a varnish or a paint, more preferably a candle, a spray, a coil, an electric diffuser, a diffusor, a rubber septum, a wristband, a patch, a collar, an ear tag, clothes, fabrics, papers, a biochar, a cardboard, celluloic pads, bed nets, a screen, curtains, a varnish or a paint.

Some of the above-mentioned consumer products may represent an aggressive medium for the compounds of formula (I), so that it may be necessary to protect the latter from premature decomposition, for example by encapsulation or by chemically binding it to another chemical which is suitable to release the invention's ingredient upon a suitable external stimulus, such as an enzyme, light, heat or a change of pH.

The present invention also relates to compounds according to formula (I): 2,3-dihydro-1H-indene-2-carbonitrile, (4aRS,9bRS)-4a,7-dimethyl-4,4a,5,9b-tetrahydroindeno[1,2-d][1,3]dioxine, (2RS,4aRS,9bRS)-2,4a,7-trimethyl-4,4a,5,9b-tetrahydroindeno[1,2-d][1,3]dioxine (2RS,4aSR,9bSR)-2,4a,7-trimethyl-4,4a,5,9b-tetrahydroindeno[1,2-d][1,3]dioxine, 2,3-dihydro-1H-inden-2-ylmethanol, (2RS,4aRS,9bSR)-2-methyl-4,4a,5,9b-tetrahydroindeno[1,2-d][1,3]dioxin (2RS,4aSR,9bRS)-2-methyl-4,4a,5,9b-tetrahydroindeno[1,2-d][1,3]dioxin, (4aRS,9bSR)-2,2-dimethyl-4,4a,5,9b-tetrahydroindeno[1,2-d][1,3]dioxin, 5-(tert-butyl)-1,3-dihydrospiro[indene-2,3'-oxetane], 4A,8-dimethyl-indano[1,2-d]-1,3-dioxan-2-one, (4aRS,9bSR)-8-methyl-4,4a,5,9b-tetrahydroindeno[1,2-d][1,3]dioxin, (2RS,4aRS,9bSR)-2-ethyl-4,4a,5,9b-tetrahydroindeno[1,2-d][1,3]dioxine (2RS,4aSR,9bRS)-2-ethyl-4,4a,5,9b-tetrahydroindeno[1,2-d][1,3]dioxine, (2R,4aS,9bS)-2,4a,8-trimethyl-4,4a,5,9b-tetrahydroindeno[1,2-d][1,3]dioxin.

### Examples

The invention will be described in further detail by way of the following examples.

### 1. Sample preparation

Ethanolic solutions of (2,5-dimethyl-2,3-dihydro-1H-inden-2yl)methanol at six concentrations ranging between 0.0008% and 2.5% (w/w) for the *in vitro* test and at five concentrations ranging between 0.02% and 10% (w/v) for the *in vivo* test were prepared.

Ethanolic solutions of 4a,8-dimethyl-indano[1,2-d]-1,3-dioxan-2-one, (2,4,6-trimethyl-2,3-dihydro-1h-inden-2-yl)methanol, 5-(tert-butyl)-1,3-dihydrospiro[indene-2,3'-oxetane], 1-(2,5-dimethyl-2-indanyl)-1-ethanol, (2,5-dimethyl-2,3-dihydro-1H-inden-2yl)methanol, (2,5-dimethyl-2-indanyl)methyl formate, (2,5-dimethyl-2,3-dihydro-1H-inden-2-yl)methyl methyl ether, 2,5-dimethyl-2,3-dihydro-1H-indene-2-carboxylic acid, (2,5-dimethyl-2,3-dihydro-1H-inden-2yl)methanol, 5-isopropyl-2-methyl-2-indanmethanol, 2-(hydroxymethyl)-2,6-dimethyl-2,3-dihydro-1*H*-inden-1-ol, 5-tert-butyl-2-methyl-2-indanmethanol, 2-((2-methoxypropan-2-yloxy)methyl)-2,6-dimethyl-2,3-dihydro-1*H*-inden-1-ol and 2-(hydroxymethyl)-2,6-dimethyl-2,3-dihydro-1*H*-inden-1-ol at three concentrations (0.0016%, 0.04% and 1% (w/w)) were prepared.

Ethanolic solutions of 5-tert-butyl-indan-2-spiro-3'-oxetane, (2-methyl-1,3-dihydroinden-2-yl)methanol, (2,6-dimethyl-1,2,3,4-tetrahydro-2-naphthalenyl)methanol, 1-(2,5-dimethyl-2,3-dihydro-1H-inden-2-yl)ethanone, (5-methyl-2,3-dihydro-1H-inden-2-yl)methanol, 1-(2,4,5-trimethyl-2,3-dihydro-1H-inden-2-yl)ethanone, 2,3-dihydro-1*H-*inden-2-ylmethanol, (4aRS,9bSR)-8-methyl-4,4a,5,9b-tetrahydroindeno[1,2-d][1,3]dioxin, (2,5,6-trimethyl-2,3-dihydro-1H-inden-2-yl)methanol, methyl 2,5-dimethyl-2,3-dihydro-1H-indene-2-carboxylate, (2-methyl-2,3-dihydro-1H-inden-2-yl)methyl acetate, 2-((methoxymethoxy)methyl)-2,5-dimethyl-2,3-dihydro-1H-indene, 2-[(1-methoxyethoxy)methyl]-2,5-dimethylindane, 4,4A,5,9B-tetrahydro-indeno[1,2-d]-1,3-dioxin, blend of 2,4-dimethyl-4,4a,5,9b-tetrahydroindeno[1,2-d][1,3]dioxine & 2,4-dimethyl-4,4a,5,9b-tetrahydroindeno[1,2-d][1,3]dioxine at up to fifteen concentrations ranging from 0.00032% to 1% (w/w)) were prepared.

Three perfumes were created containing
- 33% (w/w) of (2,5-dimethyl-2,3-dihydro-1H-inden-2yl)methanol plus two additional PRMs (perfume 1a),
- 10% (w/w) of (2,5-dimethyl-2,3-dihydro-1H-inden-2yl)methanol plus nine additional PRMs (perfume 1b).
- 6.5 % (w/w) of (2,5-dimethyl-2,3-dihydro-1H-inden-2yl)methanol plus seven additional PRMs (perfume 1e).

The solvent ethanol and two well-known repellent compounds N,N-diethyl-3-methylbenzmamide (DEET) and 3-[N-n-butyl-N-acetyl]aminopropionic acid ethylester (insect repellent 3535; IR3535) at similar dilutions were used as controls.

Blends containing a perfume and a known repellent were tested overtime in an aqueous base (: Ethylenediaminetetraacetic acid (0.05%), acid citric (0.5%), sodium citrate (1.33%), lauryl ethyl ether (3.5%), butylene glycol (12.5%), water (69.37%)). The perfumes 1a & 1e were diluted at 1% and IR3535 was diluted at 7.5% or 12.5%.

Three additional blends were created containing:
- 50% (w/w) of (2,5-dimethyl-2,3-dihydro-1H-inden-2yl)methanol plus 50% (w/w) of (+)-methyl (1R)-cis-3-oxo-2-pentyl-1-cyclopentaneacetate (blend #1).
- 50% (w/w) of (2,5-dimethyl-2,3-dihydro-1H-inden-2yl)methanol plus 50% (w/w) of 3-[N-n-butyl-N-acetyl]aminopropionic acid ethylester (blend #2).
- 75% (w/w) of 4,4A,5,9B-tetrahydro-indeno[1,2-D]-1,3-dioxin plus 25% (w/w) of 2-(2-hydroxypropan-2-yl)-5-methylcyclohexan-1-ol (blend #3).
- 0.22% (w/w) of 4,4A,5,9B-tetrahydro-indeno[1,2-D]-1,3-dioxin plus 68 additional PRMs (perfume 5f).
0.4% (w/w) of 4,4A,5,9B-tetrahydro-indeno[1,2-D]-1,3-dioxin plus 71 additional PRMs (perfume 5g).There were all diluted in ethanol at the tested concentrations.

### 2. Model organism

Controlling efficacy was tested against the yellow fever mosquito, *A. aegypti* Rockefeller strain.

*A. aegypti* is a model organism for controlling tests and one of the recommended model organism by the World Health Organization (WHO) as it is a very aggressive, anthropophilic mosquito species that shows generally low sensitivity to arthropod controlling compounds.

Observations of controlling efficacy were made on host-seeking females of uniform age, 5 to 10 days post-eclosion from pupae. Tested hungry females had access to 10% sugar solution but were not blood-fed.

Repellent efficacy of the different compounds was assessed against the sheep tick, *Ixodes ricinus. I. ricinus* is one of the recommended model organisms mentioned by the Guidance on the European Biological Products Regulation [Vol II, Efficacy - Assessment & Evaluation (Parts B+C), v. 3.0, April 2018]. Observations of repellent efficacy were made on last stage nymphs.

### 3. Description of the arthropod controlling tests

The arthropod repellency is tested using *in vitro* and *in vivo* test methods described in the following.

### 3.1. In vitro K&D assay:

The controlling efficacy *in vitro* was measured using the Klun & Debboun (K&D) assay test module [Klun JA, Kramer M, Debboun M. A new in vitro bioassay system for discovery of novel human-use mosquito controlling compounds. J Am Mosq Control Assoc. 2005; 21:64-70.], which is an adaptation of the K&D module [Klun JA, Debboun M. A new module for quantitative evaluation of controlling efficacy using human subjects. J Med Entomol. 2000; 37:177-81.], initially developed and used for quantitative measurement of the efficacy of mosquito controlling compounds on human subjects.

In the *in vitro* assay the module is coupled with a membrane-blood reservoir, consisting of a unit with six wells that are filled with 7 mL defibrinated pig blood maintained at a constant temperature of 38°C and covered with a b ovine collagen film (Devro plc, Edicol-MX400 CLR 100M REEL) as the membrane. Over the membrane a piece of cotton cloth (Baptist Roh, 100 % cotton, weight: 69.0 g/m², thread density: warp and woof 26.5 x 26.5 thread/cm, Modessa Basel) was placed after being treated with 80µL of the tested stimuli.

The K&D unit, containing five mosquitoes in each cell was finally placed on top of the treated cloth. Then the numbers of mosquitoes biting in each cell within a 3-min exposure were recorded. This procedure was repeated until 6 replicate exposures and observations (i.e. 30 mosquitoes) were made.

### 3.2. In vivo K&D assay:

For the K&D *in vivo* test [Klun JA, Debboun M. A new module for quantitative evaluation of controlling efficacy using human subjects. J Med Entomol. 2000; 37:177-81.], 3 study participants, 2 females and 1 male, showing low or no skin reactions against mosquito bites were recruited at the Swiss Tropical and Public Health Institute (Swiss TPH). The age of the study participants was 28 and 32 years for the females and 28 for the male participant. The study was approved by the Ethics Commission of Northwest and Central Switzerland (Study no. PB_2017-00436 - 220/11) and all study participants signed an informed consent form. To avoid unwanted bias the participants were asked to avoid alcohol and products such as perfume, eau de cologne and lotions for at least 12 hours before and during the experiments. During the experiments the participants were also asked to avoid rubbing, touching or wetting the arthropod controlling compound-treated area as well as any activity that might lead to increased perspiration.

In the *in vivo* assay, a template was used to delineate 4 cm x 5 cm areas on the skin that correspond to each of the six cell openings (3 cm x 4 cm) on the bottom of the K&D module. Five areas were treated with the different tested stimuli. The module, with five host-seeking, 5- to 10-day-old mosquitoes in each cell, was then positioned over the treated skin area. The number of mosquitoes blood feeding within a 2-minute exposure was recorded. This procedure was repeated until six replicate exposures and observations (i.e. 30 mosquitoes) were made for the three study participants.

### 3.3. In vitro warm body assay:

### 3.3.1. In vitro warm body assay for insects such as mosquitos

The controlling effect according to the present invention is determined by testing the Warm Body assay against the yellow fever mosquito, *A. aegypti* Rockefeller strain. *A. aegypti* is a model organism for controlling tests and one of the recommended model organisms by the World Health Organization (WHO) as it is a very aggressive, anthropophilic mosquito species that shows generally low sensitivity to arthropod controlling compounds. Observations of controlling efficacy were made on host-seeking females of uniform age, 5 to 10 days old selected as mentioned in the publication mentioned hereinabove. Tested hungry females had access to 10% sugar solution but were not blood-fed.

The controlling effect according to the present invention was assessed using an adapted Warm Body assay as defined in Kröber T, Kessler S, Frei J, Bourquin M, Guerin PM. An in vitro assay for testing mosquito controlling compounds employing a warm body and carbon dioxide as a behavioral activator. J Am Mosq Control Assoc. 2010; 26:381-386.

The published protocol has been adapted in not manually counting the landing mosquitoes but automatically using an automatic counting software, the switch from *Anopheles gambiae* to *Aedes aegypti* led to a decrease of mosquitoes' number placed in the tested cage due to the size difference *(i.e.* 30 mosquitoes instead of 50) and to an increase of lighting as *Aedes aegypti* is a diurnal mosquitoes *(i.e.* 150 lux instead of 4 lux).

In this *in-vitro* assay the number of *A. aegypti* landing on a warm body treated with the tested stimuli was measured in order to assess the repellency effect. Three replicates were carried on the three different doses: 0.0016%, 0.04% and 1% in ethanol, using 25 *A. aegypti* 10-12 days old females per cage.

### 3.3.2. In vitro warm plate assay for arachnids such as ticks

The repellent efficacy was assessed using the protocol of the in-vitro Warm Plate Assay as defined in Kröber T, Bourquin M, Guerin PM. A standardized in vivo and in vitro test method for evaluating tick repellents. Pesticide Biochemistry and Physiology. 2013; 107(2):160-168.].

### 3.4. In vivo arm in cage test:

The Arm in the box method was adapted from the WHO Guidelines for efficacy testing of mosquito repellents for human skin (WHO/CDS/NTD/WHOPES/2009.4). The readiness of 100 hungry female mosquitoes *A. aegypti* to probe is assessed by inserting an untreated arm into the cage (40x40x40cm) for 30 seconds (negative control) three times (once at the beginning, once at the fourth hour and once at the eighth hour), to determine probing activity. Then, the product is applied onto the skin of the forearm of a human volunteer (1 ml per 600 cm²) and after 5 minutes, this arm is inserted into the cage and exposed for 3 minutes. The assay take place in temperature (27±2°C) and humidity (80±10%RH) regulated room on three different volunteers.

### 4. Evaluation of the arthropod controlling effect of the present invention

The arthropod repellency of the present invention is tested using *in vitro* and *in vivo* test methods as generally described in the following.

### 4.1. In vitro K&D assay:

The *In-vitro* repulsion of mosquitoes *Aedes aegypti* is measured against different dilutions of (2,5-dimethyl-2,3-dihydro-1H-inden-2yl)methanol in the K&D test and shown in the following Table 1.

**Table 1 - in-vitro repulsion of Aedes aegypti is measured against different dilutions of (2,5-dimethyl-2,3-dihydro-1H-inden-2yl)methanol in the K&D test.**

| **compound** | **% concentration** | **% repulsion** | **% ± SD** |
|---|---|---|---|
| 2,5-dimethyl-2,3-dihydro-1H-inden- | 0.0008 | 21.5 | 6.7 |
| | 0.004 | 25.3 | 10.3 |
| | 0.02 | 33.8 | 13.6 |
| 2yl)methanol | 0.1 | 57.1 | 13.0 |
| | 0.5 | 84.6 | 8.7 |
| | 2.5 | 92.3 | 6.7 |
| ethanol | 100 | 28.5 | 7.1 |

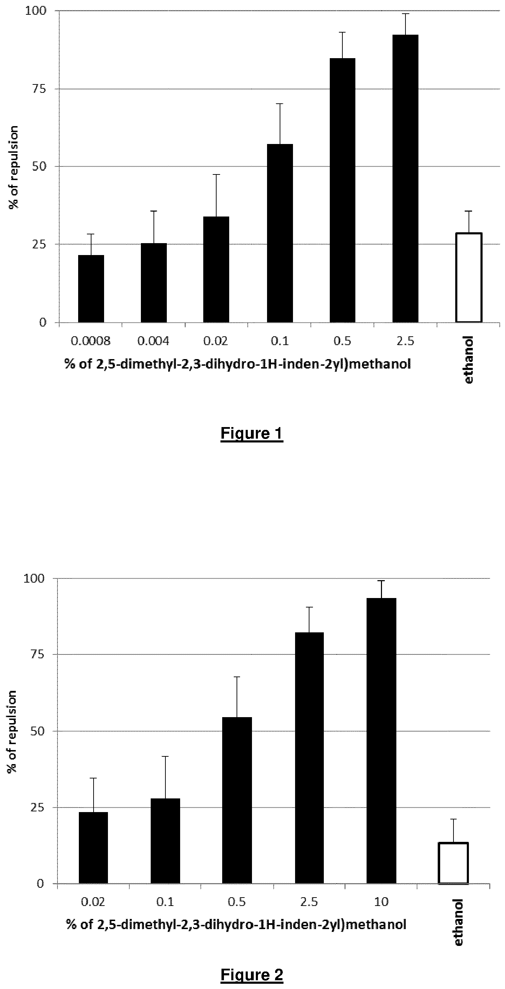

The *In-vitro* repulsion of mosquitoes *Aedes aegypti* is measured against different dilutions of (2,5-dimethyl-2,3-dihydro-1H-inden-2yl)methanol in the K&D test according to Table 1 is illustrated in Figure 1.

The percentage of repulsion of *Aedes aegypti* increases with the increase tested amount of (2,5-dimethyl-2,3-dihydro-1H-inden-2yl)methanol demonstrating the biological effect of (2,5-dimethyl-2,3-dihydro-1H-inden-2yl)methanol *(cf.* Table 1 and Figure 1).

At low concentrations (0.0008%, 0.004% and 0.02%), the repulsion was equivalent to the repulsion elicited by the solvent ethanol, illustrating the non-efficacy of (2,5-dimethyl-2,3-dihydro-1H-inden-2yl)methanol on its own at these concentrations (cf. Table 1 and Figure 1). At concentration of (2,5-dimethyl-2,3-dihydro-1H-inden-2yl)methanol above 0.02%, the percentage of repulsion was consistently higher than the percentage of repulsion elicited by the solvent ethanol *(cf.* Table 1 and Figure 1).

Using the K&D *in-vitro* assay, (2,5-dimethyl-2,3-dihydro-1H-inden-2yl)methanol has shown on its own a significant repellent effect against *Aedes aegypti* as soon as its concentration was equal or above 0.1% in ethanol.

### 4.2. In vivo K&D assay:

The *In-vivo* repulsion of mosquitoes *Aedes aegypti* measured against different dilutions of (2,5-dimethyl-2,3-dihydro-1H-inden-2yl)methanol in the K&D test is shown in the following Table 2.

**Table 2 - in-vitro repulsion of Aedes aegypti is measured against different dilutions of (2,5-dimethyl-2,3-dihydro-1H-inden-2yl)methanol in the K&D test.**

| **compound** | **% concentration** | **% repulsion** | **% ± SD** |
|---|---|---|---|
| 2,5-dimethyl-2,3-dihydro-1H-inden-2yl)methanol | 0.02 | 23.3 | 11.5 |
| | 0.1 | 27.8 | 14.2 |
| | 0.5 | 54.4 | 13.2 |
| | 2.5 | 82.2 | 8.3 |
| | 10 | 93.3 | 5.9 |
| ethanol | 100 | 13.5 | 7.8 |

The *in-vivo* repulsion of mosquitoes *Aedes aegypti* measured against different dilutions of (2,5-dimethyl-2,3-dihydro-1H-inden-2yl)methanol in the K&D test according to Table 2 is illustrated in Figure 2.

The percentage of repulsion of *Aedes aegypti* increases with the increase tested amount of (2,5-dimethyl-2,3-dihydro-1H-inden-2yl)methanol demonstrating the biological effect of (2,5-dimethyl-2,3-dihydro-1H-inden-2yl)methanol *(cf.* Table 2 and Figure 2).

At low concentrations (0.02% and 0.1%), the repulsion was above the repulsion elicited by the solvent, but with high variations not allowing to claim a clear repellent action of (2,5-dimethyl-2,3-dihydro-1H-inden-2yl)methanol on its own at these concentrations *(cf.* Table 2 and Figure 2). Nevertheless, at concentration of (2,5-dimethyl-2,3-dihydro-1H-inden-2yl)methanol above 0.1%, the percentage of repulsion was consistently higher than the percentage of repulsion elicited by the solvent ethanol *(cf.* Table 2 and Figure 2).

Using the K&D *in-vivo* assay, (2,5-dimethyl-2,3-dihydro-1H-inden-2yl)methanol has shown on its own a significant repellent effect against *Aedes aegypti* as soon as its concentration was equal or above 0.5% in ethanol.

### 4.3. In vitro Warm Body assay:

The *in-vitro* mean numbers of mosquitoes *Aedes aegypti* landing on the Warm Body loaded with different dilutions of DEET, IR3535, (2,5-dimethyl-2,3-dihydro-1H-inden-2yl)methanol or the solvent ethanol is shown in the following Table 3. Due to variation between tests, the number of mosquitoes landing during control with 100% ethanol was used to normalize the number of mosquitoes landing with the stimuli.

| | **% of mosquitoes' repellency** | | |
|---|---|---|---|
| **% concentration** | DEET | IR3535 | (2,5-dimethyl-2,3-dihydro-1H-inden-2yl)methanol |
| 0.00032 | 17% | 8% | 18% |
| 0.00071 | 10% | n.a. | 46% |
| 0.0016 | 5% | 0% | 14% |
| 0.0036 | 19% | n.a. | 46% |
| 0.0053 | 46% | n.a. | 0% |
| 0.0080 | 61% | 0% | 26% |
| 0.018 | 79% | n.a. | 26% |
| 0.040 | 89% | 69% | 61% |
| 0.089 | 97% | n.a. | 60% |
| 0.20 | 98% | 78% | 80% |
| 0.45 | 95% | n.a. | 93% |
| 1 | 97% | 89% | 91% |

| | | | |
|---|---|---|---|
| Table 3 - percentage of repellency of mosquitoes *Aedes aegypti* after application of different dilutions of DEET, IR3535 or (2,5-dimethyl-2,3-dihydro-1H-inden-2yl)methanol in the *in-vitro* WBA. 100% correspond to the number of mosquitoes landing during the control test with 100% ethanol. *n.a.* means no test was carried at this concentration. | | | |

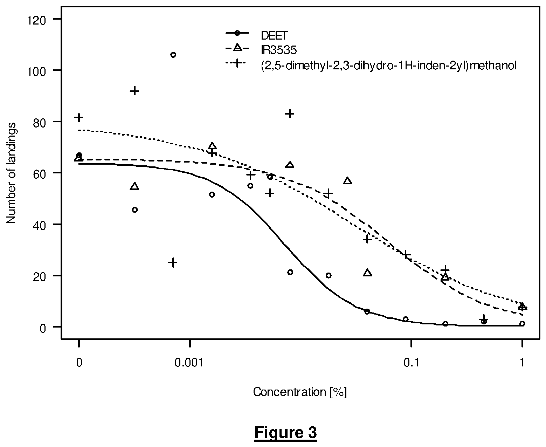

The number of mosquitoes *Aedes aegypti* landing on the warm body decreases with the increase tested amount of stimuli demonstrating the biological effect of all stimuli *(cf.* Table 3 and Figure 3).

At concentrations lower than 0.04%, IR3535 and (2,5-dimethyl-2,3-dihydro-1H-inden-2yl)methanol showed only low repulsion *(cf.* Table 3 and Figure 3). At concentration of stimuli equal or above 0.20%, the repulsion was consistently higher with less mosquitoes landing on the warm body loaded with the tested compounds *(cf.* Table 3 and Figure 3).

Using the Warm Body *in-vivo* assay, perfumes containing (2,5-dimethyl-2,3-dihydro-1H-inden-2yl)methanol demonstrated a significant repellent effect against *Aedes aegypti* similar to the repellent effect of IR3535.

### 4.4. In vitro Warm Body assay:

The *in-vitro* mean ± SD numbers of mosquitoes *Aedes aegypti* landed on the Warm Body loaded with different dilutions of two different perfumes containing 33% & 10% of (2,5-dimethyl-2,3-dihydro-1H-inden-2yl)methanol (*Perfumes 1a & 1b* respectively), two reference compounds: DEET & IR3535 or the solvent ethanol is shown in the following Table 4.

| | 0.0016% | 0.04% | 1% | 100% |
|---|---|---|---|---|
| Perfume 1a | 62.3±3.1 | 26.3±3.9 | 6.7±1.9 | |
| Perfume 1b | 58.3±3.5 | 23.0±3.5 | 7.0±1.3 | |
| IR3535 | 70.0±14. 3 | 20.7±2.1 | 7.7±1.6 | |
| DEET | 53.3±12. 5 | 5.7±1.0 | 0.7±0.3 | |
| ethanol | | | | 66.2±7.5 |

| | | | | |
|---|---|---|---|---|
| Table 4 - *In-vitro* mean±SD numbers of mosquitoes *Aedes aegypti* landed on the Warm Body loaded with different dilutions of two different perfumes containing 33% & 10% of (2,5-dimethyl-2,3-dihydro-1H-inden-2yl)methanol *(Perfumes 1a & 1b* respectively), two reference compounds: DEET & IR3535 or the solvent ethanol. | | | | |

The *In-vitro* repulsion of mosquitoes *Aedes aegypti* measured in the Warm Body Assay according to Table 4 is illustrated in the following Figure 4.

The number of mosquitoes *Aedes aegypti* landing on the warm body increases with the increase tested amount of stimuli demonstrating the biological effect of the stimuli *(cf.* Table 4 and Figure 4).

At low concentrations (0.0016%), none of the stimulus tested elicited repulsion; the number of landed mosquito was similar between all stimuli and the solvent ethanol (cf. Table 4 and Figure 4). Nevertheless, at concentration of stimuli equal or above 0.04%, the repulsion was consistently higher with less mosquitoes landing on the warm body loaded with the tested stimuli compared to ethanol *(cf.* Table 4 and Figure 4). The two perfumes containing (2,5-dimethyl-2,3-dihydro-1H-inden-2yl)methanol showed a controlling effect similar to IR3535 at iso-dose *(cf.* Table 4 and Figure 4).

Using the Warm Body *in-vivo* assay, perfumes containing (2,5-dimethyl-2,3-dihydro-1H-inden-2yl)methanol demonstrated a significant controlling effect against *Aedes aegypti* similar to the controlling effect of IR3535.

### 4.5. In vitro Warm Body assay, test in combination with known repellent:

The percentage of mosquitoes *Aedes aegypti* landing on the Warm Body loaded with 5µL of blends made of 1% of one perfume containing 33% or 6.5% of (2,5-dimethyl-2,3-dihydro-1H-inden-2yl)methanol *(Perfumes 1a & 1e* respectively) and 7.5% or 12.5% of IR3535 are illustrated in Figure 5.

The numbers of mosquitoes *Aedes aegypti* landing on the warm body increase overtime. Less than 60% of mosquitoes landed on the Warm Body assay baited 6 hours earlier, demonstrating a repellent effect of the three different blends *(cf.* Figure 5).

The percentage of mosquitoes *Aedes aegypti* landing on the Warm Body 4h post application of 5µL of blends made of 0.25% or 1% perfume 1a containing 33% of (2,5-dimethyl-2,3-dihydro-1H-inden-2yl)methanol and 12.5% of IR3535 are illustrated in Figure 6.

The numbers of mosquitoes *Aedes aegypti* landing on the warm body decrease with the increase of the perfume 1a in the formulation *(cf.* Figure 6).

### 4.6. In vitro Warm Body assay, test in combination with co-ingredient which contributes to both, the arthropod control activity and to the olfactory character of the composition:

The percentage of mosquitoes *Aedes aegypti* landing on the Warm Body loaded with a blend made of 50% of (2,5-dimethyl-2,3-dihydro-1H-inden-2yl)methanol + 50% of methyl (1R)-cis-3-oxo-2-pentyl-1-cyclopentaneacetate or pure methyl (1R)-cis-3-oxo-2-pentyl-1-cyclopentaneacetate demonstrated a similar repellent effect at the 3 concentrations tested as illustrated in Figure 7.

Therefore, replacement of half of (1R)-cis-3-oxo-2-pentyl-1-cyclopentaneacetate by 2,5-dimethyl-2,3-dihydro-1H-inden-2yl)methanol did not impact the repellent efficacy of the stimulus but advantageously improve its hedonic.

### 4.7. In-vitro Warm body assay with two additional sets of arthropod controlling compounds

In this in-vitro assay, the number of *Aedes aegypti* landing on a warm body treated with the tested stimuli was measured in order to assess the repellency effect. Three replicates were carried on the three different doses: 0.0016%, 0.04% and 1% in ethanol.

Using the in-vitro Warm Body assay, additional compounds demonstrated a variable repellent effect against *Aedes aegypti.* All tested compounds demonstrated a significant repellent effect.

The number of mosquitoes *Aedes aegypti* landing on the Warm Body decreases with the increase amount of stimuli tested demonstrating the biological effect of the stimuli (Figures 8, 9 and 10).

### 4.7.1. First set of arthropod controlling compounds

At low concentrations (0.0016%), two compounds, 4a,8-dimethyl-indano[1,2-d]-1,3-dioxan-2-one and (2,4,6-trimethyl-2,3-dihydro-1H-inden-2-yl)methanol, provided a decrease of landing >25%, similar to DEET (Figure 8).

At a concentration of 0.04%, (2,4,6-trimethyl-2,3-dihydro-1h-inden-2-yl)methanol continued to be as efficient as DEET to avoid mosquitoes' landing with a decrease of 88% and 92% of landings respectively (Figure 5). The four additional compounds tested at 0.04%, 4a,8-dimethyl-indano[1,2-d]-1,3-dioxan-2-one, 5-ethyl-2-methyl-2-indanmethanol, 1-(2,5-dimethyl-2-indanyl)-1-ethanol and (2,5-dimethyl-2,3-dihydro-1H-inden-2yl)methanol, elicited a decrease of landing of ~70% similar to the effect of IR3535 (Figure 8).

All compounds and references tested at 1% demonstrated a great avoidance by the mosquitoes with less than 15% of the mosquitoes landing on the warm body (Figure 8).

It is also worth noticing that no mosquito landed when (2,4,6-trimethyl-2,3-dihydro-1H-inden-2-yl)methanol was applied at 1% (Figure 8).

### 4.7.2. Second set of arthropod controlling compounds

At low concentrations (0.0016%), the five tested compounds, (2,5-dimethyl-2-indanyl)methyl formate, (2,5-dimethyl-2,3-dihydro-1H-inden-2-yl)methyl methyl ether, 2,5-dimethyl-2,3-dihydro-1h-indene-2-carboxylic acid, (2,5-dimethyl-2,3-dihydro-1H-inden-2yl)methanol and 5-isopropyl-2-methyl-2-indanmethanol, provided a decrease of landing similar or higher than IR3535 with 4% to 29% decrease of landings (Figure 9).

At a concentration of 0.04%, the compounds decreased the number of landing by 29% to 52%, but this decrease was lower than the one obtained by IR3535 or DEET (Figure 9).

At a concentration of 1%, all compounds decreased the number of landing by more than 50%, (2,5-dimethyl-2,3-dihydro-1H-inden-2-yl)methyl methyl ether eliciting an effect similar to IR3535: 86% landing decrease (Figure 9).

### 4.7.3 Third set of arthropod controlling compounds

2-(hydroxymethyl)-2,6-dimethyl-2,3-dihydro-1H-inden-1-ol (orange) and 5-tert-butyl-2-methyl-2-indanmethanol (pink), showed a low decrease of landing with the increase amount of stimuli tested (Figure 10).

Other stimuli such as a blend of (1RS,2RS)-2-((2-methoxypropan-2-yloxy)methyl)-2,6-dimethyl-2,3-dihydro-1H-inden-1-ol + (1RS,2SR)-2-((2-methoxypropan-2-yloxy)methyl)-2,6-dimethyl-2,3-dihydro-1H-inden-1-ol (brown) and a blend of (1RS,2RS)-2-(hydroxymethyl)-2,6-dimethyl-2,3-dihydro-1H-inden-1-ol + (1RS,2SR)-2-(hydroxymethyl)-2,6-dimethyl-2,3-dihydro-1H-inden-1-ol (green), demonstrated a decreases of landing with the increasing amount of stimuli but with a significant lower repellent effect than DEET or IR3535 (Figure 10).

The compounds of the third set of arthropod controlling compounds showed a significant repellent effect. Compared to the first two sets of compounds mentioned above, however, these compounds elicited a lower repellency effect (Figures 8, 9 and 10).

### 4.7.4 Fourth set of arthropod controlling compounds

**Table 6 - In-vitro number of mosquitoes Aedes aegypti landed on the Warm Body loaded with three dilutions of compounds. The control was made with pure ethanol (used as solvent) applied to the warm plate.**

| | ethanol | 0.0016% | 0.04% | 1% |
|---|---|---|---|---|
| (2-methyl-1,3-dihydroinden-2-yl)methanol | 70 | 65 | 16 | 1 |
| (+-)-(2,6-dimethyl-1,2,3,4-tetrahydro-2-naphthalenyl)methanol | 42 | 57 | 65 | 3 |
| (+-)-1-(2,4,5-trimethyl-2,3-dihydro-1H-inden-2-yl)ethanone | 42 | 31 | 5 | 18 |
| (+-)-(5-methyl-2,3-dihydro-1H-inden-2-yl)methanol | 47 | 65 | 7 | 5 |
| (+-)-1-(2,5-dimethyl-2,3-dihydro-1H-inden-2-yl)ethanone | 76 | 119 | 15 | 6 |
| 2,3-dihydro-1*H*-inden-2-ylmethanol | 54 | 32 | 1 | 0 |
| (4aRS,9bSR)-8-methyl-4,4a,5,9b-tetrahydroindeno[1,2-d][1,3]dioxin | 69 | 103 | 8 | 8 |
| 2,3-dihydro-1*H*-indene-2-carbonitrile | 92 | 59 | 13 | 0 |
| (+)-2,4-dimethyl-4,4a,5,9b-tetrahydroindeno[1,2-d][1,3]dioxine + (-)-2,4-dimethyl-4,4a,5,9b-tetrahydroindeno[1,2-dl[1,3]dioxine | 74 | 58 | 37 | 3 |
| 4,4A,5,9B-tetrahydro-indeno[1,2-d]-1,3-dioxin (ingredient #3) | 80 | 76 | 6 | 2 |
| (4aRS,9bRS)-2,2,4a,7-tetramethyl-4,4a,5,9b-tetrahydroindeno[1,2- | 41 | 51 | 44 | 8 |
| d][1,3]dioxine | | | | |
| (4aRS,9bRS)-4a,7-dimethyl-4,4a,5,9b-tetrahydroindeno(1,2-d][1,3]dioxine | 61 | 17 | 0 | 0 |
| (2RS,4aRS,9bRS)-2,4a,7-trimethyl-4,4a,5,9b-tetrahydroindeno[1,2-d][1,3]dioxine + (2RS,4aSR,9bSR)-2,4a,7-trimethyl-4,4a,5,9b-tetrahydroindeno[1,2-d][1,3]dioxine | 55 | 30 | 0 | 0 |
| (2RS,4aSR,9bSR)-2,4a-dimethyl-4,4a,5,9b-tetrahydroindeno[1,2-d][1,3]dioxin | 51 | 34 | 18 | 6 |
| (4aRS,9bRS)-2,2,4a-trimethyl-4,4a,5,9b-tetrahydroindeno[1,2-d][1,3]dioxine | 49 | 42 | 47 | 2 |
| (2RS,4aRS,9bSR)-2-methyl-4,4a,5,9b-tetrahydroindeno[1,2-d][1,3]dioxin + (2RS,4aSR,9bRS)-2-methyl-4,4a,5,9b-tetrahydroindeno[1,2-d][1,3]dioxin | 46 | 28 | 2 | 2 |
| (2RS,4aSR,9bRS)-2-ethyl-4,4a,5,9b-tetrahydroindeno[1,2-d][1,3]dioxine + (2RS,4aRS,9bSR)-2-ethyl-4,4a,5,9b-tetrahydroindeno[1,2-d][1,3]dioxine | 46 | 43 | 6 | 5 |
| (4aRS,9bSR)-2,2-dimethyl-4,4a,5,9b-tetrahydroindeno[1,2-d][1,3]dioxin | 61 | 30 | 1 | 0 |
| (2RS,4aSR,9bRS)-2,8-dimethyl-4,4a,5,9b-tetrahydroindeno[1,2-d][1,3]dioxine + 2RS,4aRS,9bSR)-2,8-dimethyl-4,4a,5,9b-tetrahydroindeno[1,2-d][1,3]dioxine | 40 | 38 | 14 | 4 |
| (2RS,4aRS,9bSR)-2-ethyl-8-methyl-4,4a,5,9b-tetrahydroindeno[1,2-d][1,3]dioxin + (2RS,4aSR,9bRS)-2-ethyl-8-methyl-4,4a,5,9b-tetrahydroindeno[1,2-d][1,3]dioxin | 51 | 45 | 24 | 10 |
| (2R,4aS,9bS)-2,4a,8-trimethyl-4,4a,5,9b-tetrahydroindeno[1,2-d][1,3]dioxin | 50 | 40 | 8 | 10 |
| (4aRS,9bRS)-4a,8-dimethyl-4,4a,5,9b-tetrahydroindeno[1,2-d][1,3]dioxin | 50 | 34 | 20 | 5 |
| (2RS,4RS,4ARS,9BSR)-2,4-dimethyl-4,4a,5,9b-tetrahydroindeno[1,2-d][1,3]dioxine | 40 | 17 | 13 | 0 |
| (2RS,4RS,4ASR,9BRS)-2,4-dimethyl-4,4a,5,9b-tetrahydroindeno[1,2-d][1,3]dioxine | 63 | 11 | 6 | 2 |
| (4aRS,9bRS)-2,2,4a,8-tetramethyl-4,4a,5,9b-tetrahydroindeno[1,2-d][1,3]dioxine | 48 | 59 | 12 | 11 |

At the exception of (2,6-dimethyl-1,2,3,4-tetrahydro-2-naphthalenyl)methanol, (4aRS,9bRS)-2,2,4a,7-tetramethyl-4,4a,5,9b-tetrahydroindeno[1,2-d][1,3]dioxine and (4aRS,9bRS)-2,2,4a-trimethyl-4,4a,5,9b-tetrahydroindeno[1,2-d][1,3]dioxine, all compounds tested managed to reduce the number of landing of *A. aegypti* by more than 50% at the intermediate dose of 0.04%. This reduction was even above 90% for seven compounds; 2,3-dihydro-1H-inden-2-ylmethanol, the blend of (2RS,4aRS,9bSR)-2-methyl-4,4a,5,9b-tetrahydroindeno[1,2-d][1,3]dioxin + (2RS,4aSR,9bRS)-2-methyl-4,4a,5,9b-tetrahydroindeno[1,2-d][1,3]dioxin, (4aRS,9bSR)-2,2-dimethyl-4,4a,5,9b-tetrahydroindeno[1,2-d][1,3]dioxin, (2RS,4RS,4ASR,9BRS)-2,4-dimethyl-4,4a,5,9b-tetrahydroindeno[1,2-d][1,3]dioxine and 4,4A,5,9B-tetrahydro-indeno[1,2-d]-1,3-dioxin. And this reduction was maximal with two compounds as no mosquito landed at all; (4aRS,9bRS)-4a,7-dimethyl-4,4a,5,9b-tetrahydroindeno[1,2-d][1,3]dioxine & the blend of (2RS,4aRS,9bRS)-2,4a,7-trimethyl-4,4a,5,9b-tetrahydroindeno[1,2-d][1,3]dioxine + (2RS,4aSR,9bSR)-2,4a,7-trimethyl-4,4a,5,9b-tetrahydroindeno[1,2-d][1,3]dioxine (Table 6).

At 1%, sixteen compounds decreased the number of landing of *A. aegypti* by more than 90% (Table 6). At this dose of 1%, five ingredients; 2,3-dihydro-1H-inden-2-ylmethanol, 2,3-dihydro-1H-indene-2-carbonitrile, (4aRS,9bRS)-4a,7-dimethyl-4,4a,5,9b-tetrahydroindeno[1,2-d][1,3]dioxine, (2RS,4RS,4ARS,9BSR)-2,4-dimethyl-4,4a,5,9b-tetrahydroindeno[1,2-d][1,3]dioxine & the blend of (2RS,4aRS,9bRS)-2,4a,7-trimethyl-4,4a,5,9b-tetrahydroindeno[1,2-d][1,3]dioxine + (2RS,4aSR,9bSR)-2,4a,7-trimethyl-4,4a,5,9b-tetrahydroindeno[1,2-d][1,3]dioxine even managed to keep the number of mosquito landing to zero (Table 6).

### 4.8 In-vitro Warm Body Assay with blends

In this in-vitro assay, the number of *Aedes aegypti* landing on a warm body treated with the tested stimuli was measured in order to assess the repellency effect. One replicate was carried at different doses ranging from 0.0016% up to 1% in ethanol.

Using the in-vitro Warm Body assay, different stimuli containing one ingredient related to formula I demonstrated a variable repellent effect against *A. aegypti.* The trend of the number of mosquitoes *A. aegypti* landing on the Warm Body decreases with the increase amounts of stimuli tested demonstrating the biological effect of the stimuli (Table 7).

**Table 7 - In-vitro number of mosquitoes Aedes aegypti landed on the Warm Body loaded with different dilutions of stimuli. The stimuli were five single compounds: (2,5-dimethyl-2,3-dihydro-1H-inden-2yl)methanol (ingredient #2), 4,4A,5,9B-tetrahydro-indeno[1,2-D]-1,3-dioxin (ingredient #3), (+)-methyl (1R)-cis-3-oxo-2-pentyl-1-cyclopentaneacetate (ingredient #4), 3-[N-n-butyl-N-acetyl]aminopropionic acid ethylester (IR3535), 2-(2-hydroxypropan-2-yl)-5-methylcyclohexan-1-ol (PMD); three blends of these compounds: blend #1 (= ingredients #2 & #4 [1:1]), blend #2 (= ingredient #2 & IR3535 [1:1]) & blend #3 (= ingredient #3 & PMD [3:1]); three perfumes containing 6.5% of (2,5-dimethyl-2,3-dihydro-1H-inden-2yl)methanol (Perfumes 1e), or 0.22% & 0.4% of 4,4A,5,9B-tetrahydro-indeno[1,2-D]-1,3-dioxin (Perfumes 5f & 5g respectively). The control was made with pure ethanol (used as solvent) applied to the warm body. n.d. indicates that no test was carried at this specific concentration.**

| | **control** | **0.0016%** | **0.008%** | **0.0119%** | **0.04%** | **0.0598%** | **0.2%** | **0.447%** | **1%** |
|---|---|---|---|---|---|---|---|---|---|
| **ingredient #2** | 85 | 68 | 61 | n.d. | 22 | n.d. | 9 | 3 | 8 |
| **ingredient #3** | 80 | 76 | 61 | 42 | 6 | 4 | 0 | 2 | 2 |
| **ingredient #4** | 65 | 66 | 53 | n.d. | 16 | n.d. | 17 | n.d. | 4 |
| **IR3535** | 70 | 70 | 63 | 22 | 21 | 6 | 9 | 0 | 8 |
| **PMD** | 62 | 58 | 40 | 36 | 8 | 1 | 1 | n.d. | 0 |
| **blend #1** | 69 | n.d. | 45 | 33 | 20 | 19 | 11 | 5 | n.d. |
| **blend #2** | 57 | 25 | n.d. | n.d. | 3 | n.d. | n.d. | n.d. | 1 |
| **blend #3** | 43 | 10 | n.d. | n.d. | 0 | n.d. | n.d. | n.d. | 0 |
| **Perfume 1e** | 55 | 37 | n.d. | n.d. | 39 | n.d. | n.d. | n.d. | 1 |
| **perfume 5f** | 59 | 51 | 36 | n.d. | 32 | n.d. | 9 | n.d. | 4 |
| **perfume 5g** | 66 | n.d. | 43 | n.d. | 22 | n.d. | 4 | n.d. | n.d. |

All blends and perfumes at a concentration above 0.2% elicited a significant repellent effect as they allowed to decrease the number of landing by more than 80% (Table 7). This threshold was even reach at lower concentrations with the blend containing a known insect repellent compounds *i.e.* blends #2 & #3 with 80% reductions at a concentration of 0.04%.

On top of that, at low concentrations (0.0016%), blends #2 & #3 display a significant higher decrease of landings compared to the single compounds composing the blends e.g. 77% reduction of landing with blend #3 versus 5% and 11% respectively for 4,4A,5,9B-tetrahydro-indeno[1,2-D]-1,3-dioxin and 2-(2-hydroxypropan-2-yl)-5-methylcyclohexan-1-ol at this same dose.

It is also worth noticing that no mosquito landed when the blend #3 was applied at 0.04% and 1% (Table 7).

Using the same *in-vitro* setup, the blend #3 made of 75% of 4,4A,5,9B-tetrahydro-indeno[1,2-D]-1,3-dioxin and 25% of 2-(2-hydroxypropan-2-yl)-5-methylcyclohexan-1-ol was assessed overtime.

**Table 8 - In-vitro number of mosquitoes Aedes aegypti landed on the Warm Body loaded with a blend of 4,4A,5,9B-tetrahydro-indeno[1,2-D]-1,3-dioxin (75%) and 2-(2-hydroxypropan-2-yl)-5-methylcyclohexan-1-ol (25%) at different time post-application. The control was made with pure ethanol (used as solvent) applied to the warm body.**

| | **control** | **Time post-application** | | |
|---|---|---|---|---|
| | | **2h** | **4h** | **6h** |
| **blend #3** | 59.0 | 0.0 | 0.0 | 60.0 |

As displayed in the Table 8, no mosquito *Aedes aegypti* landed on the warm body 2h & 4h after application of the blend #3. Six hours after application, the blend was not efficient anymore with the same number of landing than with the control.

### 4.9 In-Vivo Arm in cage test

A solution made of 20% of (2,5-dimethyl-2,3-dihydro-1H-inden-2yl)methanol diluted in ethanol was applied to the forearm of three volunteers. As displayed in Table 15, (2,5-dimethyl-2,3-dihydro-1H-inden-2yl)methanol allowed to reduce the number of mosquitoes landing on the volunteers' arm by more than 90% for up to 7h.

**Table 15: Percentage of repellency overtime of A. aegypti to 20% of (2,5-dimethyl-2,3-dihydro-1H-inden-2yl)methanol in an arm in cage test. Three different volunteers were tested.**

| **Time Post Application** | **% of repellency** | |
|---|---|---|
| | **MEAN** | **±SD** |
| **0.05 h** | 99.1 | 0.22 |
| **1 h** | 98.83 | 0.23 |
| **2 h** | 97.79 | 0.95 |
| **3 h** | 96.72 | 2.34 |
| **4 h** | 94.71 | 0.68 |
| **5 h** | 94.05 | 1.62 |
| **6 h** | 91.99 | 2.47 |
| **7 h** | 90.91 | 2.22 |
| **8 h** | 84.67 | 3.87 |

### 5 In-vitro Warm plate assay with two arthropod controlling compounds

### 5.1 In-vitro Warm Plate Assay on single compounds

The mean ± SD number of ticks *Ixodes ricinus* walking down (affected), falling (affected) or walking-up (non affected) from the warm plate treated with ethanol, N,N-diethyl-3-methylbenzamide, (2,5-dimethyl-2,3-dihydro-1H-inden-2yl)methanol (Ingredient 2) or (2,4,6-trimethyl-2,3-dihydro-1H-inden-2-yl)methanol (Ingredient 1) diluted in ethanol at 0.4mg/mL and 10 mg/mL was assessed on n groups of 12 ticks (n=2-5).

**Table 5 - In-vitro mean ± SD numbers of ticks Ixodes ricinus affected on the Warm Plate loaded with 0.04% or 1% of two ingredients: (2,5-dimethyl-2,3-dihydro-1H-inden-2yl)methanol & (2,4,6-trimethyl-2,3-dihydro-1H-inden-2-yl)methanol or one reference compound: DEET or the solvent ethanol.**

| | **concentration [mg/mL]** | ***n=*** | **MEAN % affected** | **+/-SD % affected** |
|---|---|---|---|---|
| **Ethanol** | 1000 | 5 | 7% | 3% |
| **N,N-diethyl-3-methylbenzamide** | 0.4 | 2 | 50% | 0% |
| **(DEET)** | 10 | 2 | 96% | 3% |
| **(2,5-dimethyl-2,3-dihydro-1H-inden-2yl)methanol (ingredient 2)** | 0.4 | 2 | 21% | 3% |
| | 10 | 2 | 92% | 0% |
| **(2,4,6-trimethyl-2,3-dihydro-1H-inden-2-yl)methanol (Ingredient 1)** | 0.4 | 2 | 67% | 6% |
| | 10 | 2 | 100% | 0% |

Only 6.6% ± 3.5% of the ticks were affected when the warm plate was treated with the solvent ethanol; a mean of 0.8 tick over the 12 ticks tested per group did not display a natural behavior of feeding spot seeking (Table 5 & Figure 11).

On the other hand, the two ingredients tested as well as DEET elicited a repellent effect at the two concentrations tested (0.04% and 1%; Table 5 & Figure 11).

(2,5-dimethyl-2,3-dihydro-1H-inden-2yl)methanol was the least effective at 0.04% but only one tick over 12 was not affected at a concentration of 1% on both replicate, reaching a result similar to the one obtained with DEET applied at similar concentration (Table 5 & Figure 11).

(2,4,6-trimethyl-2,3-dihydro-1H-inden-2-yl)methanol was the most efficient, even more than DEET at both concentration tested, all the ticks being affected at high concentration (1%) and only three and five ticks over 12 being non-affected at the lowest concentration tested (0.04%) (Table 5 & Figure 11).

Further compounds were assessed using the Warm Plate Assay on ticks *I. ricinus* applying the protocol mentioned above (Table 9, 10 & 11).

**Table 9 - Percentage of ticks Ixodes ricinus being affected during the in-vitro Warm Plate Assay with the plate loaded with three different compounds at 15 different doses ranging from 0.00032% to 1%. The three compounds were: (2,5-dimethyl-2,3-dihydro-1H-inden-2yl)methanol (ingredient #2), 4,4A,5,9B-tetrahydro-indeno[1,2-D]-1,3-dioxin (ingredient #3) & 2,4-dimethyl-4,4a,5,9b-tetrahydroindeno[1,2-d][1,3]dioxine (ingredient #5). The control was made with pure ethanol (used as solvent) applied to the warm plate.**

| | **ingredient #2** | **ingredient #3** | **ingredient #5** |
|---|---|---|---|
| **control** | 3% | 0% | 0% |
| **0.00032%** | 0% | 0% | 0% |
| **0.00071%** | 15% | 8% | 0% |
| **0.0016%** | 17% | 0% | 17% |
| **0.00357%** | 0% | 0% | 0% |
| **0.0053%** | 15% | 0% | 0% |
| **0.008%** | 0% | 0% | 0% |
| **0.0119%** | 8% | 0% | 0% |
| **0.0178%** | 17% | 0% | 8% |
| **0.0267%** | 38% | 0% | 0% |
| **0.04%** | 0% | 13% | 15% |
| **0.0598%** | 31% | 6% | 0% |
| **0.089%** | 29% | 0% | 7% |
| **0.2%** | 85% | 24% | 38% |
| **0.447%** | 100% | 67% | 71% |
| 1% | 71% | 92% | 62% |

In all tests, we can observe a clear trend of having more ticks *I. ricinus* affected in relation to the increase amounts of stimuli applied on the Warm Plate (Table 9), that demonstrate a clear biological dose response effect of the stimuli. At the highest concentrations, all compounds were efficient to repel the ticks, the threshold being above 0.447% for 4,4A,5,9B-tetrahydro-indeno[1,2-D]-1,3-dioxin & 2,4-dimethyl-4,4a,5,9b-tetrahydroindeno[1,2-d][1,3]dioxine and above 0.2% for (2,5-dimethyl-2,3-dihydro-1H-inden-2yl)methanol (Table 9).

**Table 10 - Percentage of ticks Ixodes ricinus affected on the Warm Plate loaded with three to six concentrations of ten ingredients. The control was made with pure ethanol (used as solvent) applied to the warm plate. n.d. indicates that no test was carried at this specific concentration.**

| | **control** | **0.00032 %** | **0.0016 %** | **0.008 %** | **0.04 %** | **0.2%** | **1%** |
|---|---|---|---|---|---|---|---|
| **2-[(1-methoxyethoxy)methyl]-2,5-dimethylindane** | 4% | 8% | 0% | 21% | 33% | 64% | 100 % |
| **5-isopropyl-2-methyl-2-indanmethanol** | 0% | 0% | 21% | 0% | 71% | 87% | 92% |
| **2-((methoxymethoxy)methyl)-2,5-dimethyl-2,3-dihydro-1H-indene** | 7% | 0% | 17% | 0% | 33% | 54% | 100 % |
| **(2,6-dimethyl-1,2,3,4-tetrahydro-2-naphthalenyl)methanol** | 0% | n.d. | n.d. | n.d. | 8% | 71% | 100 % |
| **1-(2,4,5-trimethyl-2,3-dihydro-1H-inden-2-yl)ethanone** | 0% | n.d. | n.d. | n.d. | 0% | 38% | 92% |
| **(5-methyl-2,3-dihydro-1H-inden-2-yl)methanol** | 0% | n.d. | n.d. | n.d. | 0% | 100 % | 100 % |
| **1-(2,5-dimethyl-2,3-dihydro-1H-inden-2-yl)ethanone** | 0% | n.d. | n.d. | n.d. | 0% | 42% | 100 % |
| **2,3-dihydro-1H-inden-2-yl methanol** | 4% | n.d. | n.d. | n.d. | 29% | 100 % | 92% |
| **(2-methyl-2,3-dihydro-1H-inden-2-yl)methyl acetate** | 8% | n.d. | n.d. | n.d. | 25% | 58% | 100 % |
| **(4aRS,9bSR)-8-methyl-4,4a,5,9b-tetrahydroindeno[1,2-d][1,3]dioxin** | 0% | n.d. | n.d. | n.d. | 8% | 33% | 85% |
| **2,3-dihydro-1H-indene-2-carbonitrile** | 0% | n.d. | n.d. | n.d. | 29% | 71% | 100 % |

In all tests, we can observe a clear trend of having more ticks *I. ricinus* affected in relation to the increase amounts of stimuli applied on the Warm Plate (Table 10), that demonstrate a clear biological dose response effect of the stimuli. At the highest concentration tested (1%), all the eleven compounds affected more than 85% of the ticks (Table 10). (5-methyl-2,3-dihydro-1H-inden-2-yl) methanol, 2,3-dihydro-1H-inden-2-yl methanol & 5-isopropyl-2-methyl-2-indanmethanol managed to reach this level of efficacy at an even lower dose of 0.2% (Table 10).

**Table 11 - Percentage of ticks Ixodes ricinus affected on the Warm Plate loaded with three concentrations of eleven ingredients. The control was made with pure ethanol (used as solvent) applied to the warm plate.**

| | **control** | **0.0016%** | **0.04%** | **1%** |
|---|---|---|---|---|
| **(2-methyl-1,3-dihydroinden-2-yl)methanol** | 0% | 0% | 14% | 92% |
| **5-(tert-butyl)-1,3-dihydrospiro[indene-2,3'-oxetane]** | 0% | 0% | 17% | 86% |
| **1-(2,5-dimethyl-2-indanyl)-1-ethanol** | 0% | 15% | 0% | 100% |
| **(2,5-dimethyl-2-indanyl)methyl formate** | 0% | 0% | 7% | 85% |
| **(2,5-dimethyl-2,3-dihydro-1H-inden-2-yl)methyl methyl ether** | 8% | 0% | 17% | 79% |
| **(2,5,6-trimethyl-2,3-dihydro-1H-inden-2-yl)methanol** | 0% | 17% | 38% | 77% |
| **(2,5-dimethyl-2-indanyl)methyl formate)methanol** | 0% | 0% | 15% | 100% |
| **(2,5-dimethyl-2,3-dihydro-1H-inden-2yl)methanol** | 0% | 8% | 0% | 92% |
| **(+-)-5-tert-butyl-2-methyl-2-indanmethanol** | 0% | 0% | 8% | 92% |
| **(+-)-(2,4,6-trimethyl-2,3-dihydro-1H-inden-2-yl)methanol** | 8% | 20% | 57% | 96% |
| (**+-)-methyl 2,5-dimethyl-2,3-dihydro-1H-indene-2-carboxylate** | 8% | 7% | 18% | 100% |

In all tests, we can observe a clear trend of having more ticks *I. ricinus* affected in relation to the increase amounts of stimuli applied on the Warm Plate (Table 11), that demonstrate a clear biological dose response effect of the stimuli. All the eleven compounds affected more than 75% of the ticks at the 1% concentration (Table 11).

### 5.2 In-vitro Warm Plate Assay on blends

### 5.2.1 tests at different concentrations

In this in-vitro assay, the percentage of ticks *Ixodes ricinus* affected by the stimulus loaded on the warm plate was measured in order to assess the repellency effect. Twelve ticks were assessed per concentrations that goes from 0.0016% up to 1% in ethanol.

In all tests, we can observe a clear trend of having more ticks *I. ricinus* affected in relation to the increase amounts of stimuli applied on the Warm Plate (Table 12 & 13), that demonstrate a clear biological dose response effect of the stimuli.

**Table 12 - Percentage of ticks I. ricinus affected on the Warm Plate by the stimulus loaded at different concentrations. The stimuli were six single compounds: (2,4,6-trimethyl-2,3-dihydro-1H-inden-2-yl)methanol (ingredient #1), (2,5-dimethyl-2,3-dihydro-1H-inden-2yl)methanol (ingredient #2), 4,4A,5,9B-tetrahydro-indeno[1,2-D]-1,3-dioxin (ingredient #3), (+)-methyl (1R)-cis-3-oxo-2-pentyl-1-cyclopentaneacetate (ingredient #4), 3-[N-n-butyl-N-acetyl]aminopropionic acid ethylester (IR3535) & 2-(2-hydroxypropan-2-yl)-5-methylcyclohexan-1-ol (PMD) and three blends of these compounds: blend #1 (= ingredients #2 & #4 [1:1]), blend #2 (= ingredient #2 & IR3535 [1:1]) & blend #3 (= ingredient #3 & PMD [3:1]). The control was made with pure ethanol (used as solvent) applied to the warm plate.**

| | **ingredie nt #1** | **ingredie nt #2** | **ingredie nt #3** | **ingredie nt #4** | **IR353 5** | **PMD** | **blend #1** | **blend #2** | **blend #3** |
|---|---|---|---|---|---|---|---|---|---|
| **control** | 5% | 3% | 0% | 0% | 2% | 3% | 6% | 2% | 0% |
| **0.0016 %** | 20% | 17% | 0% | 15% | 0% | 0% | 0% | 0% | 0% |
| **0.0053 %** | 7% | 15% | 0% | 17% | 0% | 12% | 8% | 0% | 0% |
| **0.008%** | 8% | 0% | 0% | 8% | 8% | 4% | 31% | 29% | 0% |
| **0.0119 %** | 23% | 8% | 0% | 0% | 0% | 4% | 13% | 7% | 6% |
| **0.0178 %** | 0% | 17% | 0% | 33% | 0% | 4% | 40% | 8% | 14% |
| **0.0267 %** | 7% | 38% | 0% | 36% | 0% | 0% | 50% | 21% | 14% |
| **0.04%** | 57% | 0% | 13% | 92% | 33% | 12% | 83% | 17% | 13% |
| **0.0598 %** | 13% | 31% | 6% | 100% | 12% | 19% | 92% | 36% | 43% |
| **0.089%** | 41% | 29% | 0% | 100% | 46% | 42% | 100% | 83% | 46% |
| **0.2%** | 85% | 85% | 24% | 100% | 57% | 84% | 100% | 86% | 63% |
| **0.447%** | 92% | 100% | 67% | 92% | 53% | 96% | 100% | 86% | 92% |
| **1%** | 96% | 71% | 92% | 100% | 56% | 96% | 92% | 100% | 92% |

All blends and perfumes at a concentration above 0.45% elicited a significant repellent effect as they allowed to increase the number of ticks affected above a threshold of 85% (Table 12). This threshold was even reach at lower concentrations with the blend #1 (0.06%) and blend #2 (0.2% ; Table 12).

On top of that, at low concentrations, blends #2 & #3 display a significant higher decrease of landings compared to the single compounds composing the blends e.g. at a dose of 0.89%, 83% of the ticks were affected by blend #2 versus 29% and 46% respectively for (2,5-dimethyl-2,3-dihydro-1H-inden-2yl)methanol and 3-[N-n-butyl-N-acetyl]aminopropionic acid ethylester at this same dose, and similarly at a dose of 0.598%, 43% of the ticks were affected by blend #3 versus 6% and 19% respectively for 4,4A,5,9B-tetrahydro-indeno[1,2-D]-1,3-dioxin and 2-(2-hydroxypropan-2-yl)-5-methylcyclohexan-1-ol at this same dose (Table 12).

**Table 13 - Percentage of ticks I. ricinus affected on the Warm Plate by the stimulus loaded at different concentrations. The stimuli were three perfumes containing 33% of (2,5-dimethyl-2,3-dihydro-1H-inden-2yl)methanol (Perfume 1a), 6.5% of (2,5-dimethyl-2,3-dihydro-1H-inden-2yl)methanol (Perfumes 1e) or 0.4% of 4,4A,5,9B-tetrahydro-indeno[1,2-D]-1,3-dioxin (Perfume 5g). The control was made with pure ethanol (used as solvent) applied to the warm plate. n.d. indicates that no test was carried at this specific concentration.**

| | **control** | **0.00032%** | **0.0016%** | **0.008%** | **0.04%** | **0.2%** | **1%** |
|---|---|---|---|---|---|---|---|
| **perfume 1a** | 0% | 21% | 0% | 8% | 7% | 42% | 100% |
| **perfume 1e** | 0% | n.d. | 0% | n.d. | 0% | n.d. | 100% |
| **perfume 5g** | 4% | 15% | 8% | 33% | 8% | 71% | 92% |

All three perfumes managed to significantly affect the ticks when applied on the warm plate at a dose of 1% (Table 13). Perfumes 1a and 1e even managed to affect all the 12 ticks tested at this dose of 1% (Table 13).

### 5.2.2 tests overtime

Using the same *in-vitro* setup, the effect on ticks behavior (n≥12) of different stimuli diluted in ethanol at 20% (w/w) was assessed overtime.

**Table 14 - Percentage of ticks I. ricinus (n≥12) affected by the stimulus loaded on the Warm Plate overtime. The stimuli were four single compounds: (2,5-dimethyl-2,3-dihydro-1H-inden-2yl)methanol (ingredient #2), 4,4A,5,9B-tetrahydro-indeno[1,2-D]-1,3-dioxin (ingredient #3), 3-[N-n-butyl-N-acetyl]aminopropionic acid ethylester (IR3535), 2-(2-hydroxypropan-2-yl)-5-methylcyclohexan-1-ol (PMD) & two blends of these compounds: blend #2 (= ingredient #2 & IR3535 [1:1]) & blend #3 (= ingredient #3 & PMD [3:1]) The control was made with pure ethanol (used as solvent) applied to the warm plate.**

| | **control** | **Time post-application** | | | | | |
|---|---|---|---|---|---|---|---|
| | | **0.25h** | **0.5h** | **1h** | **2h** | **4h** | **6h** |
| **Ingredient #2** | 0% | 100% | 83% | 100% | 100% | 0% | 8% |
| **Ingredient #3** | 4% | 100% | 92% | 8% | 17% | 15% | 33% |
| **IR3535** | 4% | 83% | 40% | 83% | 58% | 58% | 0% |
| **PMD** | 0% | 42% | 42% | 83% | 50% | 8% | 17% |
| **Blend #2** | 0% | 100% | 100% | 92% | 100% | 100% | 92% |
| **Blend #3** | 0% | 79% | 100% | 96% | 100% | 62% | 17% |

As displayed in the Table 14, the relevant effect of blend #2 overtime can be mostly attributed to (2,5-dimethyl-2,3-dihydro-1H-inden-2yl)methanol that displayed a better ticks' repellency than the known repellent 3-[N-n-butyl-N-acetyl]aminopropionic acid ethylester in this assay. However, a synergistic effect seems to occur at 3h & 4h as the blend was more effective the two single compounds alone during these time points (Table 14).

Similarly, the good activity at the blend #3 overtime, can be attributed to the dual and synergistic action of 4,4A,5,9B-tetrahydro-indeno[1,2-D]-1,3-dioxin and 2-(2-hydroxypropan-2-yl)-5-methyl cyclohexan-1-ol (Table 14). Indeed, 4,4A,5,9B-tetrahydro-indeno[1,2-D]-1,3-dioxin very effective at the first time points (0.25h & 0.5h) while 2-(2-hydroxypropan-2-yl)-5-methylcyclohexan-1-ol that was not efficient during these first time points but became more efficient later-on and therefore took over to prolongate the efficacy of the blend #3 (Table 14).

### 1. New compounds according to the invention

1.1. 2-(hydroxymethyl)-2,6-dimethyl-2,3-dihydro-1H-inden-1-ol/(1RS,2SR)-2-(hydroxymethyl)-2,6-dimethyl-2,3-dihydro-1H-inden-1-ol
   MS (EI): 192 (M⁺)
1.2. 4a,8-dimethyl-indane[1,2-d]-1,3-dioxan-2-one
   MS (EI): 218 (M⁺)
1.3. (2,5-dimethyl-2-indanyl)methyl formate
   MS (EI): 204 (M⁺)
1.4. 8-methyl-4,4a,5-9b-tetrahydroindeno[1,2-d][1,3]dioxin/(4aRS,9bSR)-8-methyl-4,4a,5,9b-tetrahydroindeno[1,2-d][1,3]dioxin
   MS (EI): 190 (M⁺)
1.5. 5-(tert-butyl)-1,3-dihydrospiro[indene-2,3'-oxetane]
   MS (EI): 216 (M⁺)

### Preparation of compounds disclosed herein

A three-necked flask was charged with LiAlH₄ under N₂ (6.19 g, 163 mmol, 1.0 eq) followed by the addition of 200 mL of dry Et₂O. Then a solution of 2-(hydroxymethyl)-2,6-dimethyl-2,3-dihydro-1H-inden-1-one (31 g, 163 mmol, 1.0 eq) in 150 mL of dry Et₂O was added to the mixture over a 2 hour-period. The resulting grey suspension was kept under stirring during 3 hours at rt.

The reaction mixture was cooled to 0°C and 5 mL H ₂O, 5 mL NaOH, 15 MI H₂O were added in this order.

The suspension was stirred at rt and then treated with MgSO₄. After stirring for 30 min, the white solid was filtered off through celite and washed with Et₂O. Volatiles were removed under reduced pressure to afford the crude desired alcohol as a viscous pale yellow oil.

This residue was purified by Kugelrohr distillation to afford the pure alcohol 2-(hydroxymethyl)-2,5-dimethyl-2,3-dihydro-1H-inden-1-one as a colorless oil (31.3 g, yield 98%).

¹H NMR (CDCl₃, 400 MHz): δ 1.05 (s, 3H), 2.35 (s, 3H), 2.55-2.76 (q, 2H), 3.69 (d, 1H), 3.70 (s, 1H), 5.05 (d, 1H), 7.13-7.18 (m, 3H); ¹³C NMR (CDCl₃, 100 MHz): δ 143.9 (s), 137.3 (s), 136.4 (s), 129.0 (s), 125.0 (d, 2C), 80.2 (s), 70.3 (s), 50.2 (s), 39.9 (s), 21.4 (s), 16.8 (s).

### (4aR,9bR)-4a,7-dimethyl-4,4a,5,9b-tetrahydroindeno[1,2-d][1,3]dioxin-2-one

1,1'-Carbonyldiimidazole (4.5 g, 28 mmol, 1.5 eq) and N,N-dimethylpyridin-4-amine (0.57 g, 5 mmol, 0.25 eq) were dissolved in 88 mL of dichloromethane. The resulting solution was added dropwise to a solution of 2-(hydroxymethyl)-2,5-dimethyl-2,3-dihydro-1H-inden-1-one in 88 mL of dichloromethane over a 30 min-period. The resulting clear mixture was stirred overnight at rt.

The mixture was poured into water, the layers were separated and the aqueous layer was extracted three times with dichloromethane. Combined organic layers were washed with brine, dried over MgSO₄ and concentrated under reduced pressure to give a crude pale yellow solid.

This crude product was purified by flash column chromatography on silica gel to obtain the desired product (4aR,9bR)-4a,7-dimethyl-4,4a,5,9b-tetrahydroindeno[1,2-d][1,3]dioxin-2-one as a viscous oil (1.24 g, yield 31%).

¹H NMR (CDCl₃, 400 MHz): δ 1.39 (s, 3H), 2.35 (s, 3H), 2.08-3.07 (q, 2H), 4.08-4.14 (q, 2H), 5.44 (s, 1H), 7.13-7.18 (m, 3H); ¹³C NMR (CDCl₃, 100 MHz): δ 150.3 (s), 138.6 (s), 137.7 (d, 2C), 131.1 (s), 126.5 (s), 125.1 (s), 91.2 (s), 72.9 (s), 40.6 (s), 22.1 (s), 21.25 (s).

### Methyl 6-methyl-1-oxo-2,3-dihydro-1H-indene-2-carboxylate

A three-necked flask was charged with NaH (10.9 g, 250 mmol, 3.8 eq) followed by the addition of 95 mL of dry THF. The resulting mixture was stirred at 0°C (ice bath) and dimethyl carbonate (47.8 g, 526 mmol, 8.0 eq) was then added, followed by sodium tert-butoxide (0.63 g, 6.57 mmol, 0.1 eq) and a solution of 6-methyl-indanone (9.8 g, 66 mmol, 1.0 eq) in 35 mL of dry THF. The reaction mixture gradually became yellowish and then brownish. Upon complete addition, the ice bath was removed and the reaction mixture was stirred at rt overnight. Further excess of NaH (10.9 g, 250 mmol, 3.8 eq), NaOtBu (0.63 g, 6.57 mmol, 0.1 eq) and dimethyl carbonate (47.8 g, 526 mmol, 8.0 eq) were added and the mixture was heated at 40-42°C for 2 hours. The reaction mixture was stirred at rt overnight.

The reaction mixture was cooled to 0°C and quenched by addition of MeOH (60 mL ), water (60 mL ) and aqueous 1M HCl (90 mL ). The aqueous layer was extracted three times with EtOAc. The combined organic layers were washed twice with water, twice with brine, dried over MgSO₄, filtered and concentrated under reduced pressure.

Flash column chromatography on silica gel afforded Methyl 6-methyl-1-oxo-2,3-dihydro-1H-indene-2-carboxylate as a brownish crystalline product (8.04 g, yield 60%).

### Methyl 1-hydroxy-6-methyl-2,3-dihydro-1H-indene-2-carboxylate

A solution of Methyl 6-methyl-1-oxo-2,3-dihydro-1H-indene-2-carboxylate (8 g, 39 mmol, 1.0 eq) in 100 mL of methanol was cooled at 0°C and NaB H₄ (1.5 g, 39 mmol, 1.0 eq) was added portionwise to the. The mixture was then stirred for 2 h at 0°C and at rt for one additional hour, after which GC analysis showed complete conversion of the starting ketone. The reaction was quenched by careful addition of saturated NH₄Cl and water.

The aqueous solution was extracted three times with EtOAc (500 mL overall). The combined organic layers were washed with water, brine, dried over MgSO₄ , filtered and the solvent was removed under reduced pressure. A pale orange crude oil was obtained.

Flash column chromatography on silica gel afforded the desire hydroxy ester Methyl 1-hydroxy-6-methyl-2,3-dihydro-1H-indene-2-carboxylate as a pale yellow oil (7.02 g, yield 86%).

### 2-(hydroxymethyl)-6-methyl-2,3-dihydro-1H-inden-1-ol

A three-necked flask was charged with LiAlH₄ pellets under N₂ (1.81 g, 48 mmol, 1.4 eq) followed by the addition of 100 mL of dry Et₂O. The mixture was stirred at room temperature until complete dissolution of the LiAlH₄ pellets. The resulting suspension was cooled at 0°C and a solution of hydroxy ester Methyl 1-hydroxy-6-methyl-2,3-dihydro-1H-indene-2-carboxylate in 80 mL of dry Et₂O was added dropwise over a 1 hour-period.

GC analysis showed complete conversion of the starting material.

The reaction mixture was cooled to 0°C and 3 mL H ₂O, 1.5 mL NaOH 15% w/w, 3 mL H₂O were added carrefully in this order.

The suspension was stirred at rt and treated with MgSO₄. After stirring for 30 min, the white solid was filtered off through celite and washed several times with dichloromethane and EtOAc. The organic solution was concentrated under reduced pressure to afford 2-(hydroxymethyl)-6-methyl-2,3-dihydro-1H-inden-1-ol as a pale yellow solid (4.35 g, yield 69%).

### (4aR,9bS)-8-methyl-4,4a,5,9b-tetrahvdroindeno[1,2-d][1,3]dioxine

A two-necked flask was charged with diol 2-(hydroxymethyl)-6-methyl-2,3-dihydro-1H-inden-1-ol under N₂ (0.98 g, 5.5 mmol, 1.0 eq) and 11 mL of dichloromethane. Paraformaldehyde (0.17 g, 5.5 mmol, 1.0 eq) was added, followed by *p*TsOH.H₂O (1.05 g, 5.5 mmol, 1.0 eq) and the mixture was stirred at 35°C for 1 hour. The organic mixture was washed with a saturated aqueous NaHCO₃ solution.The aqueous layer was extracted three times with ether and the combined organic layers were washed twice with brine, dried over MgSO 4 , filtered and concentrated under reduced pressure.

Flash column chromatography on silica gel followed by Kugelrohr distillation afforded (4aR,9bS)-8-methyl-4,4a,5,9b-tetrahydroindeno[1,2-d][1,3]dioxine as colorless solid (0.67g, 64% yield).

¹H NMR (CDCl₃, 400 MHz): δ 2.35 (s, 3H), 2.37-2.42 (m, 1H), 2.84-3.06 (m, 2H), 3.89-4.06 (m, 2H), 4.79 (d, 1H), 4.90-5.03 (m, 2H), 7.09-7.19 (m, 3H); ¹³C NMR (CDCl₃, 100 MHz): δ 141.3 (d, 2C), 136.5 (s), 129.8 (s), 125.1 (d, 2C), 91.1 (s), 80.2 (s), 79.2 (s), 67.6 (s), 38.4 (s), 32.7 (s), 21.2 (s).

### 2,5-dimethyl-2,3-dihydro-1H-inden-2-yl)methyl formate

A mixture of acetic anhydride (1.9 ml, 20mmol, , 4.35 eq) and formic acid (0.74 ml, 20 mmol, 4.35 eq) was stirred at 50°C for 10min, then cooled at 0°C. (2,5-dimethyl-2,3-dihydro-1H-inden-2-yl)methanol (0.84g purity 97%, 4.6 mmol) was added and the mixture was stirred at room temperature for 3.5 hours. The mixture was concentrated under reduced pressure, the residue evaporated twice from toluene to give a colorless liquid. Kugelrohr distillation gave 2,5-dimethyl-2,3-dihydro-1H-inden-2-yl)methyl formate as an oil (0.89 g, yield 89%).

¹H NMR (CDCl₃, 400 MHz): δ 1.20 (s, 3H), 2.30 (s, 3H), 2.63-2.91 (m, 4H), 4.08 (s, 2H), 6.92-7.17 (m, 3H), 8.08 (s, 1H); ¹³C NMR (CDCl₃, 100 MHz): δ 161.4 (s), 142.1 (s), 138.8 (s), 136.1 (s), 127.2 (s), 125.5 (s), 124.6 (s), 70.8 (s), 42.9 (t, 3C), 24.3 (s), 21.2 (s).

### 2,5-dimethyl-2,3-dihydro-1 H-indene-2-carboxylic acid

A three-necked flask was charged with tetrabutylammonium bromide under N₂ (0.915 g, 2.84 mmol, 0.1 eq) and PIPO (0.170 g, 0.58 mmol, 0.02 eq) followed by the addition of 12.5mL of EtOAc. The mixture was stirred at rt for 5 min then (2,5-dimethyl-2,3-dihydro-1H-inden-2-yl)methanol (5.0 g, 28.4 mmol, 1.0 eq) was added. The reaction mixture was kept stirring at rt and NaOCl (36.4 g, 70.9 mmol, 2.5 eq) was added dropwise over a 6.5 hour-period.

The layers were separated. The aqueous one was treated with HCl aq and extracted twice with EtOAc. The combined organic layers were washed with a solution of NaOH 5%, dried over MgSO₄ , filtered and concentrated under reduced pressure to afford after acid-base extraction the carboxylic acid 2,5-dimethyl-2,3-dihydro-1H-indene-2-carboxylic acid as a white solid (2.25 g, purity 87%, yield 36%).

¹H NMR (CDCl₃, 400 MHz): δ 1.40 (s, 3H), 2.34 (s, 3H), 2.78-3.49 (m, 4H), 6.96-7.08 (m, 3H), 11.90 (s, 1H); ¹³C NMR (CDCl₃, 100 MHz): δ 184.5 (s), 141.6 (s), 138.1 (s), 136.5 (s), 127.6 (s), 125.2 (s), 124.4 (s), 49.7 (s), 47.7 (d, 2C), 24.8 (s), 21.2 (s).

5-(*tert*-butyl)-1,3-dihydrospiro[indene-2,3'-oxetane] was prepared as described in J. Org. Chem. 1982, 47, 3090-3094 for the synthesis of 1,3-dihydrospiro[indene-2,3'-oxetane], using 1,2-bis(bromomethyl)-4-(tert-butyl)benzene and diethyl malonate as starting materials.

¹H NMR (CDCl₃, 400 MHz): δ 1.30 (s, 9H), 3.20 (s, 2H), 3.23 (s, 2H), 4.67 (s, 4H), 7.11-7.23 (m, 3H); ¹³C NMR (CDCl₃, 100 MHz): δ 150.0 (s), 141.4 (s), 138.6 (s), 124.0 (d), 123.8 (d), 121.5 (d), 83.8 (t, 2C), 46.9 (s), 44.3 (t), 43.8 (t), 34.6 (s), 31.5 (q, 3C).

## Claims

1. Nontherapeutic use of a composition comprising
- a compound of formula (I) 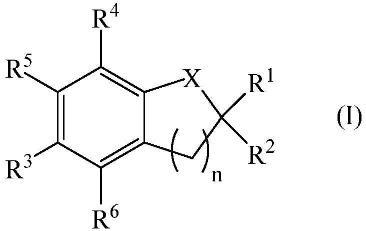
in the form of any one of its stereoisomers or a mixture thereof, and wherein
n represents 1 or 2;
X represents a CH₂, CH(OH) or C=N-OH group;
R¹ represents a CN, CHR⁷OR⁸ or a COR⁹ group,
R⁷ being a hydrogen atom or a methyl group,
R⁸ being a hydrogen atom, a C₁₋₃ hydrocarbon group, optionally substituted by a hydroxyl or a C₁₋₃ alkoxy group, **a** C₃₋₁₀ trialkylsilyl group, or a COR⁹ group, and R⁹ being a hydrogen atom, a hydroxyl group, a C₁₋₃ alkoxy group, a C₁₋₈ hydrocarbon group, or a phenyl, each optionally substituted by one to three groups selected amongst a C₁₋₃ alkyl, alkoxy or amine group;
R² represents a hydrogen atom or a methyl, ethyl or CH₂OH group;
R³ represents a hydrogen atom or a C₁₋₅ hydrocarbon group or a C₁₋₃ alkoxyl group; and
R⁴, R⁵ and R⁶ represents, independently from each other, a hydrogen atom or a C₁₋₅ alkyl or C₂₋₅ alkenyl group; or
R¹ and R² when taken together represent a CH₂-O-C(=O)-O, CH₂-O-CH₂-O or CH₂-O-CH₂ group; or
X and R¹ when taken together represent a CH-O-C(=O)-O-C(R¹⁰)₂ or CH-O-C(R¹⁰) ₂-O-C(R¹⁰) ₂ group wherein R¹⁰, independently from each other, represents a hydrogen atom or a C₁₋₃ alkyl group;
- optionally, at least one arthropod control co-ingredient,
for arthropod control.

2. The nontherapeutic use of the composition according to claim 1, wherein the arthropod is an insect, preferably a mosquito.

3. The nontherapeutic use of the composition according to claim 1, wherein the arthropod is an arachnid, preferably a tick.

4. The nontherapeutic use of the composition according to any one of claims 1 to 3, wherein the composition comprises the compound of formula (I) in an arthropod control effective amount, preferably in an amount of from 0.02 to 80 wt.%, more preferably in an amount of from 0.05 to 70 wt.%, even more preferably in an amount of from 0.1 to 60 wt.%, such as from 0.1 to 5 wt.% or more particular from 0.15 to 4 wt.% or from 11 to 35 wt.% or more particular from 15 to 30 wt.%, based on the total weight of the composition.

5. The nontherapeutic use of the composition according to any one of claims 1 to 4, which comprises at least one arthropod control co-ingredient selected from the group consisting of N,N-diethyl-3-methylbenzamide (DEET), ethyl butylacetylaminopropionate (IR3535); para-menthan-3,8-diol (PMD); 1-(1-methylpropoxycarbonyl)-2-(2-hydroxaethyl)piperidin (Icaridin); Cedarwood oil (China), Cedarwood oil (Texas), Cedarwood oil (Virginia), Cinnamon oil, Citronella oil, Clove oil, Cornmint oil, Cymbopogon winterianus oil, fractionated, hydrated, cyclized, Decanoic acid, Eucalyptus citriodora oil and citronellal, hydrated, cyclized, Eucalyptus citriodora oil, hydrated, cyclized, Eugenol, Garlic oil, Geraniol, Geranium oil, Lavender, Lavandula hybrida, ext./Lavandin oil, Lemon oil, Lemongrass oil, Margosa extract, Mentha arvensis, ext., Metofluthrin, Mixture of cis- and trans-p-menthane-3,8 diol, N,N-diethyl-meta-toluamide, Nonanoic acid, Peppermint oil, Pyrethrins and Pyrethroids, Rosemary oil, Spearmint oil, Thyme oil, Wintergreen oil, 2,3,4,5-bis(butyl-2-ene)tetrahydrofurfural (MGK Repellent 11), cineole, cinnamaldehyde, citral, Citronellal, citronellol, Citronella oil Java, coumarin, dibutyl phthalate, diethyl phthalate, dimethyl anthranilate, dimethyl phthalate, ethyl vanillin, eucalyptus oil, delta - octalactone, delta - nonalactone, delta - decalactone, delta - undecalactone , delta - dodecalactone, gamma - octalactone, gamma - nonalactone, gamma - decalactone, gamma - undecalactone , gamma - dodecalactone, hydroxy citronellal, lime oil, limonene, linalool, methyl anthranilate, Mint arvensis, Mint oil, Mint piperita, Mint spicata, Myrcene, Neem oil, Sabinene, β-Caryophyllene, (1H-indol-2-yl)acetic acid, anethole, anise oil, basil oil, bay oil, camphor, ethyl salicylate, evergreen oils (pine oil) , (1,3,4,5,6,7-hexahydro-1,3-dioxo-2H-isoindol-2-yl)methyl (1R-trans)-2,2-dimethyl-3-(2-methylprop-1-enyl)cyclopropanecarboxylate (d-Tetramethrin), (RS)-3-Allyl-2-methyl-4-oxocyclopent-2-enyl-(1R,3R;1R,3S)-2,2-dimethyl-3-(2- methylprop-1-enyl)-cyclopropanecarboxy late (mixture of 4 isomers 1R trans, 1R:1R trans, 1S:1R cis, 1R: 1R cis, 1S 4:4:1:1) (d-Allethrin), (RS)-α-cyano-3phenoxybenzyl-(1RS)-cis, trans-3-(2,2-dichlorovinyl)-2,2-dimethylcyclopropanecarboxylate (Cypermethrin), 2-methyl-4-oxo-3-(prop-2-ynyl)cyclopent-2-en-1-yl 2,2-dimethyl-3-(2-methylprop-1-enyl)cyclopropanecarboxylate (Prallethrin), Acetamiprid, Azadirachtin, Bendiocarb, Bifenthrin, Boric Acid, Chlorpyrifos, deltamethrin, Diazinon, Dichlorvos, Eugenol, fipronil, imidacloprid, Linalool, Malathion, Maltodextrin, Margosa extract, Metofluthrin, Nicotine, Permethrin, Pyrethrins and Pyrethroids, rotenone, Silicium dioxide (Silicium dioxide/Kieselguhr), S-Methoprene, Spinosad (Spinosyn A), Spinosyn D, Tetramethrin, Transfluthrin.

6. The nontherapeutic use of the composition according to any one of claims 1 to 5, which comprises N,N-diethyl-3-methylbenzamide (DEET), ethyl butylacetylaminopropionate (IR3535), para-menthan-3,8-diol (PMD) or 1-(1-methylpropoxycarbonyl)-2-(2-hydroxaethyl)piperidin (Icaridin), preferably in an amount of from 0.02 to 80 wt.%, more preferably in an amount of from 0.05 to 70 wt.%, even more preferably in an amount of from 0.1 to 60 wt.%, based on the total weight of the composition.

7. The nontherapeutic use of the composition according to any one of claims 1 to 6, which comprises at least one arthropod control co-ingredient which contributes to both, the arthropod control activity and to the olfactory character of the composition.

8. The nontherapeutic use of the composition according to claim 7, wherein the at least one arthropod control co-ingredient is selected from the group consisting of
- an acid perfuming raw material family selected from the group consisting of pentanoic to dodecanoic acids, preferably hexanoic acid, heptanoic acid, octanoic acid and nonanoic acid,
- an alkene perfuming raw material family selected from the group consisting of 1-isopropyl-4-methyl-1,3-cyclohexadiene, 4-isopropenyl-1-methylcyclohexene, 4-isopropylidene-1-methylcyclohexene, and beta-ocimene,
- an alcohol and phenol perfuming raw material family selected from the group consisting of 1-decanol, 2-methyl-4-phenyl-2-butanol, 8-p-menthen-2-ol, 3,4-dimethylphenol, 3,7-dimethyl-2,6-octadien-1-ol, 2-isopropylphenol, 2-methoxy-4-vinylphenol, 1,8-p-menthadien-7-ol, 4-ethylphenol, 4-methylphenol, 4-(trimethyl-1-cyclohexen-1-yl)-2-butanone and 2,5,5-trimethyl-1,2,3,4,4a,5,6,7-octahydro-2-naphthalenol,
- an aldehyde perfuming raw material family selected from the group consisting of decanal, 2-nonenal, 5-ethyl-2-methylnonanal, 3-phenylpropanal, 7-hydroxy-3,7-dimethyloctanal, 2-decenal, 4-hexenal, 8-nonenal, 2-pentenal, 2,4-nonadienal, 2,6-dimethyl-5-heptenal, 2-octenal and 2,6-nonadienal,
- an amine perfuming raw material family selected from the group consisting of methyl 2-aminobenzoate, ethyl 2-aminobenzoate, 3-methylbutylamine, 1-(2-aminophenyl)-1-ethanone and 3-methylindole,
- a ketone perfuming raw material family selected from the group consisting of 2-hexylidene-1-cyclopentanone, 3-methyl-2-(pentyloxy)-2-cyclopenten-1-one, 1-(2,6,6-trimethyl-1,3-cyclohexadien-1-yl)-2-buten-1-one, octalactone, preferably 6-propyloxan-2-one and 5-butyloxolan-2-one, nonalactone, preferably 6-butyloxan-2-one and, decalactone, preferably and 5-hexyloxolan-2-one, undecalactone, preferably 6-hexyloxan-2-one and 5-heptyloxolan-2-one, dodecalactone, preferably 6-heptyloxan-2-one and e, 1-(2,6,6-trimethyl-1-cyclohexen-1-yl)-2-buten-1-one, 1-(2,6,6-trimethyl-2-cyclohexen-1-yl)-2-buten-1-one, methyl {3-oxo-2-[(2Z)-2-penten-1-yl]cyclopentyl}acetate, methyl 2-hexyl-3-oxocyclopentane-1-carboxylate, 6-methyl-5-hepten-2-one, 3-methyl-2-[2-penten-1-yl]-2-cyclopenten-1-one, 1-(5,5-dimethyl-1-cyclohexen-1-yl)-4-penten-1-one, 2-hydroxy-3-methyl-2-cyclopenten-1-one, 4-(2-methyl-2-propanyl)cyclohexanone, 5-methyltricyclo[6.2.1.0^{2,7}]undecan-4-one, 2,2,6-trimethylcyclohexanone and methyl (1R)-cis-3-oxo-2-pentyl-1-cyclopentaneacetate,
- an ester perfuming raw material family selected from the group consisting of allyl 3-phenyl-2-propenoate, 3-phenyl-2-propenyl 3-phenyl-2-propenoate, 1,5-dimethyl-1-vinyl-4-hexenyl 3-phenylpropenoate, ethyl 3-phenyl-2-propenoate, isobutyl 3-phenyl-2-propenoate, 2-phenylethyl 2-butenoate, 2-phenylethyl 3-methyl-2-butenoate, methyl 3-hexenoate and 2-phenylethyl 2-methyl-2-butenoate,
- an ether, epoxide and acetal perfuming raw material family selected from the group consisting of trimethyl-13-oxabicyclo[10.1.0]trideca-4,8-diene, 1,3,3-trimethyl-2-oxabicyclo[2.2.2]octane and 3,4'-dimethylspiro[oxirane-2,9'-tricyclo[6.2.1.0^{2,7}]undec[4]ene,
- a lactone perfuming raw material family selected from the group consisting of 4-nonanolide, 3-propylidene-2-benzofuran-1(3h)-one, 3-butylidene-1-benzo[C]furanone, 6-pentyltetrahydro-2H-pyran-2-one, 6-propyltetrahydro-2H-pyran-2-one, 2-chromenone, 8-oxatricyclo[5.3.1.0(2,6)]undecan-9-one, 6-pentyloxan-2-one, ethylidene-3-oxatricyclo[6.2.1.0^{2,7}]undecan-4-one, 8-decen-5-olide, 5-nonanolide, 5-butyloxolan-2-one, 2-chromanone, 1-oxaspiro[4.5]decan-2-one and 6-hexyltetrahydro-2H-pyran-2-one,
- a N-hetero aromatic perfuming raw material family selected from the group consisting of 1-(2-pyridyl)-1-ethanone, 3-(1-butenyl)pyridine, 1-(3-pyridyl)-1-ethanone, 3-ethylpyridine, butylquinoline, 2-isobutylquinoline, 2-ethyl-3-methylpyrazine, 4-(4,8-dimethyl-3,7-nonadien-1-yl)pyridine, 5-methylquinoxaline and 6-methylquinoline,
- a complex mixture perfuming raw material family selected from the group consisting of artemisia oil, calamus oil, chamomile oil, lemongrass oil, magnolia oil, tansy oil, Pennyroyal oil, patchouli oil and birch oil and
- mixtures thereof,
preferably wherein the at least one arthropod control co-ingredient is selected from the group consisting of
- an acid perfuming raw material family member selected from the group consisting of octanoic acid,
- an alkene perfuming raw material family member selected from the group consisting of 1-isopropyl-4-methyl-1,3-cyclohexadiene, 4-isopropenyl-1-methylcyclohexene, 4-isopropylidene-1-methylcyclohexene and beta-ocimene,
- an alcohol and phenol perfuming raw material family member is selected from the group consisting of 2,5,5-trimethyl-1,2,3,4,4a,5,6,7-octahydro-2-naphthalenol and 4-(trimethyl-1-cyclohexen-1-yl)-2-butanone,
- an aldehyde perfuming raw material family member selected from the group consisting of decanal, 7-hydroxy-3,7-dimethyloctanal, 2-decenal, 2,6-dimethyl-5-heptenal and 2,6-nonadienal,
- an amine perfuming raw material family member selected from the group consisting of methyl 2-aminobenzoate,
- a ketone perfuming raw material family member selected from the group consisting of 1-(2,6,6-trimethyl-1,3-cyclohexadien-1-yl)-2-buten-1-one, 1-(2,6,6-trimethyl-2-cyclohexen-1-yl)-2-buten-1-one, (2E)-1-(2,6,6-trimethyl-1-cyclohexen-1-yl)-2-buten-1-one, methyl 2-hexyl-3-oxocyclopentane-1-carboxylate, 1-(5,5-dimethyl-1-cyclohexen-1-yl)-4-penten-1-one 5-methyltricyclo[6.2.1.0^{2,7}]undecan-4-one and methyl (1R)-cis-3-oxo-2-pentyl-1-cyclopentaneacetate,
- an ether, expoxide and acetal perfuming raw material family member selected from the group consisting of trimethyl-13-oxabicyclo[10.1.0]trideca-4,8-diene, 1,3,3-trimethyl-2-oxabicyclo[2.2.2]octane and 3,4'-dimethylspiro[oxirane-2,9'-tricyclo[6.2.1.0^{2,7}]undec[4]ene,
- a lactone perfuming raw material family member selected from the group consisting of 4-nonanolide, 6-pentyltetrahydro-2h-pyran-2-one, 2-chromenone, 6-pentyloxan-2-one, ethylidene-3-oxatricyclo[6.2.1.0^{2,7}]undecan-4-one and 5-butyloxolan-2-one,
- a N-hetero aromatic perfuming raw material family member selected from the group consisting of isobutylquinoline,
- a complex mixture perfuming raw material family member selected from the group consisting of lemongrass oil and patchouli oil and
- mixtures thereof.

9. The nontherapeutic use of the composition according to claim 8, wherein
- the acid perfuming raw material family member, when present in the composition, is in an amount of 0.002 to 0.007 wt.%, more preferably 0.0025 to 0.0065 wt.%,
- the alkene perfuming raw material family member, when present in the composition, is in an amount of 0.2 to 16 wt.%, preferably 1 to 14 wt.%, more preferably 3 to 13 wt.%,
- the alcohol or phenol perfuming raw material family member, when present in the composition, is in an amount of 0.15 to 4 wt.%, preferably 0.2 to 3.5 wt.%, more preferably 0.4 to 3 wt.%
- the aldehyde perfuming raw material family member, when present in the composition, is present in an amount of 0.001 to 30 wt.%, preferably 0.005 to 25 wt.%, more preferably 0.01 to 20 wt.%,
- the amine perfuming raw material family member, when present in the composition, is in an amount of 0.01 to 3 wt.%, preferably 0.05 to 2 wt.%, more preferably 0.1 to 0.8 wt.%,
- the ketone perfuming raw material family member, when present in the composition, is in an amount of 0.001 to 15 wt.%, preferably 0.01 to 10 wt.%, more preferably 0.1 to 5.5 wt.%,
- the ether, epoxide and acetal perfuming raw material family member, when present in the composition, is in an amount of 0.002 to 20 wt.%, preferably 0.02 to 15 wt.%, more preferably 0.2 to 12.5 wt.%,
- the lactone perfuming raw material family member, when present in the composition, is in an amount of 0.002 to 65 wt.%, preferably 0.02 to 50 wt.%, more preferably 0.2 to 38.8 wt.%,
- the N-hetero aromatic perfuming raw material family member, when present in the composition, is in an amount of 0.002 to 5 wt.%, preferably 0.02 to 2 wt.%, more preferably 0.2 to 0.4 wt.%, and
- the complex mixture perfuming raw material family member, when present in the composition, is in an amount of 0.002 to 10 wt.%, preferably 0.02 to 7.5 wt.%, more preferably 0.2 to 4.8 wt.%,
based on the total weight of the composition.

10. The nontherapeutic use of the composition according to any one of claims 1 to 9, wherein the compound of formula (I) is 2,3-dihydro-1H-indene-2-carbonitrile, 2,4-dimethyl-4,4a,5,9b-tetrahydroindeno[1,2-d][1,3]dioxine, 2,4-dimethyl-4,4a,5,9b-tetrahydroindeno[1,2-d][1,3]dioxine, (2,5-dimethyl-2,3-dihydro-1H-inden-2yl)methanol, (4aRS,9bRS)-4a,7-dimethyl-4,4a,5,9b-tetrahydroindeno[1,2-d][1,3]dioxine, (2RS,4aRS,9bRS)-2,4a,7-trimethyl-4,4a,5,9b-tetrahydroindeno[1,2-d][1,3]dioxine, (2RS,4aSR,9bSR)-2,4a,7-trimethyl-4,4a,5,9b-tetrahydroindeno[1,2-d][1,3]dioxine, (2-methyl-1,3-dihydroinden-2-yl)methanol, 2,3-dihydro-1H-inden-2-ylmethanol, (2RS,4RS,4ASR,9BRS)-2,4-dimethyl-4,4a,5,9b-tetrahydroindeno[1,2-d][1,3]dioxine, (2RS,4aRS,9bSR)-2-methyl-4,4a,5,9b-tetrahydroindeno[1,2-d][1,3]dioxin, (2RS,4aSR,9bRS)-2-methyl-4,4a,5,9b-tetrahydroindeno[1,2-d][1,3]dioxin, (4aRS,9bSR)-2,2-dimethyl-4,4a,5,9b-tetrahydroindeno[1,2-d][1,3]dioxin, (2,4,6-trimethyl-2,3-dihydro-1H-inden-2-yl)methanol, 5-(tert-butyl)-1,3-dihydrospiro[indene-2,3'-oxetane], (2RS,4RS,4ARS,9BSR)-2,4-dimethyl-4,4a,5,9b-tetrahydroindeno[1,2-d][1,3]dioxine, 4A,8-dimethyl-indano[1,2-d]-1,3-dioxan-2-one, 1-(2,5-dimethyl-2,3-dihydro-1H-inden-2-yl)ethanone, (5-methyl-2,3-dihydro-1H-inden-2-yl)methanol, (4aRS,9bSR)-8-methyl-4,4a,5,9b-tetrahydroindeno[1,2-d][1,3]dioxin, (2RS,4aRS,9bSR)-2-ethyl-4,4a,5,9b-tetrahydroindeno[1,2-d][1,3]dioxine, (2RS,4aSR,9bRS)-2-ethyl-4,4a,5,9b-tetrahydroindeno[1,2-d][1,3]dioxine, (2R,4aS,9bS)-2,4a,8-trimethyl-4,4a,5,9b-tetrahydroindeno[1,2-d][1,3]dioxin, 1-(2,5-dimethyl-2-indanyl)-1-ethanol, (2,5-dimethyl-2,3-dihydro-1H-inden-2yl)methanol, (2RS,4aRS,9bSR)-2,8-dimethyl-4,4a,5,9b-tetrahydroindeno[1,2-d][1,3]dioxine (2RS,4aSR,9bRS)-2,8-dimethyl-4,4a,5,9b-tetrahydroindeno[1,2-d][1,3]dioxine, (2RS,4aSR,9bSR)-2,4a-dimethyl-4,4a,5,9b-tetrahydroindeno[1,2-d][1,3]dioxin, (4aRS,9bRS)-4a,8-dimethyl-4,4a,5,9b-tetrahydroindeno[1,2-d][1,3]dioxin, 1-(2,4,5-trimethyl-2,3-dihydro-1H-inden-2-yl)ethenone, (2RS,4aRS,9bSR)-2-ethyl-8-methyl-4,4a,5,9b-tetrahydroindeno[1,2-d][1,3]dioxin, (2RS,4aSR,9bRS)-2-ethyl-8-methyl-4,4a,5,9b-tetrahydroindeno[1,2-d][1,3]dioxin, (4aRS,9bRS)-2,2,4a,8-tetramethyl-4,4a,5,9b-tetrahydroindeno[1,2-d][1,3]dioxine, 5-tert-butyl-indan-2-spiro-3'-oxetane, (2,6-dimethyl-1,2,3,4-tetrahydro-2-naphthalenyl)methanol, (4aRS,9bRS)-2,2,4a-trimethyl-4,4a,5,9b-tetrahydroindeno[1,2-d][1,3]dioxine, or (4aRS,9bRS)-2,2,4a,7-tetramethyl-4,4a,5,9b-tetrahydroindeno[1,2-d][1,3]dioxine.

11. Nontherapeutic Method for arthropod, preferably insect, control which comprises bringing an arthropod, preferably insect, into direct contact or in contact with vapors of a composition as defined in any one of claims 1 to 10 or a compound of formula (I) as defined in any one of claims 1 or 10.

12. The compound of formula (I) as defined in claim 1, wherein the compound is selected from 2,3-dihydro-1H-indene-2-carbonitrile, (4aRS,9bRS)-4a,7-dimethyl-4,4a,5,9b-tetrahydroindeno[1,2-d][1,3]dioxine, (2RS,4aRS,9bRS)-2,4a,7-trimethyl-4,4a,5,9b-tetrahydroindeno[1,2-d][1,3]dioxine, (2RS,4aSR,9bSR)-2,4a,7-trimethyl-4,4a,5,9b-tetrahydroindeno[1,2-d][1,3]dioxine, 2,3-dihydro-1H-inden-2-ylmethanol, (2RS,4aRS,9bSR)-2-methyl-4,4a,5,9b-tetrahydroindeno[1,2-d][1,3]dioxin, (2RS,4aSR,9bRS)-2-methyl-4,4a,5,9b-tetrahydroindeno[1,2-d][1,3]dioxin, (4aRS,9bSR)-2,2-dimethyl-4,4a,5,9b-tetrahydroindeno[1,2-d][1,3]dioxin, 5-(tert-butyl)-1,3-dihydrospiro[indene-2,3'-oxetane], 4A,8-dimethyl-indano[1,2-d]-1,3-dioxan-2-one, (4aRS,9bSR)-8-methyl-4,4a,5,9b-tetrahydroindeno[1,2-d][1,3]dioxin, (2RS,4aRS,9bSR)-2-ethyl-4,4a,5,9b-tetrahydroindeno[1,2-d][1,3]dioxine, (2RS,4aSR,9bRS)-2-ethyl-4,4a,5,9b-tetrahydroindeno[1,2-d][1,3]dioxine, and (2R,4aS,9bS)-2,4a,8-trimethyl-4,4a,5,9b-tetrahydroindeno[1,2-d][1,3]dioxin.

## Patentansprüche

1. Nichttherapeutische Verwendung einer Zusammensetzung umfassend:
- eine Verbindung von Formel (I): 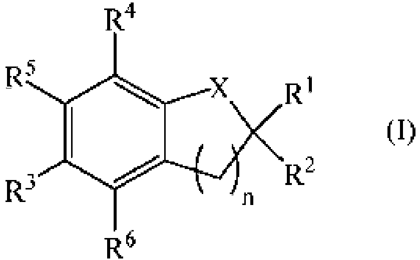
in Form eines beliebigen seiner Stereoisomere oder als Gemisch davon, und wobei
n für 1 oder 2 steht;
X für eine CH₂-, CH(OH)- oder C=N-OH-Gruppe steht;
R¹ für ein CN, CHR⁷OR⁸ oder eine COR⁹-Gruppe steht,
R⁷ ein Wasserstoffatom oder eine Methylgruppe ist,
R⁸ ein Wasserstoffatom, eine C₁₋₃-Kohlenwasserstoffgruppe ist, die optional durch ein Hydroxyl oder eine C₁₋₃-Alkoxygruppe, eine C₃₋₁₀-Trialkylsilylgruppe oder eine COR⁹-Gruppe substituiert ist, und
R⁹ ein Wasserstoffatom, eine Hydroxylgruppe, eine C₁₋₃-Alkoxygruppe, eine C₁₋₈-Kohlenwasserstoffgruppe oder ein Phenyl ist, die jeweils optional durch eine bis drei Gruppen substituiert sind, die aus einer C₁₋₃-Alkyl-, Alkoxy- oder Aminogruppe ausgewählt sind;
R² für ein Wasserstoffatom oder eine Methyl-, Ethyl- oder CH₂OH-Gruppe steht;
R³ für ein Wasserstoffatom oder eine C₁₋₅- Kohlenwasserstoffgruppe oder eine C₁₋₃-Alkoxylgruppe steht; und
R⁴, R⁵ und R⁶ unabhängig voneinander für ein Wasserstoffatom oder eine C₁₋₅-Alkyl- oder C₂₋₅-Alkenylgruppe stehen; oder
R¹ und R² zusammen für eine CH₂-O-C(=O)-O, CH₂-O-CH₂-O- oder CH₂-O-CH₂-Gruppe stehen; oder
X und R¹ zusammen für eine CH-O-C(=O)-O-C(R¹⁰)₂ oder CH-O-C(R¹⁰)₂-O-C(R¹⁰)₂-Gruppe stehen, wobei R¹⁰ unabhängig voneinander für ein Wasserstoffatom oder eine C₁₋₃ Alkylgruppe steht;
- optional mindestens einen Arthropodenbekämpfungs-Zusatzstoff
zur Bekämpfung von Arthropoden.

2. Nichttherapeutische Verwendung der Zusammensetzung nach Anspruch 1, wobei es sich bei dem Arthropoden um ein Insekt, vorzugsweise eine Mücke, handelt.

3. Nichttherapeutische Verwendung der Zusammensetzung nach Anspruch 1, wobei es sich bei dem Arthropoden um ein Spinnentier, vorzugsweise eine Zecke, handelt.

4. Nichttherapeutische Verwendung der Zusammensetzung nach einem der Ansprüche 1 bis 3, wobei die Zusammensetzung die Verbindung der Formel (I) in einer zur Bekämpfung von Arthropoden wirksamen Menge, vorzugsweise in einer Menge von 0,02 bis 80 Gew.-%, bevorzugter in einer Menge von 0,05 bis 70 Gew.-%, noch bevorzugter in einer Menge von 0,1 bis 60 Gew.-%, wie etwa von 0,1 bis 5 Gew.-%, oder insbesondere von 0,15 bis 4 Gew.-% oder von 11 bis 35 Gew.-%, oder insbesondere von 15 bis 30 Gew.-%, basierend auf dem Gesamtgewicht der Zusammensetzung umfasst.

5. Nichttherapeutische Verwendung der Zusammensetzung nach einem der Ansprüche 1 bis 4, die mindestens einen Arthropodenbekämpfungs-Zusatzstoff umfasst, ausgewählt aus der Gruppe bestehend aus N,N-Diethyl-3-methylbenzamid (DEET), Ethylbutylacetylaminopropionat (IR3535); para-Menthan-3,8-diol (PMD); 1-(1-Methylpropoxycarbonyl)-2-(2-hydroxyethyl)piperidin (Icaridin); Zedernholzöl (China), Zedernholzöl (Texas), Zedernholzöl (Virginia), Zimtöl, Citronellaöl, Nelkenöl, Ackerminzeöl, Cymbopogon winterianus-Öl, fraktioniert, hydratisiert, cyclisiert, Decansäure, Eucalyptus citriodora-Öl und Citronellal, hydratisiert, cyclisiert, Eucalyptus citriodora-Öl, hydratisiert, cyclisiert, Eugenol, Knoblauchöl, Geraniol, Geranienöl, Lavendel, Lavandula hybrida ext./Lavandin Öl, Zitronenöl, Lemongrassöl, Nelkenextrakt, Mentha arvensis, Extrakt, Metofluthrin, Gemisch aus cis- und trans-p-Menthan-3,8-diol, N,N-Diethyl-meta-toluamid, Nonansäure, Pfefferminzöl, Pyrethrine und Pyrethroide, Rosmarinöl, Grüne Minzeöl, Thymianöl, Wintergrünöl, 2,3,4,5-Bis(butyl-2-en)tetrahydrofurfural (MGK Repellent 11), Cineol, Zimtaldehyd, Citral, Citronellal, Citronellol, Citronellaöl Java, Cumarin, Dibutylphthalat, Diethylphthalat, Dimethylanthranilat, Dimethylphthalat, Ethylvanillin, Eukalyptusöl, Delta-Octalacton, Delta-Nonalacton, Delta-Decalacton, Delta-Undecalacton Delta-Dodecalacton, Gamma-Octalacton, Gamma-Nonalacton, Gamma-Decalacton, Gamma-Undecalacton, Gamma-Dodecalacton, Hydroxycitronellal, Limettenöl, Limonen, Linalool, Methylanthranilat, Ackerminze, Pfefferminze, Spitzminze, Myrcen, Neemöl, Sabinen, β-Caryophyllen, (1H-Indol-2-yl)essigsäure, Anethol, Anisöl, Basilikumöl, Lorbeeröl, Kampfer, Ethylsalicylat, Nadelbaumöl (Kiefernöl), (1,3,4,5,6,7-Hexahydro-1,3-dioxo-2H-isoindol-2-yl)methyl (1R-trans)-2,2-Dimethyl-3-(2-methylprop-1-enyl)cyclopropancarboxylat (d-Tetramethrin), (RS)-3-Allyl-2-methyl-4-oxocyclopent-2-enyl-(1R,3R;1R,3S)-2,2-dimethyl-3-(2-methylprop-1-enyl)-cyclopropanecarboxylat (Gemisch aus 4 Isomeren 1R trans, 1R: 1R trans, 1S: 1R cis, 1R: 1R cis, 1S4:4:1:1) (d-Allethrin), (RS)-α-Cyano-3-phenoxybenzyl-(1RS)-cis, trans-3-(2,2-Dichlorvinyl)-2,2-dimethylcyclopropancarboxylat (Cypermethrin), 2-Methyl-4-oxo-3-(prop-2-inyl)cyclopent-2-en-1-yl, 2,2-Dimethyl-3-(2-methylprop-1-enyl)cyclopropancarboxylat (PraIIethrin), Acetamiprid, Azadirachtin, Bendiocarb, Bifenthrin, Borsäure, Chlorpyrifos, Deltamethrin, Diazinon, Dichlorvos, Eugenol, Fipronil, Imidacloprid, Linalool, Malathion, Maltodextrin, Neemextrakt, Metofluthrin, Nikotin, Permethrin, Pyrethrine und Pyrethroide, Rotenon, Siliciumdioxid (Siliciumdioxid/Kieselguhr), S-Methopren, Spinosad (Spinosyn A), Spinosyn D, Tetramethrin, Transfluthrin.

6. Nichttherapeutische Verwendung der Zusammensetzung nach einem der Ansprüche 1 bis 5, die N,N-Diethyl-3-methylbenzamid (DEET), Ethylbutylacetylaminopropionat (IR3535), para-Menthan-3,8-diol (PMD) oder 1-(1-Methylpropoxycarbonyl)-2-(2-hydroxyethyl)piperidin (Icaridin) umfasst, vorzugsweise in einer Menge von 0,02 bis 80 Gew.-%, bevorzugter in einer Menge von 0,05 bis 70 Gew.-%, noch bevorzugter in einer Menge von 0,1 bis 60 Gew.-%, basierend auf dem Gesamtgewicht der Zusammensetzung umfasst.

7. Nichttherapeutische Verwendung der Zusammensetzung nach einem der Ansprüche 1 bis 6, die mindestens einen Arthropodenbekämpfungs-Zusatzstoff umfasst, der sowohl zur Arthropodenbekämpfungsaktivität als auch zum olfaktorischen Charakter der Zusammensetzung beiträgt.

8. Nichttherapeutische Verwendung der Zusammensetzung nach Anspruch 7, wobei der mindestens eine Arthropodenbekämpfungs-Zusatzstoff ausgewählt ist aus der Gruppe bestehend aus
- einer Familie von sauren Duftrohstoffen, ausgewählt aus der Gruppe bestehend aus Pentansäure bis Dodecansäure, vorzugsweise Hexansäure, Heptansäure, Octansäure und Nonansäure,
- einer Familie von Alken-Duftrohstoffen, ausgewählt aus der Gruppe bestehend aus 1-Isopropyl-4-methyl-1,3-cyclohexadien, 4-Isopropenyl-1-methylcyclohexen, 4-Isopropyliden-1-methylcyclohexen und beta-Ocimen,
- einer Familie von Alkohol- und Phenol-Duftrohstoffe, ausgewählt aus der Gruppe bestehend aus 1-Decanol, 2-Methyl-4-phenyl-2-butanol, 8-p-Menthen-2-ol, 3,4-Dimethylphenol, 3,7-Dimethyl-2,6-octadien-1-ol, 2-Isopropylphenol, 2-Methoxy-4-vinylphenol, 1,8-p-Menthadien-7-ol, 4-Ethylphenol, 4-Methylphenol, 4-(Trimethyl-1-cyclohexen-1-yl)-2-butanon und 2,5,5-Trimethyl-1,2,3,4,4a,5,6,7-octahydro-2-naphthalenol,
- einer Familie von Aldehyd-Duftrohstoffen, ausgewählt aus der Gruppe bestehend aus Decanal, 2-Nonenal, 5-Ethyl-2-methylnonanal, 3-Phenylpropanal, 7-Hydroxy-3,7-dimethyloctanal, 2-Decenal, 4-Hexenal, 8-Nonenal, 2-Pentenal, 2,4-Nonadienal, 2,6-Dimethyl-5-heptenal, 2-Octenal und 2,6-Nonadienal,
- einer Familie von Amin-Duftrohstoffen, ausgewählt aus der Gruppe bestehend aus Methyl-2-aminobenzoat, Ethyl-2-aminobenzoat, 3-Methylbutylamin, 1-(2-Aminophenyl)-1-ethanon und 3-Methylindol,
- einer Familie von Keton-Duftrohstoffen, ausgewählt aus der Gruppe bestehend aus 2-Hexyliden-1-cyclopentanon, 3-Methyl-2-(pentyloxy)-2-cyclopenten-1-on, 1-(2,6,6-Trimethyl-1,3-cyclohexadien-1-yl)-2-buten-1-on, Octalacton, vorzugsweise 6-Propyloxan-2-on und 5-Butyloxolan-2-on, Nonalacton, vorzugsweise 6-Butyloxan-2-on und, Decalacton, vorzugsweise und 5-Hexyloxolan-2-on, Undecalacton, vorzugsweise 6-Hexyloxan-2-on und 5-Heptyloxolan-2-on, Dodecalacton, vorzugsweise 6-Heptyloxan-2-on und e, 1-(2,6,6-Trimethyl-1-cyclohexen-1-yl)-2-buten-1-on, 1-(2,6,6-Trimethyl-2-cyclohexen-1-yl)-2-buten-1-on, Methyl {3-oxo-2-[(2Z)-2-penten-1-yl]cyclopentyl}acetat, Methyl-2-hexyl-3-oxocyclopentan-1-carboxylat, 6-Methyl-5-hepten-2-on, 3-Methyl-2-[2-penten-1-yl]-2-cyclopenten-1-on, 1-(5,5-Dimethyl-1-cyclohexen-1-yl)-4-penten-1-on, 2-Hydroxy-3-methyl-2-cyclopenten-1-on, 4-(2-Methyl-2-propanyl)cyclohexanon, 5-Methyltricyclo[6.2.1.0^{2,7}]undecan-4-on, 2,2,6-Trimethylcyclohexanon und Methyl-(1R)-cis-3-oxo-2-pentyl-1-cyclopentanacetat,
- einer Familie von Ester-Duftrohstoffen, ausgewählt aus der Gruppe bestehend aus Allyl-3-phenyl-2-propenoat, 3-Phenyl-2-propenyl-3-phenyl-2-propenoat, 1,5-Dimethyl-1-vinyl-4-hexenyl-3-phenylpropenoat, Ethyl-3-phenyl-2-propenoat, Isobutyl-3-phenyl-2-propenoat, 2-Phenylethyl-2-butenoat, 2-Phenylethyl-3-methyl-2-butenoat, Methyl-3-hexenoat und 2-Phenylethyl-2-methyl-2-butenoat,
- einer Familie von Ether-, Epoxid- und Acetal-Duftrohstoffen, ausgewählt aus der Gruppe bestehend aus Trimethyl-13-oxabicyclo[10.1.0]trideca-4,8-dien, 1,3,3-Trimethyl-2-oxabicyclo[2.2.2]octan und 3,4'-Dimethylspiro[oxiran-2,9'-tricyclo[6.2.1.0^{2,7}]undec[4]en,
- einer Familie von Lacton-Duftrohstoffen, ausgewählt aus der Gruppe bestehend aus 4-Nonanolid, 3-Propyliden-2-benzofuran-1(3h)-on, 3-Butyliden-1-benzo[C]furanone, 6-Pentyltetrahydro-2H-pyran-2-on, 6-Propyltetrahydro-2H-pyran-2-on, 2-Chromenon, 8-Oxatricyclo[5.3.1.0(2,6)]undecan-9-on, 6-Pentyloxan-2-on, Ethyliden-3-oxatricyclo[6.2.1.0^{2,7}]undecan-4-on, 8-Decen-5-olid, 5-Nonanolid, 5-Butyloxolan-2-on, 2-Chromanon, 1-Oxaspiro[4.5]decan-2-on und 6-Hexyltetrahydro-2H-pyran-2-on,
- einer Familie von N-heteroaromatischen Duftrohstoffen, ausgewählt aus der Gruppe bestehend aus 1-(2-Pyridyl)-1-ethanon, 3-(1-Butenyl)pyridin, 1-(3-Pyridyl)-1-ethanon, 3-Ethylpyridin, Butylchinolin, 2-Isobutylchinolin, 2-Ethyl-3-methylpyrazin, 4-(4,8-Dimethyl-3,7-nonadien-1-yl)pyridin, 5-Methylchinoxalin und 6-Methylchinolin,
- einem komplexen Gemisch der Familie von Duftrohstoffen, ausgewählt aus der Gruppe bestehend aus Beifußöl, Kalmusöl, Kamillenöl, Zitronengrasöl, Magnolienöl, Rainfarnöl, Poleiminzeöl, Patschuliöl und Birkenöl und
- sowie Gemische davon,
vorzugsweise wobei der mindestens eine Arthropodenbekämpfungs-Zusatzstoff aus der Gruppe ausgewählt ist bestehend aus:
- einem Mitglied der Familie der sauren Duftrohstoffe, ausgewählt aus der Gruppe bestehend aus Octansäure,
- einem Mitglied der Familie der Alken-Duftrohstoffe, ausgewählt aus der Gruppe bestehend aus 1-Isopropyl-4-methyl-1,3-cyclohexadien, 4-Isopropenyl-1-methylcyclohexen, 4-Isopropyliden-1-methylcyclohexen und beta-Ocimen,
- einem Mitglied der Familie der Alkohol- und Phenol-Duftrohstoffe, ausgewählt aus der Gruppe bestehend aus 2,5,5-Trimethyl-1,2,3,4,4a,5,6,7-octahydro-2-naphthalenol und 4-(Trimethyl-1-cyclohexen-1-yl)-2-butanon,
- einem Mitglied der Familie der Aldehyd-Duftrohstoffe, ausgewählt aus der Gruppe bestehend aus Decanal, 7-Hydroxy-3,7-dimethyloctanal, 2-Decenal, 2,6-Dimethyl-5-heptenal und 2,6-Nonadienal,
- einem Mitglied der Familie der Amin-Duftrohstoffe, ausgewählt aus der Gruppe bestehend aus Methyl-2-aminobenzoat,
- einem Mitglied der Familie der Keton-Duftrohstoffe, ausgewählt aus der Gruppe bestehend aus 1-(2,6,6-Trimethyl-1,3-cyclohexadien-1-yl)-2-buten-1-on, 1-(2,6,6-Trimethyl-2-cyclohexen-1-yl)-2-buten-1-on, (2E)-1-(2,6,6-Trimethyl-1-cyclohexen-1-yl)-2-buten-1-on, Methyl-2-hexyl-3-oxocyclopentan-1-carboxylat, 1-(5,5-Dimethyl-1-cyclohexen-1-yl)-4-penten-1-on 5-methyltricyclo[6.2.1.0^{2,7}]undecan-4-on und Methyl-(1R)-cis-3-oxo-2-pentyl-1-cyclopentanacetat,
- einem Mitglied der Familie der Ether-, Epoxid- und Acetal-Duftrohstoffe, ausgewählt aus der Gruppe bestehend aus Trimethyl-13-oxabicyclo[10.1.0]trideca-4,8-dien, 1,3,3-Trimethyl-2-oxabicyclo[2.2.2]octan und 3,4'-Dimethylspiro[oxiran-2,9'- tricyclo[6.2.1.0^{2,7}]undec[4]en,
- einem Mitglied der Familie der Lacton-Duftrohstoffe, ausgewählt aus der Gruppe bestehend aus 4-Nonanolid, 6-Pentyltetrahydro-2H-pyran-2-on, 2-Chromenon, 6-Pentyloxan-2-on, Ethyliden-3-oxatricyclo[6.2.1.0^{2,7}]undecan-4-on und 5-Butyloxolan-2-on,
- einem Mitglied der Familie der N-heteroaromatischen Duftrohstoffe, ausgewählt aus der Gruppe bestehend aus Isobutylchinolin,
- einem komplexen Gemisch der Mitglieder der Familie von Duftrohstoffen, ausgewählt aus der Gruppe bestehend aus Zitronengrasöl und Patschuliöl und
- sowie Gemische davon.

9. Nichttherapeutische Verwendung der Zusammensetzung nach Anspruch 8, wobei
- das Mitglied der Familie der sauren Duftrohstoffe, sofern es in der Zusammensetzung vorhanden ist, in einer Menge von 0,002 bis 0,007 Gew.-%, bevorzugter 0,0025 bis 0,0065 Gew.-% vorliegt,
- das Mitglied der Familie der Alken-Duftrohstoffe, sofern es in der Zusammensetzung vorhanden ist, in einer Menge von 0,2 bis 16, Gew.-%, vorzugsweise 1 bis 14 Gew.-%, bevorzugter 3 bis 13 Gew.-% vorliegt,
- das Mitglied der Familie der Alkohol- oder Phenol-Duftrohstoffe, sofern es in der Zusammensetzung vorhanden ist, in einer Menge von 0,15 bis 4 Gew.-%, vorzugsweise 0,2 bis 3,5 Gew.-%, bevorzugter 0,4 bis 3 Gew.-% vorliegt,
- das Mitglied der Familie der Aldehyd-Duftrohstoffe, sofern es in der Zusammensetzung vorhanden ist, in einer Menge von 0,001 bis 30 Gew.-%, vorzugsweise 0,005 bis 25 Gew.-%, bevorzugter 0,01 bis 20 Gew.-% vorliegt,
- das Mitglied der Familie der Amin-Duftrohstoffe, sofern es in der Zusammensetzung vorhanden ist, in einer Menge von 0,01 bis 3 Gew.-%, vorzugsweise 0,05 bis 2 Gew.-%, bevorzugter 0,1 bis 0,8 Gew.-% vorliegt,
- das Mitglied der Familie der Keton-Duftrohstoffe, sofern es in der Zusammensetzung vorhanden ist, in einer Menge von 0,001 bis 15 Gew.-%, vorzugsweise 0,01 bis 10 Gew.-%, bevorzugter 0,1 bis 5,5 Gew.-% vorliegt,
- das Mitglied der Familie der Ether-, Epoxid- und Acetal-Duftrohstoffe, sofern es in der Zusammensetzung vorhanden ist, in einer Menge von 0,002 bis 20 Gew.-%, vorzugsweise 0,02 bis 15 Gew.-%, bevorzugter 0,2 bis 12,5 Gew.-% vorliegt,
- das Mitglied der Familie der Lacton-Duftrohstoffe, sofern es in der Zusammensetzung vorhanden ist, in einer Menge von 0,002 bis 65 Gew.-%, vorzugsweise 0,02 bis 50 Gew.-%, bevorzugter 0,2 bis 38,8 Gew.-% vorliegt,
- das Mitglied der Familie der N-heteroaromatischen Duftrohstoffe, sofern es in der Zusammensetzung vorhanden ist, in einer Menge von 0,002 bis 5 Gew.-%, vorzugsweise 0,02 bis 2 Gew.-%, bevorzugter 0,2 bis 0,4 Gew.-% vorliegt, und
- das komplexe Gemisch der Mitglieder der Familie von Duftrohstoffen, sofern es in der Zusammensetzung vorhanden ist, in einer Menge von 0,002 bis 10 Gew.-%, vorzugsweise 0,02 bis 7,5 Gew.-%, bevorzugter 0,2 bis 4,8 Gew.-% vorliegt,
basierend auf dem Gesamtgewicht der Zusammensetzung.

10. Nichttherapeutische Verwendung der Zusammensetzung nach einem der Ansprüche 1 bis 9, wobei es sich bei der Verbindung der Formel (I) um Folgendes handelt: 2,3-Dihydro-1H-inden-2-carbonitril, 2,4-Dimethyl-4,4a,5,9b-tetrahydroindeno[1,2-d][1,3]dioxin, 2,4-Dimethyl-4,4a,5,9b-tetrahydroindeno[1,2-d][1,3]dioxin, (2,5-Dimethyl-2,3-dihydro-1H-inden-2-yl)methanol, (4aRS,9bRS)-4a,7-Dimethyl-4,4a,5,9b-tetrahydroindeno[1,2-d][1,3]dioxin, (2RS,4aRS,9bRS)-2,4a,7-Trimethyl-4,4a,5,9b-tetrahydroindeno[1,2-d][1,3]dioxin, (2RS,4aSR,9bSR)-2,4a,7-Trimethyl-4,4a,5,9b-tetrahydroindeno[1,2-d][1,3]dioxin, (2-Methyl-1,3-dihydroinden-2-yl)methanol, 2,3-Dihydro-1H-inden-2-ylmethanol, (2RS,4RS,4ASR,9BRS)-2,4-Dimethyl-4,4a,5,9b-tetrahydroindeno[1,2-d][1,3]dioxin, (2RS,4aRS,9bSR)-2-Methyl-4,4a,5,9b-tetrahydroindeno[1,2-d][1,3]dioxin, (2RS,4aSR,9bRS)-2-Methyl-4,4a,5,9b-tetrahydroindeno[1,2-d][1,3]dioxin, (4aRS,9bSR)-2,2-Dimethyl-4,4a,5,9b-tetrahydroindeno[1,2-d][1,3]dioxin, (2,4,6-Trimethyl-2,3-dihydro-1H-inden-2-yl)methanol, 5-(tert-Butyl)-1,3-dihydrospiro[inden-2,3'-oxetan], (2RS,4RS,4ARS,9BSR)-2,4-Dimethyl-4,4a,5,9b-tetrahydroindeno[1,2-d][1,3]dioxin, 4A,8-Dimethyl-indano[1,2-d]-1,3-dioxan-2-on, 1-(2,5-Dimethyl-2,3-dihydro-1H-inden-2-yl)ethanon, (5-Methyl-2,3-dihydro-1H-inden-2-yl)methanol, (4aRS,9bSR)-8-Methyl-4,4a,5,9b-tetrahydroindeno[1,2-d][1,3]dioxin, (2RS,4aRS,9bSR)-2-Ethyl-4,4a,5,9b-tetrahydroindeno[1,2-d][1,3]dioxin, (2RS,4aSR,9bRS)-2-Ethyl-4,4a,5,9b-tetrahydroindeno[1,2-d][1,3]dioxin, (2R,4aS,9bS)-2,4a,8-Trimethyl-4,4a,5,9b-tetrahydroindeno[1,2-d][1,3]dioxin, 1-(2,5-Dimethyl-2-indanyl)-1-ethanol, (2,5-Dimethyl-2,3-dihydro-1H-inden-2-yl)methanol, (2RS,4aRS,9bSR)-2,8-Dimethyl-4,4a,5,9b-tetrahydroindeno[1,2-d][1,3]dioxin (2RS,4aSR,9bRS)-2,8-dimethyl-4,4a,5,9b-tetrahydroindeno[1,2-d][1,3]dioxin, (2RS,4aSR,9bSR)-2,4a-Dimethyl-4,4a,5,9b-tetrahydroindeno[1,2-d][1,3]dioxin, (4aRS,9bRS)-4a,8-Dimethyl-4,4a,5,9b-tetrahydroindeno[1,2-d][1,3]dioxin, 1-(2,4,5-Trimethyl-2,3-dihydro-1H-inden-2-yl)ethenon, (2RS,4aRS,9bSR)-2-Ethyl-8-methyl-4,4a,5,9b-tetrahydroindeno[1,2-d][1,3]dioxin, (2RS,4aSR,9bRS)-2-Ethyl-8-methyl-4,4a,5,9b-tetrahydroindeno[1,2-d][1,3]dioxin, (4aRS,9bRS)-2,2,4a,8-Tetramethyl-4,4a,5,9b-tetrahydroindeno[1,2-d][1,3]dioxin, 5-tert-Butyl-indan-2-spiro-3'-oxetan, (2,6-Dimethyl-1,2,3,4-tetrahydro-2-naphthalenyl)methanol, (4aRS,9bRS)-2,2,4a-Trimethyl-4,4a,5,9b-tetrahydroindeno[1,2-d][1,3]dioxin oder (4aRS,9bRS)-2,2,4a,7-Tetramethyl-4,4a,5,9b-tetrahydroindeno[1,2-d][1,3]dioxin.

11. Nichttherapeutisches Verfahren zur Bekämpfung von Arthropoden, vorzugsweise Insekten, welches darin besteht, einen Arthropoden, vorzugsweise ein Insekt, in direkten Kontakt oder in Kontakt mit Dämpfen einer Zusammensetzung nach einem der Ansprüche 1 bis 10 oder einer Verbindung der Formel (I) nach einem der Ansprüche 1 oder 10 zu bringen.

12. Verbindung der Formel (I) nach Anspruch 1, wobei die Verbindung ausgewählt ist aus 2,3-Dihydro-1H-inden-2-carbonitril, (4aRS,9bRS)-4a,7-Dimethyl-4,4a,5,9b-tetrahydroindeno[1,2-d][1,3]dioxin, (2RS,4aRS,9bRS)-2,4a,7-Trimethyl-4,4a,5,9b-tetrahydroindeno[1,2-d][1,3]dioxin, (2RS,4aSR,9bSR)-2,4a,7-Trimethyl-4,4a,5,9b-tetrahydroindeno[1,2-d][1,3]dioxin, 2,3-Dihydro-1H-inden-2-ylmethanol, (2RS,4aRS,9bSR)-2-Methyl-4,4a,5,9b-tetrahydroindeno[1,2-d][1,3]dioxin, (2RS,4aSR,9bRS)-2-Methyl-4,4a,5,9b-tetrahydroindeno[1,2-d][1,3]dioxin, (4aRS,9bSR)-2,2-Dimethyl-4,4a,5,9b-tetrahydroindeno[1,2-d][1,3]dioxin, 5-(tert-Butyl)-1,3-dihydrospiro[inden-2,3'-oxetan], 4A,8-Dimethyl-indano[1,2-d]-1,3-dioxan-2-on, (4aRS,9bSR)-8-Methyl-4,4a,5,9b-tetrahydroindeno[1,2-d][1,3]dioxin, (2RS,4aRS,9bSR)-2-Ethyl-4,4a,5,9b-Tetrahydroindeno[1,2-d][1,3]dioxin, (2RS,4aSR,9bRS)-2-Ethyl-4,4a,5,9b-tetrahydroindeno[1,2-d][1,3]dioxin und (2R,4aS,9bS)-2,4a,8-Trimethyl-4,4a,5,9b-tetrahydroindeno[1,2-d][1,3]dioxin.

## Revendications

1. Utilisation non thérapeutique d'une composition comprenant
- un composé de Formule (I) : 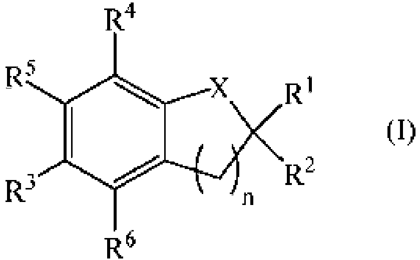
sous la forme de l'un quelconque de ses stéréoisomères ou d'un mélange de ceux-ci, et dans laquelle
n représente 1 ou 2 ;
X représente un groupe CH₂, CH(OH) ou C=N-OH ;
R¹ représente un groupe CN, CHR⁷OR⁸ ou un groupe COR⁹,
R⁷ étant un atome d'hydrogène ou un groupe méthyle,
R⁸ étant un atome d'hydrogène, un groupe hydrocarboné en C₁₋₃, facultativement substitué par un hydroxyle ou un groupe alcoxy en C₁₋₃, un groupe trialkylsilyle en C₃₋₁₀ ou un groupe COR⁹, et
R⁹ étant un atome d'hydrogène, un groupe hydroxyle, un groupe alcoxy en C₁₋₃, un groupe hydrocarboné en C₁₋₈ ou un phényle, chacun facultativement substitué par un à trois groupes choisis parmi un groupe alkyle en C₁₋₃, alcoxy ou amine ;
R² représente un atome d'hydrogène ou un groupe méthyle, éthyle ou CH₂OH ;
R³ représente un atome d'hydrogène ou un groupe hydrocarboné en C₁₋₅ ou un groupe alcoxyle en C₁₋₃ ; et
R⁴, R⁵ et R⁶ représentent, indépendamment les uns des autres, un atome d'hydrogène ou un groupe alkyle en C₁₋₅ ou un groupe alcényle en C₂₋₅ ; ou
R¹ etR², pris ensemble, représentent un groupe CH₂-O-C(=O)-O, CH₂-O-CH₂-O ou CH₂-O-CH₂ ; ou
X et R¹, pris ensemble, représentent un groupe CH-O-C(=O)-O-C(R¹⁰)₂ ou CH-OC(R¹⁰)₂-OC(R¹⁰)₂, dans laquelle R¹⁰, indépendamment l'un de l'autre, représente un atome d'hydrogène ou un groupe alkyle en C₁₋₃ ;
- facultativement, au moins un co-ingrédient de lutte contre les arthropodes,
pour la lutte contre les arthropodes.

2. Utilisation non thérapeutique de la composition selon la revendication 1, dans laquelle l'arthropode est un insecte, de préférence un moustique.

3. Utilisation non thérapeutique de la composition selon la revendication 1, dans laquelle l'arthropode est un arachnide, de préférence une tique.

4. Utilisation non thérapeutique de la composition selon l'une quelconque des revendications 1 à 3, dans laquelle la composition comprend le composé de formule (I) en une quantité efficace pour la lutte contre les arthropodes, de préférence en une quantité comprise entre 0,02 et 80 % en poids, plus préférentiellement en une quantité comprise entre 0,05 et 70 % en poids, encore plus préférentiellement en une quantité comprise entre 0,1 et 60 % en poids, par exemple de 0,1 à 5 % en poids, ou plus particulièrement de 0,15 à 4 % en poids, ou de 11 à 35 % en poids, ou plus particulièrement de 15 à 30 % en poids, sur la base du poids total de la composition.

5. Utilisation non thérapeutique de la composition selon l'une quelconque des revendications 1 à 4, comprenant au moins un co-ingrédient de lutte contre les arthropodes choisi dans le groupe constitué de N,N-diéthyl-3-méthylbenzamide (DEET), de butylacétylaminopropionate d'éthyle (IR3535), de para-menthane-3,8-diol (PMD) ; de 1-(1-méthylpropoxycarbonyl)-2-(2-hydroxyéthyl)pipéridine (icaridine) ; d'huile de cèdre (Chine), d'huile de cèdre (Texas), d'huile de cèdre (Virginie), d'huile de cannelle, d'huile de citronnelle, d'huile de clou de girofle, d'huile de menthe des champs, d'huile fractionnée, hydratée et cyclisée de Cymbopogon winterianus, d'acide décanoïque, d'huile d'Eucalyptus citriodora et de citronellal hydratés et cyclisés, d'huile d'Eucalyptus citriodora hydratée et cyclisée, d'eugénol, d'huile d'ail, de géraniol, d'huile de géranium, de lavande, d'extrait de Lavandula hybrida/huile de lavandin, d'huile de citron, d'huile de citronnelle (lemongrass), d'extrait de margousier, d'extrait de Mentha arvensis, de métofluthrine, d'un mélange de cis- et de trans-p-menthane-3,8-diol, de N,N-diéthyl-méta-toluamide, d'acide nonanoïque, d'huile de menthe poivrée, de pyréthrines et de pyréthrinoïdes, d'huile de romarin, d'huile de menthe verte, d'huile de thym, d'huile de gaulthérie, de 2,3,4,5-bis(butyl-2-ène)tétrahydrofurfural (MGK Repellent 11), de cinéole, de cinnamaldéhyde, de citral, de citronellal, de citronellol, d'huile de citronnelle de Java, de coumarine, de phtalate de dibutyle, de phtalate de diéthyle, d'anthranilate de diméthyle, de phtalate de diméthyle, de vanilline éthylique, d'huile d'eucalyptus, de δ-octalactone, de δ-nonalactone, de δ-décalactone, de δ-undécalactone, de δ-dodécalactone, de γ-octalactone, de γ-nonalactone, de γ-décalactone, de γ-undécalactone, de γ-dodécalactone, d'hydroxycitronellal, d'huile de citron vert (lime), de limonène, de linalol, d'anthranilate de méthyle, de Mint arvensis, d'huile de menthe, de Mint piperita, de Mint spicata, de myrcène, d'huile de neem, de sabinène, de β-caryophyllène, d'acide (1H-indol-2-yl)acétique, d'anéthole, d'huile d'anis, d'huile de basilic, d'huile de laurier, de camphre, de salicylate d'éthyle, d'huiles de conifères (huile de pin), de (1,3,4,5,6,7-hexahydro-1,3-dioxo-2H-isoindol-2-yl)méthyl (1R-trans)-2,2-diméthyl-3-(2-méthylprop-1-ényl)cyclopropanecarboxylate (d-tétraméthrine), de (RS)-3-allyl-2-méthyl-4-oxocyclopent-2-ényl-(1R,3R;1R,3S)-2,2-diméthyl-3-(2-méthylprop-1-ényl)cyclopropanecarboxylate (mélange de quatre isomères 1R trans, 1R ; 1R trans, 1S ; 1R cis, 1R ; 1R cis, 1S selon un rapport 4:4:1:1) (d-alléthrine), de (RS)-α-cyano-3-phénoxybenzyl-(1RS)-cis,trans-3-(2,2-dichlorovinyl)-2,2-diméthylcyclopropanecarboxylate (cyperméthrine), de 2-méthyl-4-oxo-3-(prop-2-ynyl)cyclopent-2-én-1-yl-2,2-diméthyl-3-(2-méthylprop-1-ényl)cyclopropanecarboxylate (pralléthrine), d'acétamipride, d'azadirachtine, de bendiocarbe, de bifenthrine, d'acide borique, de chlorpyrifos, de deltaméthrine, de diazinon, de dichlorvos, d'eugénol, de fipronil, d'imidaclopride, de linalol, de malathion, de maltodextrine, d'extrait de margousier, de métofluthrine, de nicotine, de perméthrine, de pyréthrines et de pyréthrinoïdes, de roténone, de dioxyde de silicium (dioxyde de silicium/kieselguhr), de S-méthoprène, de spinosad (spinosyne A), de spinosyne D, de tétraméthrine et de transfluthrine.

6. Utilisation non thérapeutique de la composition selon l'une quelconque des revendications 1 à 5, qui comprend du N,N-diéthyl-3-méthylbenzamide (DEET), du butylacétylaminopropionate d'éthyle (IR3535), du para-menthane-3,8-diol (PMD) ou de la 1-(1-méthylpropoxycarbonyl)-2-(2-hydroxyéthyl)pipéridine (icaridine), de préférence en une quantité comprise entre 0,02 et 80 % en poids, plus préférentiellement en une quantité comprise entre 0,05 et 70 % en poids, encore plus préférentiellement en une quantité comprise entre 0,1 et 60 % en poids, sur la base du poids total de la composition.

7. Utilisation non thérapeutique de la composition selon l'une quelconque des revendications 1 à 6, qui comprend au moins un co-ingrédient de lutte contre les arthropodes qui contribue à la fois à l'activité de lutte contre les arthropodes et au caractère olfactif de la composition.

8. Utilisation non thérapeutique de la composition selon la revendication 7, dans laquelle au moins un co-ingrédient de lutte contre les arthropodes est choisi parmi le groupe comprenant
- une famille de matières premières parfumantes acides sélectionnées dans le groupe constitué d'acides pentanoïques à dodécanoïques, de préférence l'acide hexanoïque, l'acide heptanoïque, l'acide octanoïque et l'acide nonanoïque,
- une famille de matières premières parfumantes à base d'alcène sélectionnées dans le groupe constitué du 1-isopropyl-4-méthyl-1,3-cyclohexadiène, du 4-isopropényl-1-méthylcyclohexène, du 4-isopropylidène-1-méthylcyclohexène et du bêta-ocimène,
- une famille de matières premières parfumantes à base d'alcool et de phénol sélectionnées dans le groupe constitué du 1-décanol, du 2-méthyl-4-phényl-2-butanol, du 8-p-menthén-2-ol, du 3,4-diméthylphénol, du 3,7-diméthyl-2,6-octadién-1-ol, du 2-isopropylphénol, du 2-méthoxy-4-vinylphénol, du 1,8-p-menthadién-7-ol, du 4-éthylphénol, du 4-méthylphénol, de la 4-(triméthyl-1-cyclohexén-1-yl)-2-butanone et du 2,5,5-triméthyl-1,2,3,4,4a,5,6,7-octahydro-2-naphtalénol,
- une famille de matières premières parfumantes à base d'aldéhyde sélectionnées dans le groupe constitué du décanal, du 2-nonénal, du 5-éthyl-2-méthylnonanal, du 3-phénylpropanal, du 7-hydroxy-3,7-diméthyloctanal, du 2-décénal, du 4-hexénal, du 8-nonénal, du 2-penténal, du 2,4-nonadiénal, du 2,6-diméthyl-5-hepténal, du 2-octénal et du 2,6-nonadiénal,
- une famille de matières premières dparfumante à base d'amine sélectionnées dans le groupe constitué du 2-aminobenzoate de méthyle, du 2-aminobenzoate d'éthyle, de la 3-méthylbutylamine, de la 1-(2-aminophényl)-1-éthanone et du 3-méthylindole,
- une famille de matières premières parfumantes à base de cétone sélectionnées dans le groupe constitué de la 2-hexylidène-1-cyclopentanone, de la 3-méthyl-2-(pentyloxy)-2-cyclopentén-1-one, de la 1-(2,6,6-triméthyl-1,3-cyclohexadién-1-yl)-2-butén-1-one, de l'octalactone, de préférence de la 6-propyloxan-2-one et de la 5-butyloxolan-2-one, de la nonalactone, de préférence de la 6-butyloxan-2-one et de la décalactone, de préférence de la 5-hexyloxolan-2-one, de l'undécalactone, de préférence de la 6-hexyloxolan-2-one et de la 5-heptyloxolan-2-one, de la dodécalactone, de préférence de la 6-heptyloxan-2-one et de l'e, de la1-(2,6,6-triméthyl-1-cyclohexén-1-yl)-2-butén-1-one, de la 1-(2,6,6-triméthyl-2-cyclohexén-1-yl)-2-butén-1-one, du {3-oxo-2-[(2Z)-2-penten-1-yl]cyclopentyl}acétate de méthyle , du 2-hexyl-3-oxocyclopentane-1-carboxylate de méthyle, de la 6-méthyl-5-heptén-2-one, 3-methyl-2-[2-penten-1-yl]-2-cyclopenten-1-one, de la1-(5,5-diméthyl-1-cyclohexén-1-yl)-4-pentén-1-one, de la 2-hydroxy-3-méthyl-2-cyclopentén-1-one, de la 4-(2-méthyl-2-propanyl)cyclohexanone, 5-methyltricyclo[6.2.1.0^{2,7}]undécan-4-one, de la 2,2,6-triméthylcyclohexanone et udu (1R)-cis-3-oxo-2-pentyl-1-cyclopentaneacétate de méthyle,
- une famille de matières premières parfumantes à base d'ester sélectionnée dans le groupe constitué du 3-phényl-2-propénoate d'allyle, du 3-phényl-2-propénoate de 3-phényl-2-propényle, du 1,5-diméthyl-1-vinyl-4-hexényl 3-phénylpropénoate, du 3-phényl-2-propénoate d'éthyle, du 3-phényl-2-propénoate d'isobutyl, du 2-phényléthyl 2-buténoate, du 3-méthyl-2-buténoate de 2-phényléthyle, du 3-hexénoate de méthyle et du 2-méthyl-2-buténoate2-de phényléthyle,
- une famille de matières premières parfumantes à base d'éther, époxyde et acétal sélectionnées dans le groupe constitué de triméthyl-13-oxabicyclo[10.1.0]tridéca-4,8-diene, 1,3,3-triméthyl-2-oxabicyclo[2.2.2]octane et 3,4'-dimethylspiro[oxirane-2,9'-tricyclo[6.2.1.0^{2,7}]undéc[4]ene,
- une famille de matières premières parfumantes à base de lactone sélectionnées dans le groupe constitué de 4-nonanolide, de 3-propylidène-2-benzofuran-1(3h)-one, de 3-butylidene-1-benzo[C]furanone, de 6-pentyltétrahydro-2H-pyran-2-one, 6-propyltétrahydro-2H-pyran-2-one, de 2-chroménone, de 8-oxatricyclo[5.3.1.0(2,6)]undécan-9-one, 6-pentyloxan-2-one, d'éthylidene-3-oxatricyclo[6.2.1.0^{2,7}]undécan-4-one, 8-décén-5-olide, de 5-nonanolide, de 5-butyloxolan-2-one, de 2-chromanone, de 1-oxaspiro[4.5]décan-2-one et de 6-hexyltétrahydro-2H-pyran-2-one,
- une famille de matières premières parfumantes N-hétéro-aromatiques sélectionnées dans le groupe constitué de 1-(2-pyridyl)-1-éthanone, de 3-(1-butényl)pyridine, de 1-(3-pyridyl)-1-éthanone, de 3-éthylpyridine, de butylquinoléine, de 2-isobutylquinoléine, de 2-éthyl-3-méthylpyrazine, de 4-(4,8-diméthyl-3,7-nonadién-1-yl)pyridine, de 5-méthylquinoxaline et de 6-méthylquinoléine,
- une famille de matières premières parfumantes de mélange complexe sélectionnées dans le groupe constitué d'huile d'armoise, d'huile de calamus, d'huile de camomille, d'huile de citronnelle, d'huile de magnolia, d'huile de tanaisie, d'huile de menthe pouliot, d'huile de patchouli et d'huile de bouleau, et
- des mélanges de celles-ci,
de préférence dans lequel au moins un co-ingrédient de lutte contre les arthropodes est choisi parmi le groupe constitué de
- un membre de la famille de matières premières parfumantes acides, sélectionné dans le groupe constitué de l'acide octanoïque,
- un membre de la famille de matières premières parfumantes à base d'alcène sélectionné dans le groupe constitué du 1-isopropyl-4-méthyl-1,3-cyclohexadiène, du 4-isopropényl-1-méthylcyclohexène, du 4-isopropylidène-1-méthylcyclohexène et du bêta-ocimène,
- un membre de la famille de matières premières parfumantes à base d'alcool et de phénol sélectionné dans le groupe constitué du 2,5,5-triméthyl-1,2,3,4,4a,5,6,7-octahydro-2-naphtalénol et de la 4-(triméthyl-1-cyclohexén-1-yl)-2-butanone,
- un membre de la famille de matières premières parfumantes à base d'aldéhyde sélectionné dans le groupe constitué du décanal, du 7-hydroxy-3,7-diméthyloctanal, du 2-décénal, du 2,6-diméthyl-5-hepténal et du 2,6-nonadiénal,
- un membre de la famille de matières premières parfumantes à base d'amine sélectionné dans le groupe constitué du 2-aminobenzoate de méthyle,
- un membre de la famille de matières premières parfumantes à base de cétone sélectionné dans le groupe constitué de 1-(2,6,6-triméthyl-1,3-cyclohexadién-1-yl)-2-butén-1-one, de 1-(2,6,6-triméthyl-2-cyclohexén-1-yl)-2-butén-1-one, (2E)-1-(2,6,6-triméthyl-1-cyclohexén-1-yl)-2-butén-1-one, de 2-hexyl-3-oxocyclopentane-1-carboxylate de méthyle, de 1-(5,5-diméthyl-1-cyclohexén-1-yl)-4-pentén-1-one 5-methyltricyclo[6.2.1.0^{2,7}]undécan-4-one et de (1R)-cis-3-oxo-2-pentyl-1-cyclopentaneacétate de méthyle,
- un membre de la famille de matières premières parfumantes à base d'éther, d'époxyde et d'acétal sélectionné dans le groupe constitué de triméthyl-13-oxabicyclo[10.1.0]tridéca-4,8-diène, de 1,3,3-triméthyl-2-oxabicyclo[2.2.2]octane et 3,4'-diméthylspiro[oxirane-2,9'-tricyclo[6.2.1.0^{2,7}]undéc[4]ene,
- un membre de la famille de matières premières parfumantes à base de lactone sélectionné dans le groupe constitué de 4-nonanolide, de 6-pentyltétrahydro-2H-pyran-2-one, de 2-chroménone, de 6-pentyloxan-2-one, d'"thylidène-3-oxatricyclo[6.2.1.0^{2,7}]undécan-4-one et de 5-butyloxolan-2-one,
- un membre de la famille de matières premières parfumantes N-hétéro-aromatiques sélectionné dans le groupe constitué d'isobutylquinoléine,
- un membre de la famille de matières premières parfumantes de mélange complexe sélectionné dans le groupe composé d'huile de citronnelle et d'huile de patchouli, et
- des mélanges de ceux-ci.

9. Utilisation non thérapeutique de la composition selon la revendication 8, dans laquelle
- le membre de la famille de matières premières parfumantes acides, lorsqu'il est présent dans la composition, est en quantité de 0,002 à 0,007 % en poids, plus préférentiellement de 0,0025 à 0,0065 % en poids,
- le membre de la famille de matières premières parfumantes à base d'alcène, lorsqu'il est présent dans la composition, est en quantité de 0,2 à 16 % en poids, de préférence de 1 à 14 % en poids, plus préférentiellement de 3 à 13 % en poids,
- le membre de la famille de matières premières parfumantes à base d'aalcool ou de phénol, lorsqu'il est présent dans la composition, est en quantité de 0,15 à 4 % en poids, de préférence de 0,2 à 3,5 % en poids, de préférence de 0,4 à 3 % en poids
- le membre de la famille de matières premières parfumantes à base d'aldéhyde, lorsqu'il est présent dans la composition, est présent en une quantité de 0,001 à 30 % en poids, de préférence de 0,005 à 25 % en poids, plus préférentiellement de 0,01 à 20 % en poids,
- le membre de la famille de matières premières parfumantes à base d'amine, lorsqu'il est présent dans la composition, est en une quantité de 0,01 à 3 % en poids, de préférence de 0,05 à 2 % en poids, plus préférentiellement de 0,1 à 0,8 % en poids,
- le membre de la famille de matières premières parfumantes à base de cétone, lorsqu'il est présent dans la composition, est en une quantité de 0,001 à 15 % en poids, de préférence de 0,01 à 10 % en poids, plus préférentiellement de 0,1 à 5,5 % en poids,
- le membre de la famille de matières premières parfumantes à base d'éther, d'époxyde et d'acétal, lorsqu'il est présent dans la composition, est en une quantité de 0,002 à 20 % en poids, de préférence de 0,02 à 15 % en poids, plus préférentiellement de 0,2 à 12,5 % en poids,
- le membre de la famille de matières premières parfumantes à base de lactone, lorsqu'il est présent dans la composition, est en une quantité de 0,002 à 65 % en poids, de préférence de 0,02 à 50 % en poids, plus préférentiellement de 0,2 à 38,8 % en poids,
- le membre de la famille de matières premières parfumantes N-hétéro-aromatiques, lorsqu'il est présent dans la composition, est en une quantité de 0,002 à 5 % en poids, de préférence de 0,02 à 2 % en poids, plus préférentiellement de 0,2 à 0,4 % en poids, et
- le membre de la famille de matières premières parfumantes de mélange complexe, lorsqu'il est présent dans la composition, est en une quantité de 0,002 à 10 % en poids, de préférence de 0,02 à 7,5 % en poids, plus préférentiellement de 0,2 à 4,8 % en poids,
sur la base du poids total de la composition.

10. Utilisation non thérapeutique de la composition selon l'une quelconque des revendications 1 à 9, dans laquelle le composé de formule (I) est 2,3-dihydro-1H-indène-2-carbonitrile, 2,4-diméthyl-4,4a,5,9b-tétrahydroindéno[1,2-d][1,3]dioxine, 2,4-diméthyl-4,4a,5,9b-tétrahydroindeno[1,2-d][1,3]dioxine, (2,5-diméthyl-2,3-dihydro-1H-indén-2-yl)méthanol, (4aRS,9bRS)-4a,7-diméthyl-4,4a,5,9b-tétrahydroindéno[1,2-d][1,3]dioxine, (2RS,4aRS,9bRS)-2,4a,7-triméthyl-4,4a,5,9b-tétrahydroindéno[1,2-d][1,3]dioxine, (2RS,4aSR,9bSR)-2,4a,7-triméthyl-4,4a,5,9b-tétrahydroindéno[1,2-d][1,3]dioxine, (2-méthyl-1,3-dihydroindén-2-yl)méthanol, 2,3-dihydro-1H-indén-2-ylméthanol, (2RS,4RS,4ASR,9BRS)-2,4-diméthyl-4,4a,5,9b-tétrahydroindéno[1,2-d][1,3]dioxine, (2RS,4aRS,9bSR)-2-méthyl-4,4a,5,9b-tétrahydroindéno[1,2-d][1,3]dioxine, (2RS,4aSR,9bRS)-2-méthyl-4,4a,5,9b-tétrahydroindéno[1,2-d][1,3]dioxiné, (4aRS,9bSR)-2,2-diméthyl-4,4a,5,9b-tétrahydroindéno[1,2-d][1,3]dioxine, (2,4,6-triméthyl-2,3-dihydro-1H-indén-2-yl)méthanol, 5-(tert-butyl)-1,3-dihydrospiro[indène-2,3'-oxétane], (2RS,4RS,4ARS,9BSR)-2,4-diméthyl-4,4a,5,9b-tétrahydroindéno[1,2-d][1,3]dioxine, 4A,8-diméthyl-indano[1,2-d]-1,3-dioxan-2-one, 1-(2,5-diméthyl-2,3-dihydro-1H-indén-2-yl)éthanone, (5-méthyl-2,3-dihydro-1H-indén-2-yl)méthanol, (4aRS,9bSR)-8-méthyl-4,4a,5,9b-tétrahydroindéno[1,2-d][1,3]dioxine, (2RS,4aRS,9bSR)-2-éthyl-4,4a,5,9b-tétrahydroindéno[1,2-d][1,3]dioxine, (2RS,4aSR,9bRS)-2-éthyl-4,4a,5,9b-tétrahydroindéno[1,2-d][1,3]dioxine, (2R,4aS,9bS)-2,4a,8-triméthyl-4,4a,5,9b-tétrahydroindéno[1,2-d][1,3]dioxine, 1-(2,5-diméthyl-2-indanyl)-1-éthanol, (2,5-diméthyl-2,3-dihydro-1H-indén-2-yl)méthanol, (2RS,4aRS,9bSR)-2,8-diméthyl-4,4a,5,9b-tétrahydroindéno[1,2-d][1,3]dioxine, (2RS,4aSR,9bRS)-2,8-diméthyl-4,4a,5,9b-tétrahydroindéno[1,2-d][1,3]dioxine, (2RS,4aSR,9bSR)-2,4a-diméthyl-4,4a,5,9b-tétrahydroindéno[1,2-d][1,3]dioxine, (4aRS,9bRS)-4a,8-diméthyl-4,4a,5,9b-tétrahydroindéno[1,2-d][1,3]dioxine, 1-(2,4,5-triméthyl-2,3-dihydro-1H-indén-2-yl)éthénone, (2RS,4aRS,9bSR)-2-éthyl-8-méthyl-4,4a,5,9b-tétrahydroindéno[1,2-d][1,3]dioxine, (2RS,4aSR,9bRS)-2-éthyl-8-méthyl-4,4a,5,9b-tétrahydroindéno[1,2-d][1,3]dioxine, (4aRS,9bRS)-2,2,4a,8-tétraméthyl-4,4a,5,9b-tétrahydroindéno[1,2-d][1,3]dioxine, 5-tert-butyl-indan-2-spiro-3'-oxétane, (2,6-diméthyl-1,2,3,4-tétrahydro-2-naphtalényl)méthanol, (4aRS,9bRS)-2,2,4a-triméthyl-4,4a,5,9b-tétrahydroindéno[1,2-d][1,3]dioxine, ou (4aRS,9bRS)-2,2,4a,7-tétraméthyl-4,4a,5,9b-tétrahydroindéno[1,2-d][1,3]dioxine.

11. Procédé non thérapeutique de lutte contre les arthropodes, de préférence un insecte, lutte qui consiste à amener un arthropode, de préférence un insecte, en contact direct ou en contact avec les vapeurs d'une composition selon l'une quelconque des revendications 1 à 10 ou d'un composé de formule (I) selon l'une quelconque des revendications 1 ou 10.

12. Composé de formule (I) tel que défini dans la revendication 1, dans lequel le composé est choisi parmi 2,3-dihydro-1H-indène-2-carbonitrile, (4aRS,9bRS)-4a,7-diméthyl-4,4a,5,9b-tétrahydroindéno[1,2-d][1,3]dioxine, (2RS,4aRS,9bRS)-2,4a,7-triméthyl-4,4a,5,9b-tétrahydroindéno[1,2-d][1,3]dioxine, (2RS,4aSR,9bSR)-2,4a,7-triméthyl-4,4a,5,9b-tétrahydroindéno[1,2-d][1,3]dioxine, 2,3-dihydro-1H-indén-2-ylméthanol, (2RS,4aRS,9bSR)-2-méthyl-4,4a,5,9b-tétrahydroindéno[1,2-d][1,3]dioxine, (2RS,4aSR,9bRS)-2-méthyl-4,4a,5,9b-ttrahydroindéno[1,2-d][1,3]dioxine, (4aRS,9bSR)-2,2-diméthyl-4,4a,5,9b-tétrahydroindéno[1,2-d][1,3]dioxine, 5-(tert-butyl)-1,3-dihydrospiro[indène-2,3'-oxetane], 4A,8-diméthyl-indano[1,2-d]-1,3-dioxan-2-one, (4aRS,9bSR)-8-méthyl-4,4a,5,9b-tétrahydroindéno[1,2-d][1,3]dioxine, (2RS,4aRS,9bSR)-2-ethyl-4,4a,5,9b-tétrahydroindéno[1,2-d][1,3]dioxine, (2RS,4aSR,9bRS)-2-éthyl-4,4a,5,9b-tétrahydroindéno[1,2-d][1,3]dioxine, et (2R,4aS,9bS)-2,4a,8-triméthyl-4,4a,5,9b-tétrahydroindéno[1,2-d][1,3]dioxine.
